(19) Europäisches Patentamt / European Patent Office / Office européen des brevets

(11) **EP 3 829 571 B1**

(12) **EUROPEAN PATENT SPECIFICATION**

(45) Date of publication and mention of the grant of the patent:
**09.04.2025 Bulletin 2025/15**

(21) Application number: **19844778.1**

(22) Date of filing: **31.07.2019**

(51) International Patent Classification (IPC):
***A61K 31/4025*** *(2006.01)* ***A61K 45/06*** *(2006.01)*
***A61K 9/08*** *(2006.01)* ***A61P 35/00*** *(2006.01)*
***A61P 31/04*** *(2006.01)* ***A61K 9/00*** *(2006.01)*
***A61K 47/02*** *(2006.01)*

(52) Cooperative Patent Classification (CPC):
(C-Sets available)
**A61K 9/0019; A61K 9/08; A61K 31/4025; A61K 39/001106; A61K 39/001118; A61K 39/001121; A61K 39/001124; A61K 39/001128; A61K 39/001129; A61K 39/001131; A61K 39/001158; A61K 39/001159; A61K 39/001166; A61K 39/001174; A61K 39/00118;** (Cont.)

(86) International application number:
**PCT/US2019/044421**

(87) International publication number:
**WO 2020/028532 (06.02.2020 Gazette 2020/06)**

(54) **ADJUVANT EFFECT OF THE TLR1/2 AGONIST DIPROVOCIM SYNERGIZES WITH CHECKPOINT-INHIBITING ANTIBODIES TO ELIMINATE CANCEROUS OR INFECTIOUS DISEASES**

ADJUVANTE WIRKUNG DES TLR1/2-AGONISTEN DISPROVOCIM IN SYNERGIE MIT CHECKPOINT-INHIBIERENDEN ANTIKÖRPERN ZUR BEKÄMPFUNG VON INFEKTIÖSEN ODER KANZERÖSEN KRANKHEITEN

SYNERGIE DE L'EFFET ADJUVANT DE L'AGONISTE TLR1/2 DIPROVOCIM ET DES ANTICORPS INHIBITEURS DE POINTS DE CONTRÔLE POUR ÉLIMINER UNE MALADIE CANCÉREUSE OU INFECTIEUSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.08.2018 US 201862713823 P**

(43) Date of publication of application:
**09.06.2021 Bulletin 2021/23**

(73) Proprietor: **The Board Of Regents Of The University Of Texas System**
**Austin, TX 78701 (US)**

(72) Inventors:
- **BEUTLER, Bruce**
  **Irving, TX 75039 (US)**
- **WANG, Ying**
  **Dallas, TX 75205 (US)**

(74) Representative: **Thurston, Joanna**
**Withers & Rogers LLP**
**2 London Bridge**
**London SE1 9RA (GB)**

(56) References cited:
**WO-A1-2014/163684**
**WO-A1-2018/005812**
**WO-A2-2016/022700**
**US-A1- 2010 173 969**
**US-A1- 2016 362 472**
**US-A1- 2018 015 161**
**US-A1- 2018 169 224**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **YING WANG ET AL: "Adjuvant effect of the novel TLR1/TLR2 agonist Diprovocim synergizes with anti?PD-L1 to eliminate melanoma in mice", PROCEEDINGS OF THE NATIONAL ACADEMY OF SCIENCES, vol. 115, no. 37, 27 August 2018 (2018-08-27), pages E8698 - E8706, XP055611937, ISSN: 0027-8424, DOI: 10.1073/pnas.1809232115**

(52) Cooperative Patent Classification (CPC): (Cont.)
**A61K 45/06; A61K 47/02; A61P 31/04; A61P 35/00**

C-Sets
**A61K 31/4025, A61K 2300/00;**
**A61K 45/06, A61K 2300/00**

## Description

### GOVERNMENTAL SUPPORT

This invention was made with governmental support under AI125581 awarded by the National Institutes of Health. The government has certain rights in the invention.

### BACKGROUND ART

By activating antigen-presenting cells (APCs) including dendritic cells (DCs) and macrophages, adjuvants hold the potential to unleash the natural functions of cytotoxic T lymphocytes (CTLs) to kill pathogens or cancer. Many adjuvants including TLR agonists engage innate immune receptors on APCs, inducing APCs to present antigens, produce cytokines, and provide costimulatory signals (1, 2) to antigen-specific CD8 T cells. In response to these signals, CD8 T cells proliferate and differentiate into CTLs capable of killing infected or tumor cells expressing their target antigen. In addition, such signals activate CD4 T cells, inducing their expansion and differentiation into Th1 or Th2 T helper cells (3).

One of the most important targets of improved adjuvant technology lies in the field of cancer immunotherapy, where the adaptive immune system is exploited to kill cancer cells based on their expression of cancer associated antigens or neo-antigens (4, 5). The effectiveness of cancer immunotherapy depends on the generation and activation of tumor-specific CTLs (5, 6) and on their maintenance of activity *in vivo,* leading to killing of tumor cells and a long lasting anti-tumor memory response (5). Thus, immune checkpoint inhibitors such as anti-PD-1, anti-PD-L1 and anti-CTLA-4 have achieved remarkable clinical success in the treatment of melanoma and other cancers through their action in blocking pathways that inhibit CTL activation (7, 8).

However, even among those tumors known to be susceptible to checkpoint blockade, response rates of only about 20% have been reported for PD-1/PD-L1 antibody treatment (5, 9), possibly due to insufficient numbers or activation of tumor-reactive CTLs, or their failure to infiltrate tumors. These deficiencies may be exacerbated by immunosuppression induced by the cancer environment.

In addition, the lack of T cell support is thought to be at least in part responsible for the lack of vigor exhibited by many synthetic vaccines against various pathogens as well as cancer-related cell surface antigens.

TLR ligands have long been known to act as adjuvants in adaptive immune responses (10, 11) signaling via adapter proteins (MyD88, TRIF, TRAM, MAL), kinases, and ubiquitin ligases to activate NF-κB and IRFs (12-14). These and other transcription factors induce the expression of thousands of genes that carry out the innate immune response (15). Several nucleotide-based adjuvants such as TLR3 agonist poly I:C (9), TLR9 agonist CpG (16), and STING agonist cGAMP (6) have been reported to improve the efficacy of immune checkpoint inhibitors in pre-clinical models for cancer treatment.
US2018/169224 discloses the use of TLR1/2 agonist to increase an immune response to an antigen in combination with a T cell activating agent.

These approaches aim to increase the number of tumor-specific CTLs upon which checkpoint inhibitors can act. However, they have relied chiefly on natural TLR ligands, which are difficult to synthesize, and in some instances quite toxic, presumably because they become widely disseminated *in vivo,* and activate myeloid cells indiscriminately, producing cytokine storm (17). Development was sought of agonists with superior pharmacologic properties, with defined structural and molecular mechanisms from which key adjuvant design principles can be learned.

By screening a library of synthetic compounds, a potent human- and mouse-active TLR1/2 agonist, Diprovocim, was identified that has no structural similarity to any microbial TLR agonist. As described below, Diprovocim illustratively elicits strong adjuvant activity in mice, successfully inhibiting tumor growth and prolonging survival when combined with a cancer antigen and immune checkpoint blockade in the B16-OVA melanoma model.

### BRIEF SUMMARY OF THE INVENTION

Adjuvants enhance adaptive immune responses, sometimes through unknown mechanisms, and can be used to augment both humoral and cellular responses to cancer antigens. The invention described herein illustrates synergistic immunological effects of the synthetic chemical adjuvant Diprovocim, which targets the innate immune receptor TLR2/TLR1 in mice and humans, that when used in conjunction with a diseased cell marker immunogen molecule and a checkpoint inhibitor provides a synergistic effect in inhibiting the growth of a diseased cell as compared to the use of the individual components or any two of those components.

The present invention an immunizing pharmacological composition for use in inhibiting the growth of cancerous or pathogen-infected cells in a mammal. Those diseased cells express a marker molecule that is absent from cells of the same type that are free of the disease or is present in the disease-free cells in significantly reduced numbers compared to said diseased cells.

The invention provides:

an immunizing pharmacological composition for use in inhibiting the growth of cancerous or pathogen-infected cells in a mammal, which diseased cells exhibit a marker molecule that is absent on cells of the same type that are free of said disease or is present on the disease-free cells in significantly reduced numbers compared to said diseased cells, the pharmaceutical composition comprising:

(i) an adjuvant-sufficient amount of a Diprovocim compound, (ii) a T cell-stimulating amount of an immune checkpoint inhibitor, and (iii) an immunizing amount of said marker molecule or portion thereof;

and

wherein said Diprovocim compound corresponds in structure to structural Formula **V,**

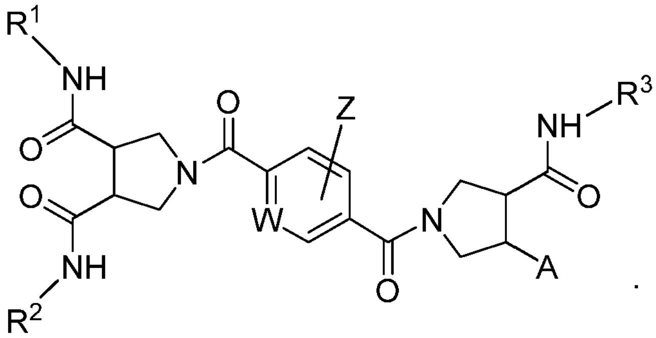

**V**

wherein

**-A** is -H (hydrido) or -C(O)NH-$R^4$;

$R^1$, $R^2$, $R^3$ and $R^4$ are the same or different and are a 2-(4-fluorophenyl)ethyl, a trans-2-phenylcyclopropyl, a trans-2-(4-fluorophenyl)-cyclopropyl or a $c_2$-$c_{18}$ hydrocarbyl group with the provisos that:

1) at least two of $R^1$, $R^2$, $R^3$ and $R^4$ ($R^{1-4}$) or at least two of $R^1$, $R^2$, and $R^3$ ($R^{1-3}$) are a trans-2-phenylcyclopropyl, a trans-2-(4-fluorophenyl)-cyclopropyl group or a mixture thereof, or each of $R^{1-4}$ is a 2-(4-fluorophenyl)ethyl group,
2) at least one depicted pyrrolidinyldicarboxamido group has the (*S*,*S*) configuration, and each depicted R substituent other than a $c_2$-$c_{18}$ hydrocarbyl group is a trans-2-phenylcyclopropyl, a trans-2-(4-fluorophenyl)-cyclopropyl group or a mixture thereof when each of $R^{1-4}$ is other than 2-(4-fluorophenyl)ethyl,
3) no more than two of $R^{1-4}$ are $c_2$-$c_{18}$ hydrocarbyl groups when **-A** is -C(O)NH-$R^4$, and
4) when A is hydrido, one of $R^{1-3}$ can be a $c_2$-$c_{18}$ hydrocarbyl group and the depicted $R^3$-containing pyrrolidinylcarboxamido group can have either the R or S configurations, or a mixture of both configurations;

-Z is one or more of halogen -H, $-NH_2$, -OH, $-OCH_3$, $-NO_2$, $-OCH_2CO_2H$, $-O(CH_2CH_20)_nCH_2CH_2CO_2H$, $-OCH_2CONH(CH_2CH_2O)_nCH_2CH_2CO_2H$, $-NHCOCH_2O-(CH_2CH_2O)_nCH_2CO_2H$, $-OCH_2CONHCH_2CONHCH(CHOH)CO_2H$, $-OCH_2CONHCH_2CONHCHCO_2H(CH_2CO_2H)$, $-OCH_2CONHCH_2CONHCH(CHOH)\ (CH_2CH_2O)_nCH_2CH_2CO_2H$, $-OCH_2CONHCH_2CONHCH[(CH_2)_4NH_2]CO_2H$, $-OCH_2CONHCH_2CONHCH(CH_2OH)CO\{NHCH[(CH_2)_4NH_2]CO\}_mNHCH-[(CH_2)_4NH_2]CO_2H$ (SEQ ID NOs: 3-8), $-OCH_2CONHCH_2CO\{NHCH[(CH_2)_4NH_2]CO\}_pNHCH[(CH_2)_4NH]CO_2H$ (SEQ ID NOs: 9-13) and $-OCH_2CONHCH_2CO\{NHCH(CH_2OH)CO\}_qNHCH(CH_2OH)CO_2H$ (SEQ ID NOs: 14-18);
W is nitrogen (N) or CH;
"n" is a number whose average value is one to about eight;
"m" is a number whose value is 1 to about 6; "p" is a number whose value is 1 to about 6; and
"q" is a number whose value is 1 to about 6.

Diprovocim-1 (WO 2018/005812; Compound 3) displayed strong adjuvant activity in mice, particularly abetting cellular immune responses.

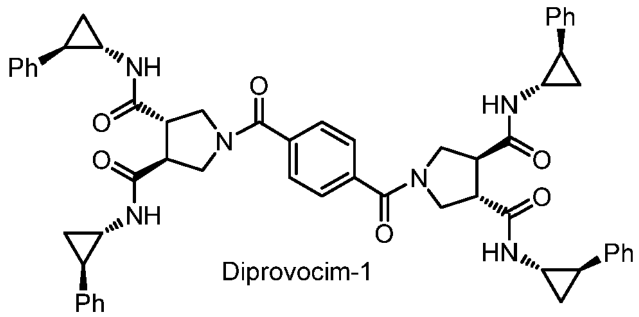

Diprovocim-1

Immunization against a genetically engineered tumor-specific antigen, ovalbumin, when adjuvanted with Diprovocim-1, inhibited the growth of B16 melanoma and prolonged survival in the presence of immune checkpoint blockade by anti-PD-L1. Immunization with ovalbumin plus the Diprovocim or ovalbumin plus the anti-PD-L1 inhibitor, exhibited some benefit, but 100% of mice responded to immunization with the three components: Diprovocim, immunogen and checkpoint inhibitor. The data suggest Diprovocim-1 boosts the success of anti-PD-L1 treatment by increasing the number and activation of tumor-specific CTLs capable of responding to this checkpoint inhibitor.

As disclosed in WO 2018/005812 and Morin et al., J Am Chem Soc, In Press (2018), several members of the Diprovocim family have been prepared, their activity assayed, and have been given numbers such as Diprovocim-1 through Diprovocim-6. Several other compounds of the Diprovocim family having similar activity profiles, similar to somewhat lesser activity values in the assays used, have also been prepared and assayed.

Diprovocim-1 has been used herein as an exemplary member of the whole family, and is to be understood hereinafter to mean a Diprovocim family member is used when the word Diprovocim is used without an added hyphenated numeral. Thus, when used in connection with the words "family" or "a" as in "Diprovocim family" or "a Diprovocim", the word "Diprovocim" is used to mean a member of the Diprovocim family as defined by Formula **V.** The word "Diprovocim" used with the word "preferred" refers to a compound of Formula **I,** and the phrase "more preferred" or "more preferably" refers to a Diprovocim family compound of Formula **Ia.** A "most preferred" Diprovocim is one of the compounds lettered **A-I** noted below.

A preferred member of the Diprovocim family of compounds is a compound that corresponds in structure to structural Formula **I,** below,

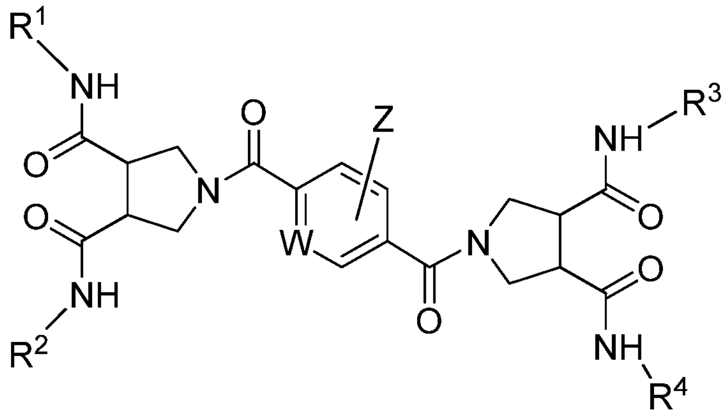

**I**

in which -A -C(O)NH-$R^4$. The depicted $R^{1-4}$, W and Z moieties are as described above.

A still more preferred member of the Diprovocim family of compounds is a compound that corresponds in structure to structural Formula **Ia,** below,

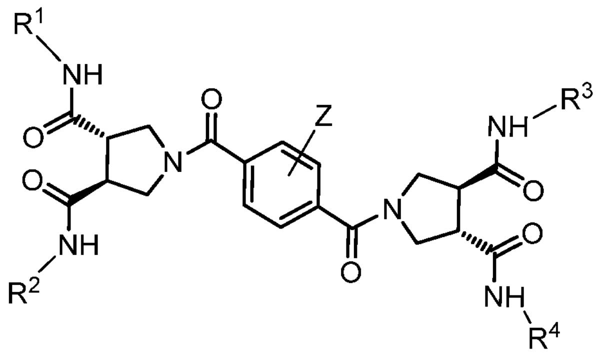

**Ia**

Structural formulas of currently most preferred compounds of Formula **Ia** in which $R^{1-4}$ are as previously defined, and -Z is hydrido (-H) are shown hereinafter as compounds of Formulas **A, B, C, D, E, F, G, H,** and **I.**

A Diprovocim is utilized in an adjuvant-sufficient amount to contact host mammal cells that express an antigenic disease-related marker molecule.

The host mammal cells that express an antigenic disease-related marker compound such as a peptide sequence are also contacted with an immune response-stimulating amount of a checkpoint inhibitor, preferably an antibody or paratope-containing antibody portion.

## BRIEF DESCRIPTION OF THE DRAWINGS

In the drawings forming a portion of this disclosure

Fig. 1A through Fig. 1F provide a series of graphs that illustrate that Diprovocim induces cytokine secretion by mouse and human cells. Fig. 1A-1D illustrates amounts of TNF in the supernatants of human THP-1 cells (Fig. 1A), human PBMC (Fig. 1B), mouse peritoneal macrophages (Fig. 1C), or mouse BMDC (Fig. 1D) after treatment with Diprovocim-1 for 4 hours (Fig. 1A, Figs. 1C-1D) or 24 hours (Fig. 1B). Fig. 1E illustrates the amounts of IL-6 in the supernatants of mouse BMDC after treatment with Diprovocim for 4 hours. In each of Fig. 1A-1E the means of three independent samples are plotted; P values were determined by one-way ANOVA to compare the responses to different doses; in all studies P < 0.0001. Results in Fig. 1A - Fig. 1E are representative of two independent studies.

Fig. 2A through Fig. 2D illustrate that a Diprovocim activates mouse and human TLR1/TLR2. Fig. 2A is a graph showing the amount of TNF in the supernatants of mouse peritoneal macrophages of the indicated genotypes after treatment with Diprovocim (500 nM) for 4 hours (n = 3 mice per genotype). Cytokine levels were normalized to those of stimulated C57BL/6J cells. P values were determined by Student's t test and represent the significance of differences between responses of stimulated C57BL/6J cells and stimulated cells of mutant genotypes; four stars indicate those with statistically significant differences. Fig. 2B is a graph showing the amount of TNF in the supernatants of human THP-1 cells pretreated with control antibody, anti-TLR1 (20 μg/ml), or anti-TLR2 (20 μg/ml) for 1 hour, followed by treatment with vehicle or Diprovocim (250 pM) for another 4 hours. P values were determined by Student's t test. In Fig. 2A and Fig. 2B the means of three independent samples are plotted. Fig. 2C and Fig. 2D are immunoblot show the results of analysis of lysates of human THP-1 cells (Fig. 2C) and mouse peritoneal macrophages (Fig. 2D) treated with Diprovocim (5 nM in THP-1 and 500 nM in mouse peritoneal macrophages) for the indicated times. All results are representative of two independent studies.

Fig. 3A through Fig. 3G illustrate through graphs and schemes that Diprovocim enhances antigen-specific antibody and CTL responses. Fig. 3A - Fig. 3C show results WT or *Tlr2*$^{-/-}$ C57BL/6J mice (4 mice per group) were immunized **i.m.** with 100 μg OVA mixed with vehicle, Diprovocim (10 mg/kg), or alum (2 mg/kg). After 14 days, serum titers of OVA-specific IgG (Fig. 3A), OVA-specific IgG1 (Fig. 3B) and OVA-specific IgG2b (Fig. 3C) were measured by ELISA. Fig. 3D shows a schematic of the experimental setup (left) and results for detection (center) and quantification (right) of CD69 expression on OT-I CD8 T cells by flow cytometery after 24 hours co-culture with DC collected from mice 24 hours after they were immunized i.m. with OVA mixed with vehicle or Diprovocim (4 mice per group).

Fig. 3E and Fig. 3F show results from mice that were unimmunized or immunized i.m. with 100 μg OVA mixed with vehicle or Diprovocim (10 mg/kg) (4 mice per group). Seven days after immunization, mice were injected i.v. with Celltrace Violet-labeled mouse splenocytes that were unpulsed (control cells) or pulsed with OVA peptide (a.a. 257-263) (target cells). Two days later, blood was collected to measure remaining live dye-labeled cells. Fig. 3E illustrates representative flow cytometry plots that count remaining target cells (right peak) and control cells (left peak) in wild type mice. Fig. 3F shows a quantitative comparison of the percentage of target cells killed in WT, *Tlr1*$^{-/-}$or *Tlr2*$^{-/-}$ mice. P values were determined by Student's t test. All results are representative of two independent studies.

Fig. 4A through Fig. 4J illustrate inhibition of B16-OVA tumor growth by pre- or post-tumor treatment with Diprovocim-adjuvanted tumor vaccination and checkpoint blockade. Fig. 4A provides a schematic of pre- and post-tumor treatment protocols. C57BL/6J mice (n=8 mice per treatment) were injected s.c. with $2x10^5$ B16-OVA melanoma cells on day 0. For pre-tumor treatment, mice were immunized i.m. with OVA (100 μg) mixed with vehicle or Diprovocim (10 mg/kg) or alum (2 mg/kg) on the same day prior to tumor injection. Mice received a booster immunization seven days later in which anti-PD-L1 (200 μg) was administered on days 3, 6 and 9 after tumor inoculation by i.p. injection. For post-tumor treatment, initial immunization was on day 3 after tumor inoculation, with a booster seven days later. Anti-PD-L1 (200 μg) was administered on days 3, 6, 9, 12, and 15 after tumor inoculation by i.p. injection. Fig.4B through Fig. 4F illustrate pre-tumor treatment results. Tumor volume (Fig. 4B and Fig. 4D) and percent mouse survival (survivors/total mice) (Fig. 4C and Fig. 4E). Fig. 4F shows naive mice (n = 8) or day 35 tumor-free survivors (n = 8) from (Fig. 4C) were challenged with $2x10^5$ cells each B16-OVA and B16F10 tumor cells by s.c. injection, and tumor volume was monitored. Fig. 4G through Fig. 4J illustrate a comparison of pre-tumor (Fig. 4G and Fig. 4H) vs. post-tumor treatment (Fig. 4I and Fig. 4J). Tumor volume (Fig. 4G and Fig. 4I) and percent mouse survival (survivors/total mice) (Fig. 4H and Fig. 4J) are shown. P values for tumor volume analysis apply to the final time point as indicated in graphs and were calculated by Student's t test. P values for survival analysis were calculated by Kaplan-Meier analysis. All results are representative of two independent studies.

Fig. 5A through Fig. 5M illustrate that a Diprovocim enhances TILs and anti-tumor CTL responses. Fig. 5A is a schematic of treatment protocol for Fig. 5A through Fig. 5J in which C57BL/6J mice (n=6 mice per treatment) were injected s.c. with $2x10^5$ B16-OVA melanoma cells on day 0 and three days later, immunized i.m. with OVA (100 μg) mixed with vehicle or Diprovocim (10 mg/kg) or alum (2 mg/kg). Mice received a booster immunization on day 10 after tumor inoculation in which anti-PD-L1 (200 μg) was administered on days 3, 6, 9, and 12 after tumor inoculation by i.p. injection. Tumors were harvested on day 14 to isolate and analyze tumor-infiltrating leukocytes (TILs). Fig. 5A through Fig. 5J illustrate the frequency of each cell type out of total tumor cells is shown. Fig. 5B shows TILs ($CD45^+$). Fig. 5C shows CD4 T cells ($CD4^+CD3^+CD45^+$). Fig. 5D shows activated CD4 T cells ($CD44^{high}CD4^+CD3^+CD45^+$). Fig. 5E shows CD8 T cells ($CD8^+CD3^+CD45^+$). Fig. 5F shows activated CD8 T cells ($CD44^{high}CD8^+CD3^+CD45^+$). Fig. 5G shows OVA-specific CD8 T cells bearing a T-cell receptor specific for $OVA_{(257-264)}$-H2Kb tetramer. Fig. 5H shows NK cells ($NK1.1^+CD3^-CD45^+$) . Fig. 5I shows DCs ($CD11c^+CD3^-CD45^+$). Fig. 5J shows macrophages ($F4/80^+CD11b^+CD45^+$) .

Fig. 5K is a schematic of treatment a protocol for Fig. 5L and Fig. 5M in which C57BL/6J mice (n=8 mice per treatment) were injected s.c. with $2x10^5$ B16-OVA melanoma cells on day 0 and three days later immunized i.m. with OVA (100 μg) mixed with vehicle or Diprovocim (10 mg/kg). Mice received a booster immunization on day 10 after tumor inoculation in which anti-PD-L1 (200 μg) was administered on day 3, 6, 9, 12, and 15 after tumor inoculation by i.p. injection. On day 0, 3, 6, 9, 12, and 15, anti-CD4 (300 μg), anti-CD8 (300 μg), anti-NK1.1 (300 μg), or a mixture of these three antibodies was administered to C57BL/6J mice by i.p. injection. Tumor volume (Fig. 5L) and percent mouse survival (survivors/total mice) (Fig. 5M) are illustrated. P values for tumor volume analysis apply to the final time point as indicated in graphs and were calculated by Student's t test. P values for survival analysis were calculated by Kaplan-Meier analysis. All results are representative of two independent studies.

Fig. 6 shows a schematic model of key cellular events mediating the antitumor effect of Diprovocim-adjuvant immunization plus checkpoint inhibition.

Fig. 7 illustrates that a Diprovocim does not stimulate IFN-β secretion by mouse peritoneal macrophages. IFN-β in the supernatants of mouse peritoneal macrophages after treatment with Diprovocim or LPS for 4 hours was assayed. The means of three independent samples are plotted. P values were determined by Student's t test; no significant differences were found between responses of unstimulated cells (0 nM) and Diprovocim-stimulated cells. Results are representative of two independent studies.

Fig. 8A and Fig. 8B illustrate that anti-PD-L1 antibodies do not inhibit B16 tumor growth in mice. C57BL/6J mice (n=8) were injected s.c. with $2x10^5$ B16-OVA melanoma cells on day 0. Anti-PD-L1 (200 μg) or mouse IgG2a isotype control antibody was administered on days 3, 6 and 9 after tumor inoculation by i.p. injection. Fig. 8A is a graph of tumor volume versus time and Fig. 8B is a graph showing percent mouse survival (survivors/total mice) versus time. The control values in both plots are shown above those for the anti-PD-L1 values where the two values diverge. The P value for tumor volume analysis applies to the final time point and was calculated by Student's t test; no significant difference was found between treatments. P values for survival analysis were calculated by Kaplan-Meier analysis; no significant difference was found between treatments. Results are representative of two independent studies.

Fig. 9A and Fig. 9B illustrate that a Diprovocim is more potent than $Pam_3CSK_4$ in activation of TNF production in human cells. Fig. 9A and Fig. 9B show TNF amounts assayed from the supernatants of human THP-1 cells (Fig. 9A) and human PBMC (Fig. 9B) after treatment with Diprovocim or $Pam_3CSK_4$ for 4 hours (Fig. 9A) or 24 hours (Fig. 9B). Data points for Diprovocim are shown generally to the left of data points for $Pam_3CSK_4$ in Fig. 9A, above data points for $Pam_3CSK_4$ where the lines diverge in Fig. 9B. The means of three independent samples are plotted. Results are representative of two independent studies.

**Definitions**

Antibody: a polypeptide that immunologically binds to a ligand group. Antibodies, as used herein, are immunoglobulin molecules and immunologically active fragments of immunoglobulin molecules. Such portions known in the art as Fab, Fab'; $F(ab')_2$ and $F_V$ are included. Typically, antibodies bind ligands that range in size from about 6 through about 34 Ångstroms (Å) with association constants in the range of about $10^4$ to about $10^{10}$ $M^{-1}$, and as high as $10^{13}$ $M^{-1}$. Antibodies can bind a wide range of ligands, including small molecules such as steroids and prostaglandins, biopolymers such as nucleic acids, proteins and polysaccharides, and synthetic polymers such as polypropylene.

An "antibody combining site" or "paratope" is that structural portion of an antibody molecule comprised of a heavy and light chain variable and hypervariable regions that specifically binds to (immunoreacts with) an "antigen" or "epitope".

The term "antibody" is meant to particularly encompass monoclonal antibodies that are suitable for injection (pharmaceutically acceptable) into a diseased mammal in need of treatment without undo adverse effects due to contaminants. Such monoclonal antibodies can be obtained from the animal species that is immunized as discussed herein, such as a human. Or, the antibodies can be induced in one animal and the antibody-producing cells modified to produce antibody protein sequences of the mammal to be immunized. Although other species of mammal are contemplated for immunization, a human is a particularly preferred recipient of the immunization. As a consequence, a contemplated monoclonal antibody that was originally induced in a mouse, for example, can be more useful to a human recipient as a so-called "humanized" antibody, or as a "chimeric" antibody. These terms are used herein as described in International Nonproprietary Names (INN) for biological and biotechnological substances (a review), World Health Organization (2016), §2.7.

The word "antigen" has been used historically to designate an entity that is bound by an antibody or receptor, and also to designate the entity that induces the production of the antibody. More current usage limits the meaning of antigen to that entity bound by an antibody or receptor, whereas the word "immunogen" is used for the entity that induces antibody production or binds to the receptor. Where an entity discussed herein is both immunogenic and antigenic, reference to it as either an immunogen or antigen is typically made according to its intended utility.

The term "immunoreact" in its various forms is used herein to refer to specific binding between an antigenic determinant-containing molecule (antigen) and a molecule containing an antibody combining site such as a whole antibody molecule or a paratope-containing portion thereof.

An "antigenic determinant" is the structural portion of the antigen that is immunologically bound by an antibody combining site or T cell receptor. The term is also used interchangeably with "epitope". Antibodies can bind a single epitope of an antigen (monoclonal) or multiple epitopes (polyclonal). In a proteinaceous material, the length of a linear epitope is usually recited as being about 5 to about 7 amino acid residues.

The articles "a" and "an" are used herein to refer to one or to more than one (i.e., to at least one) of the grammatical object of the article. By way of example, "an element" means one element or more than one element.

The word "hydrocarbyl" is used herein as a short hand term for a non-aromatic group that includes straight and branched chain aliphatic as well as alicyclic groups or radicals that contain only carbon and hydrogen. Thus, alkyl, alkenyl and alkynyl groups are contemplated, whereas aromatic hydrocarbons such as phenyl and naphthyl groups, which strictly speaking are also hydrocarbyl groups, are referred to herein as aryl groups or radicals, as discussed hereinafter.

Where a specific aliphatic hydrocarbyl substituent group is intended, that group is recited; i.e., methyl, ethyl, butyl, tert-butyl, hexyl, hexenyl, 2-ethylhexyl, dodecyl ($C_{12}$), octadecyl ($C_{18}$). A particularly preferred hydrocarbyl group is an alkyl group. As a consequence, a generalized, but more preferred substituent can be recited by replacing the descriptor "hydrocarbyl" with "alkyl" in any of the substituent groups enumerated herein.

Although long chain (e.g., $C_{18}$) hydrocarbyl groups are contemplated, examples of shorter ($C_1$-$C_4$) groups are used illustratively here. Such illustrative alkyl radicals include ethyl, n-propyl, isopropyl, n-butyl, isobutyl, sec-butyl, tert-butyl and cyclopropyl. Examples of suitable alkenyl radicals include ethenyl (vinyl), 2-propenyl, 3-propenyl, 1,4-butadienyl, 1-butenyl, 2-butenyl, and 3-butenyl. Examples of alkynyl radicals include ethynyl, 2-propynyl, 1-propynyl, 1-butynyl, 2-butynyl, 3-butynyl, and 1-methyl-2-propynyl.

As a skilled worker will understand, a substituent that cannot exist such as a $C_1$ alkenyl group is not intended to be encompassed by the word "hydrocarbyl", although such substituents with two or more carbon atoms are intended.

Usual chemical suffix nomenclature is followed when using the word "hydrocarbyl" except that the usual practice of removing the terminal **"yl"** and adding an appropriate suffix is not always followed because of the possible similarity of a resulting name to one or more substituents. Thus, a hydrocarbyl ether is referred to as a "hydrocarbyloxy" group rather than a "hydrocarboxy" group as may possibly be more proper when following the usual rules of chemical nomenclature. Illustrative hydrocarbyloxy groups include methoxy, ethoxy, n-propoxy, isopropoxy, allyloxy, n-butoxy, iso-butoxy, sec-butoxy, and tert-butoxy groups.

Ligand: a molecule having a structural region that binds specifically to a particular receptor molecule, usually via electrostatic forces and/or hydrogen bonds. An exemplary ligand is the epidermal growth factor molecule.

The "marker molecule" (antigen or immunogen) can be but need not be expressed on the cell surface, and rather

can be expressed anywhere in the diseased cell. The reason for that is that substantially all of the native cellular proteins of mammals are processed into shorter peptides by the cell and bound extracellularly by class I MHC molecules. Such native proteins are typically so processed during the organism's immaturity, and T cells or other immune cells that may be induced by those native protein portions are eliminated by the organism prior to maturity, resulting in "self protein" tolerance. As a result, except in cases of certain immunological diseases, only foreign peptides or neoantigenic peptides caused by disease such as cancer whose cells result from mutation are recognized as "foreign" and induce an immune response to the MHC-bound peptide.

Further, as to mutations, at times the mutation can be a frame-shift mutation, and an unnatural sequence of amino acids results. The marker molecule can also be a "tumor antigen;" that is, a protein that can be expressed by other cells during embryonic development, for example, but is characteristically expressed much more by tumors than by normal cells. Or the marker molecule can be an oncogene product, for example, an abnormal fusion protein created by a recombination event within tumor cells. The marker molecule can also be the product of an infectious agent such as a virus or bacterium as well.

Peptide/Polypeptide: an oligomer or polymer comprising at least two amino acid residues in which adjacent residues are linked by a peptide bond between the alpha-amino group of one residue and the alpha-carboxyl group of an adjacent residue. The primary structure of a polypeptide has a primary amine group at one terminus and a carboxylic acid group at the other terminus of the polymer. A peptide or polypeptide is depicted herein and usually in the art from left to right and in the direction from amino-terminus to carboxy-terminus. Also, a polypeptide in aqueous solution is usually in one or more zwitterionic forms depending on the pH of the solution. The words "peptide" and "polypeptide" are used interchangeably herein.

Protein: a single polypeptide or set of cross-linked polypeptides comprising more than about 100 amino acid residues. Proteins can have chemical crosslinking, e.g., via disulfide bridges, within the same polypeptide chain or between adjacent polypeptides. When a protein is glycosylated it can be called a glycoprotein. When a protein comprises one or more discrete polypeptide/protein subunits linked together, as by a peptide linkage, amino acid residue sequence, disulfide bridge, and the like, the protein is frequently termed a fusion protein, fusion polypeptide, chimeric fusion, and the like.

Receptor: a biologically active proteinaceous molecule having a structural region that specifically binds to (or with) another molecule (ligand). An exemplary receptor molecule is an antibody combining site or a transmembrane cellular protein molecule involved in intra- or intercellular signaling such as the endothelial growth hormone receptor referred to as EGFR, ERBB and also as HER2, and the like.

The term "residue" is used interchangeably with the phrase amino acid residue. All amino acid residues identified herein are in the natural or L-configuration. In keeping with standard polypeptide nomenclature, [J. Biol. Chem., 243:3557-59 (1969)], abbreviations for amino acid residues are as shown in the following Table of Correspondence.

TABLE OF CORRESPONDENCE

| 1-Letter | 3-Letter | AMINO ACID NAME |
|---|---|---|
| Y | Tyr | L-tyrosine |
| G | Gly | glycine |
| F | Phe | L-phenylalanine |
| M | Met | L-methionine |
| A | Ala | L-alanine |
| S | Ser | L-serine |
| I | Ile | L-isoleucine |
| L | Leu | L-leucine |
| T | Thr | L-threonine |
| V | Val | L-valine |
| P | Pro | L-proline |
| K | Lys | L-lysine |
| H | His | L-histidine |
| Q | Gln | L-glutamine |
| E | Glu | L-glutamic acid |
| Z | Glx | L-glutamic acid or L-glutamine |
| W | Trp | L-tryptophan |

(continued)

| TABLE OF CORRESPONDENCE | | |
|---|---|---|
| 1-Letter | 3-Letter | AMINO ACID NAME |
| R | Arg | L-arginine |
| D | Asp | L-aspartic acid |
| N | Asn | L-asparagine |
| B | Asx | L-aspartic acid or L-asparagine |
| C | Cys | L-cysteine |

The present invention has several benefits and advantages.

A salient benefit of the invention is that the combination immunization provides synergistic results in inhibiting diseased cell growth.

An advantage of the invention is that the combination immunization provides T cell help that virus- and bacteria-free vaccines have often lacked.

Another benefit that the invention provides is that those skilled in the art have been finding, studying and publishing formulas of disease-related immunogens that have been ultimately unsuccessful since the early 1980's but can now be successfully put to use.

Still further benefits and advantages of the invention will be apparent to the skilled worker from the disclosures that follow.

## DETAILED DESCRIPTION OF PREFERRED EMBODIMENTS

The present invention provides an immunizing pharmacological composition for use in inhibiting the growth of cancerous or pathogen-infected cells as defined in the claims. Those diseased cells express one or more marker molecules that are absent on cells of the same type that are free of the disease or are present in the disease-free cells in significantly reduced numbers compared to the diseased cells. Workers skilled in the art have published numerous articles and reviews discussing marker molecules that are present in diseased cells in amounts significantly greater than the amount present in disease-free cells and methods for determining those differences. See, for example, Kim et al., BMB Rep 50(6):285-298 (2017) and the citations therein. In addition, techniques such as quantitative western blots performed with optical density scans or radioactivity detection means and other means are well known in the art. Cells "of the same type" are disease-free cells from the same organ and tissue as the diseased cells.

A preferred member of the Diprovocim family of compounds of Formula **V** is a compound that corresponds in structure to structural Formula **I,** below, in which **-A** = -C(O)NH-$R^4$, and the depicted

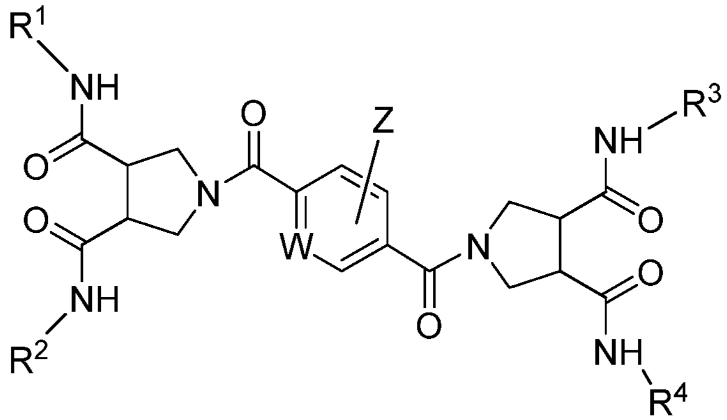

I

$R^{1-4}$, W and Z moieties are as described above.

In one more preferred compound of Formula **I,** at least one member of substituent pair $R^1$ and $R^3$ (either $R^1$ or $R^3$) or pair $R^1$ and $R^2$ (either $R^1$ or $R^2$) is a trans-2-phenylcyclopropyl or trans-2-(4-fluorophenyl)cyclopropyl group. It is also preferred that at least one member of substituent pair $R^1$ and $R^3$ or pair $R^1$ and $R^2$ has the (*1S,2R*) configuration of a trans-2-phenylcyclopropyl or trans-2-(4-fluorophenyl)cyclopropyl group.

More preferably, at least three substituents of $R^1$, $R^2$, $R^3$ and $R^4$ have the *(1S,2R)* configuration of a trans-2-phenylcyclopropyl or trans-2-(4-fluorophenyl)cyclopropyl group. More preferably still, each of $R^1$, $R^2$, $R^3$ and $R^4$ has the (1*S,*2*R*) configuration of a trans-2-phenylcyclopropyl or a trans-2-(4-fluorophenyl)cyclopropyl group.

In another preference, each depicted pyrrolidinyldicarboxamido group has the (*S*,*S*) configuration and each depicted $R^{1-4}$ substituent is a trans-2-phenylcyclopropyl, a trans-2-(4-fluorophenyl)cyclopropyl group or a mixture thereof, and bonds to the cyclopropyl moiety have a *(1S,2R)* configuration.

In another aspect, up to two of $R^{1-4}$ in a compound of Formula **I** or one of its sub-generic formulas can be a $c_2$-$c_{18}$ hydrocarbyl group. It is preferable that the hydrocarbyl group be an alkyl group and have a length of 4 to about 16 carbon atoms, and more preferably still, about 6 to about 10 carbon atoms. Straight chained hydrocarbyl groups are also preferred, although up to two methyl and ethyl group substituents or both can be present as can an carbocyclic ring, and also one or two double or triple bonds. Specific $c_2$-$c_{18}$ hydrocarbyl groups are discussed previously in the discussion of the use of the word hydrocarbyl.

As is seen from Formula **I,** each molecule contains at least one, and preferably two, 3,4-pyrrolidinyldicarboxyl groups. The carboxyl groups are bonded to amine-terminated $R^1$, $R^2$, $R^3$ and $R^4$ substituents, forming four (or three) amido linkages. The two pyrrolidinyldicarboxyl groups can also therefore also be referred to as two pyrrolidinyldicarboxamido groups.

Substituents bonded to the carboxyl groups of a pyrrolidinyldicarboxyl group can be in a *cis* or *trans* conformation, that is the two substituents can both project above or below the plane of the depicted ring (*cis*), or one can project above that plane and the other substituent project below (*trans*). *A cis*-disubstituted pyrrolidinyldicarboxyl group with two identical substituents has a symmetric configuration and does not have enantiomeric forms. A *trans*-disubstituted pyrrolidinyldicarboxyl group with those same two identical substituents has an asymmetric (chiral) configuration and has enantiomeric forms. The two chiral configurations are referred to as (*S*,*S*) and (*R,R*), and are shown below. It is

(*S,S*)
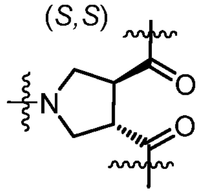
(*R,R*)
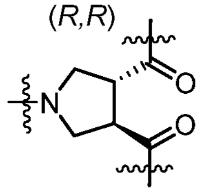

preferred that at least one, and more preferably both 3,4-pyrrolidinyldicarboxyl groups have the (*S*,*S*) configuration.

On the other hand, when **-A** of Formula **V** is hydrido and one of $R^{1-3}$ is a $c_2$-$c_{18}$ hydrocarbyl group, and more preferably, a $C_{10}$-$C_{16}$ hydrocarbyl group. A preferred compound corresponds in structure to Formula **Va,** below, in which the depicted $R^{1-3}$, W

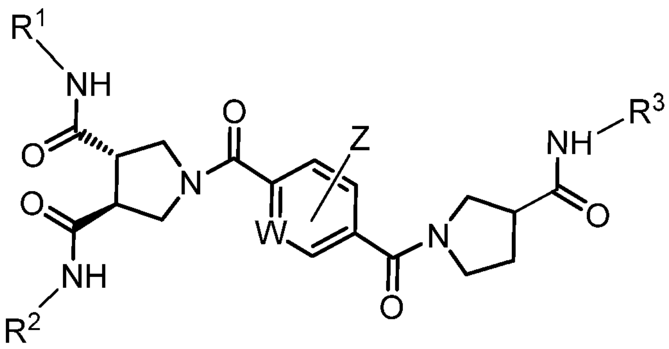
**Va**

and Z moieties are as described above. The depicted -C(O)NH-$R^3$ group can be in either the *R* configuration, the S configuration or present as a mixture of both configurations. The compounds with the *S* configuration are slightly more active and therefore more preferred.

In a compound of Formula **Va,** the number of carbon atoms of a $R^3$ group here is preferably 2 to 18, and more preferably 10 to 16 carbon atoms. This hydrocarbyl group is also more preferably an alkyl group that is a straight chained substituent although methyl and ethyl branches can be tolerated as can double and/or triple bonds in the chain. Cyclic hydrocarbyl substituent compounds and carbocyclic ring-containing substituents can also be utilized.

It is also preferred that W be CH. Structural Formula **Ia,** shown below, incorporates several of the above preferences.

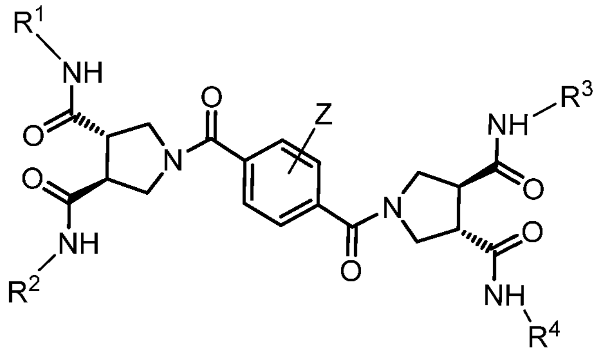

**Ia**

Structural formulas of currently most preferred compounds of Formula **Ia** in which -Z is hydrido (-H) are shown below as compounds of Formulas **A, B, C, D, E, F, G, H,** and **I.** Diprovocim numbers from WO 2018005812 are included for reference.

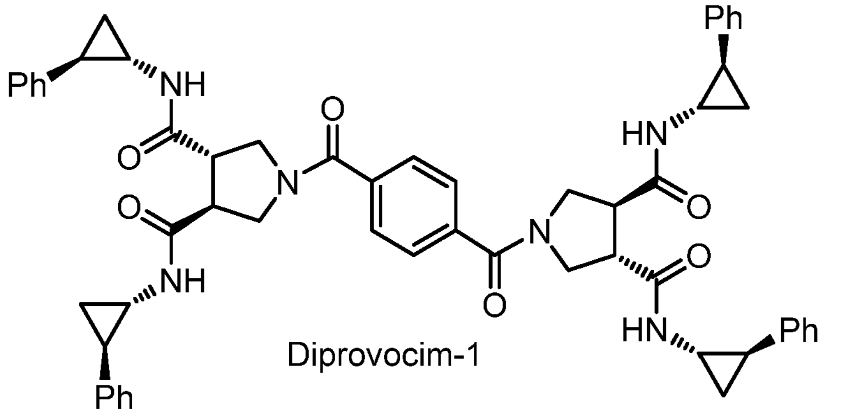

Diprovocim-1

**A**

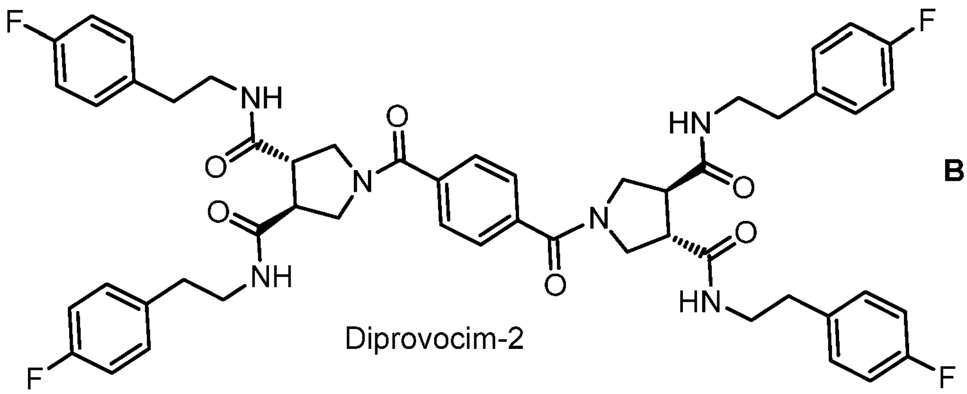

Diprovocim-2

**B**

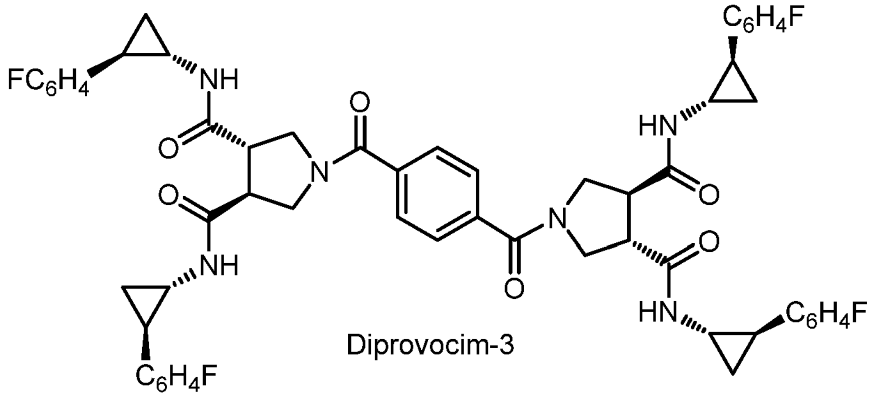

Diprovocim-3

**C**

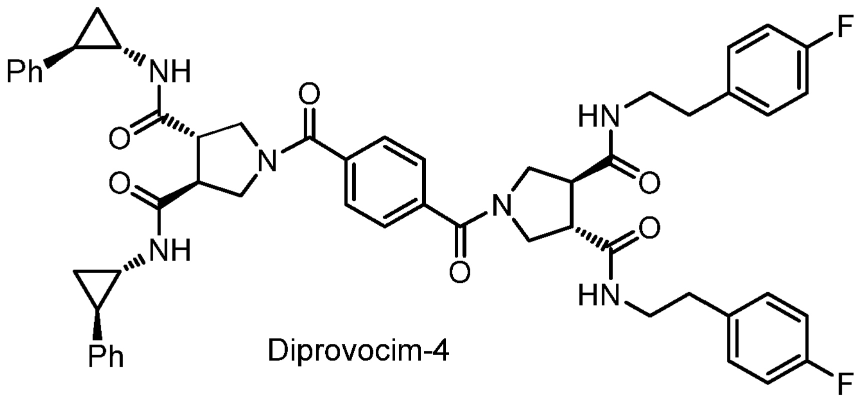

Diprovocim-4

D

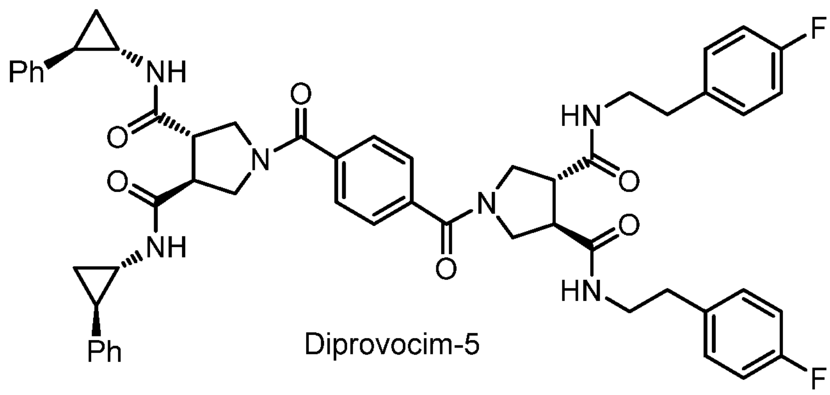

Diprovocim-5

E

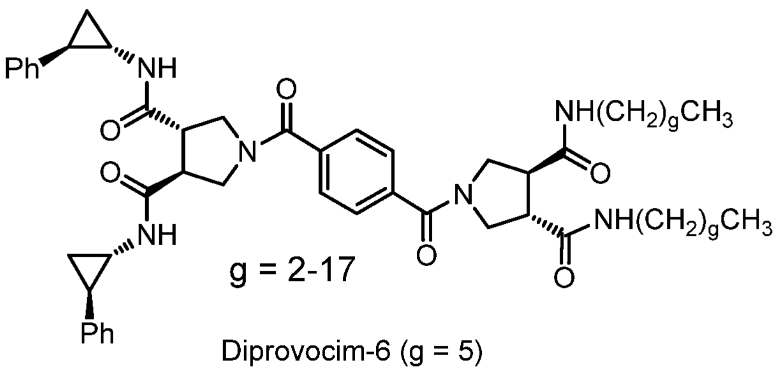

g = 2-17

Diprovocim-6 (g = 5)

F

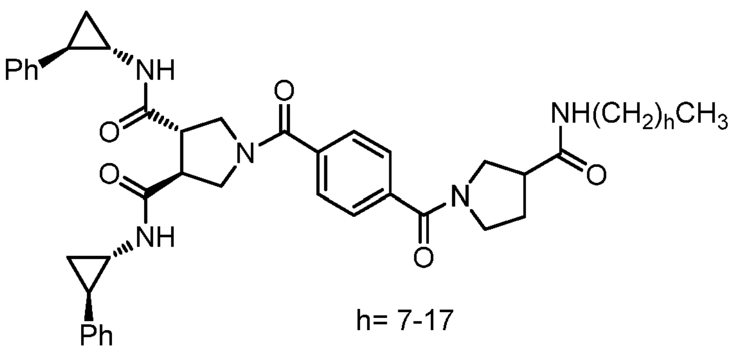

h= 7-17

G

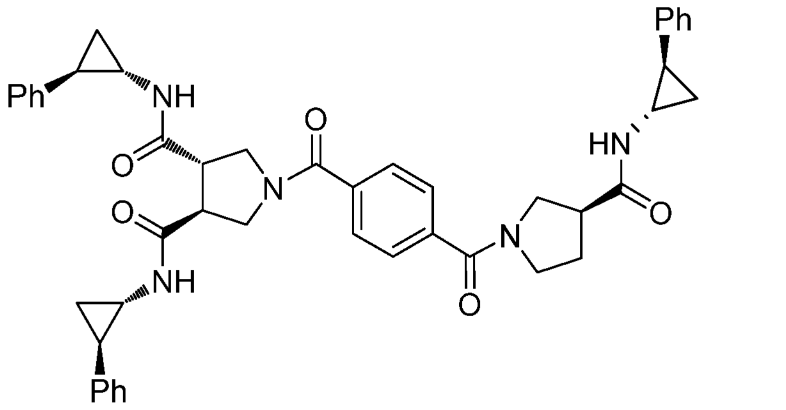

H

and

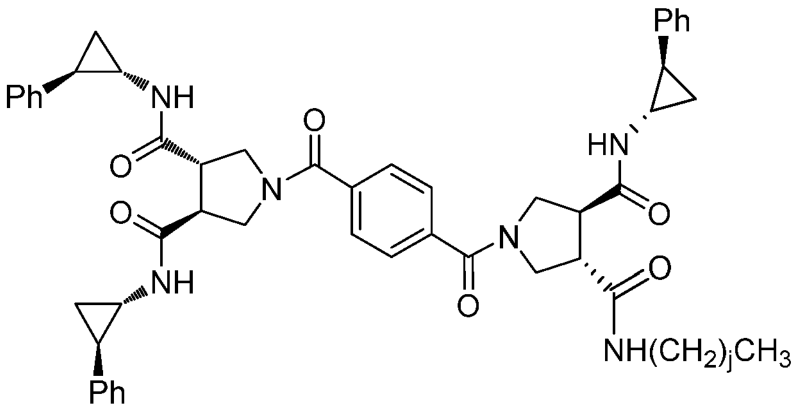

I

j = 7-17

.

For a Compound of Formula **F,** "g" in both depicted amido groups may be 2-17 methylene groups; preferably "g" is the same number (length) of 5 to 9 methylene groups for maximal activity against both human THP-1 cells and mouse macrophages, with g = 5 providing the most active compound for both cell types. For a Compound of Formula **G,** "h" in the depicted amido group is 1 to 17, preferably 7 to 17, and more preferably 9 to 15. For a Compound of Formula **I,** "j" in the depicted amido group is 1 to 17, preferably 3 to 11, and more preferably 5 to 9.

A contemplated immune checkpoint inhibitor is typically an intact antibody or the paratope-containing portion of an antibody. Such a contemplated antibody or antibody paratope-containing portion is preferably a monoclonal humanized, chimeric or human antibody. Illustrative checkpoint US FDA approved checkpoint inhibitors include Keytruda® (anti-PD-1), Yervoy® (anti-CTLA-4), Tecentriq® (anti-PD-L1), Opdivo® (anti-PD-1)], and Imfinzi® (anti-PD-L1)]. CTLA-4 itself binds to proteins B7-1 and B7-2 to inhibit T cell activity. Anti-B7-1 and anti-B7-2 paratope-containing molecules can also be used to block the CTLA-4/B7-1+B7-2 interaction, thereby providing checkpoint inhibitor activity much as do antibodies to either of the binding pair PD-1 and PD-L1.

Marker molecules are discussed in detail hereinafter. These materials are typically proteinaceous and can be the whole protein or an immunogenic portion of the protein.

When used in a method of inhibiting the growth of diseased cells in a mammal, an adjuvant-sufficient amount of a Diprovocim compound, a T cell-stimulating amount of an immune checkpoint inhibitor, and an immunizing amount of an antigenic (immunogenic) disease-related marker molecule are administered to contact host mammal cells. Those three components can be administered together, but it is preferred that the checkpoint inhibitor be administered separately from the Diprovocim and the antigen (immunogen) that can usually be administered together. Preferably, the checkpoint inhibitor is administered intravenously (IV), whereas the Diprovocim and antigen are administered together in an immunizing pharmaceutical composition intramuscularly (IM) or subcutaneously (SC).

Those administrations can be made within the span of a few minutes, hours, days or weeks. Many checkpoint inhibitors are antibodies or paratope-containing antibody portions that typically have an *in vivo* terminal half-life on the order of about 2 to about 4 weeks. [See, product label, Section 12.3 for Keytruda® (anti-PD-1), Yervoy® (anti-CTLA-4), Tecentriq® (anti-PD-L1), Opdivo® (anti-PD-1)], and Imfinzi® (anti-PD-L1)]. Thus, a checkpoint inhibitor can be administered prior to, coincidently with or a few days after administration of the Diprovocim and immunogen.

Each of the components can be administered a plurality of times during a course of treatment. The use of multiple administrations is illustrated herein.

A Diprovocim compound is administered in an adjuvant-sufficient amount. In the studies illustrated elsewhere herein, Diprovocim-1 **(A)** used illustratively herein acted as a robust *in vivo* adjuvant or TLR1/TLR2 agonist that evoked a potent TLR2-dependent adjuvant activity *in vivo* in mice at about 0.25 to about 5 mg/kg (i.m.) when co-injected with an immunogen in an immunizing pharmaceutical composition by an intramuscular route. An adjuvant-sufficient amount can be readily determined for mammals of greater weight by techniques well known in the art. In addition, Diprovocim-1 did not display the overt toxicity that is characteristic of LPS administration when used as an adjuvant.

A checkpoint inhibitor is typically utilized in an amount discussed in the product label. Illustrative dosage and administrations include the following using melanoma as exemplary diseased cell for use in the recited amounts: Keytruda®-melanoma: 2 mg/kg every 3 weeks; Yervoy®-- adjuvant melanoma: 10 mg/kg administered intravenously over 90 minutes every 3 weeks for 4 doses, followed by 10 mg/kg every 12 weeks for up to 3 years or until documented disease recurrence or unacceptable toxicity; Tecentriq®--administer 1200 mg as an intravenous infusion over 60 minutes every 3 weeks; and Opdivo®--unresectable or metastatic melanoma 240 mg every 2 weeks.

An immunizing amount of an antigenic (immunogenic) disease-related marker molecule depends upon the immunogenicity of the marker used. The selection of peptides immunogenic for B cells and T cells is well known in the art and will not be gone into here. Many such useful peptides have been reported in the art but were not as successful formulated as vaccines as desired, presumably because of a lack of T cell help. It is believed that the three-part immunizing pharmaceutical composition overcomes that deficiency.

Many small disease-related marker molecules such as peptides having a length of about 5 to about 20 residues are themselves poorly immunogenic, and are often best utilized as haptens chemically linked to a carrier molecule. Illustrative proteinaceous carrier molecules include keyhole limpet hemocyanin (KLH), hepatitis B surface molecule (HBsAg), the hepatitis B core (capsid; HBcAg), ovalbumin, bovine serum albumin, bovine gammaglobulin and human gammaglobulin have been used as a hapten carrier, as have many other molecules have been used in the literature.

A contemplated disease-related marker molecule is present in and/or on diseased cells. Diseased cells are typically cancerous or pathogen-infected.

Disease-related marker molecules or portions thereof useful as immunogens present in and/or on solid tumor cells, include cancer stem cell (CSC) markers that are rarely expressed on normal tissue cells are listed below. [Kim et al., BMB Rep 50(6):285-298 (2017).]. Exemplary CSC markers that are largely absent in normal (disease-free) cells and present in diseased cells include CD96, CD20, DLL4, CD55, TIM-3, CXCR1, CD54, CD114, LGR5, CD105, CD56, CD13, CD271, CD34, CXCR4, CD26, CD117, CD10, CD146, Notch2, CD49f, CD24, ABCG2, PODXL-2, Cripto-1, CD326, CD90, CD133, SSEA1, TRA-1-81, TRA-1-60, SSEA4, SSEA3, CD151, CD340 and CD44.

Exemplary disease-related marker molecules are typically present in and/or on solid tumor cells. Illustrative solid tumors include osteosarcoma cells, Kaposi's sarcoma cells, melanoma cells, prostate cancer cells, glioblastoma cells, small cell lung carcinoma cells, breast cancer cells, liver cancer cells, colon cancer cells, ovarian cancer cells, renal cancer cells, gastric cancer cells, neuroblastoma cells, pancreatic cancer cells, and Hodgkin's lymphoma cells.

A contemplated disease-related marker molecule or portion thereof can also be present in and/or on pathogen-infected cells. Illustrative infecting pathogens include one or more of a virus, bacterium, fungus and unicellular parasite.

Illustrative viruses include influenza, hepatitis viruses A, B, C and D, herpes viruses such as Varicella zoster (chickenpox), Herpes simplex 1 and 2 (HSV1 and HSV2), human papilloma virus (HPV), and the like. Illustrative bacterial pathogens include *E. coli, E. faecalis, S. aureus,* and the like. An illustrative unicellular parasite is the malaria sporozoite of *P. falciparum, P. vivax, P. bergeii* or *P. yoelli.*

Illustrative proteinaceous immunogens include the following disease-related marker molecule peptides that are listed below with a citation to their publication source.

US Patent No. 6,942,866

Malarial B Cell Epitopes

| *P. falciparum* | |
|---|---|
| $(NANP)_4$ | SEQ ID NO: 1 |
| $NANPNVDP(NANP)_3NVDP$ | SEQ ID NO: 2 |
| $NANPNVDP(NANP)_3$ | SEQ ID NO: 3 |
| $(NANP)_3NVDPNANP$ | SEQ ID NO: 4 |
| $NANPNVDP(NANP)_3NVDPNANP$ | SEQ ID NO: 5 |
| $NPNVDP(NANP)_3NV$ | SEQ ID NO: 6 |

(continued)

| *P. falciparum* | |
|---|---|
| NPNVDP$(NANP)_3$NVDP | SEQ ID NO: 7 |
| NPNVDP$(NANP)_3$NVDPNA | SEQ ID NO: 8 |
| NVDP$(NANP)_3$NV | SEQ ID NO: 9 |
| NVDP$(NANP)_3$NVDP | SEQ ID NO: 10 |
| NVDP$(NANP)_3$NVDPNA | SEQ ID NO: 11 |
| DP$(NANP)_3$NV | SEQ ID NO: 12 |
| DP$(NANP)_3$NVDP | SEQ ID NO: 13 |
| DP$(NANP)_3$NVDPNA | SEQ ID NO: 14 |
| *P. vivax* | |
| DRAAGQPAGDRADGQPAG ANGAGNQPGANGAGDQPGA- | SEQ ID NO: 15 |
| NGADNQPGANGADDQPG | SEQ ID NO: 16 |
| ANGAGNQPGANGAGDQPG | SEQ ID NO: 17 |
| ANGADNQPGANGADDQPG | SEQ ID NO: 18 |
| ANGAGNQPGANGADNQPG | SEQ ID NO: 19 |
| ANGADNQPGANGADDQPG | SEQ ID NO: 20 |
| APGANQEGGAAAPGANQEGGAA | SEQ ID NO: 21 |
| *P. bergeii* | |
| $(DP_4NPN)_2$ | SEQ ID NO: 22 |
| *P. yoelli* | |
| $(QGPGAP)_4$ | SEQ ID NO: 23 |

Malarial Universal T Cell Epitope

| *P. falciparum* | |
|---|---|
| GIEYLNKIQNSLSTEWSPCSVT | SEQ ID NO: 24 |
| *P. vivax* | |
| YLDKVRATVGTEWTPCSVT | SEQ ID NO: 25 |
| *P. yoelli* | |
| EFVKQISSQLTEEWSQCSVT | SEQ ID NO: 26 |

US patent No. 8,017,127

Influenza A M2 Protein B Cell Epitopes

As is noted in US Patent No. 8,017,127, the M2 protein is expressed in cells infected by the influenza A strains. The N-terminal residues 1-24 of the M2 protein extends through the infected cell's membrane. That extracellular portion of the protein is referred to as M2e. As a consequence, use of the influenza A extracellular M2e portion of that protein as the immunogenic marker can provide protection from all of the influenza strains. Thus, the yearly changes in influenza vaccine selection can be avoided.

| Sequence | SEQ ID NO |
|---|---|
| SLLTEVETPIRNEWGCRCNGSSD | 27 |
| SLLTEVETPIRNEWGCRCNDSSD | 28 |
| SLLTEVETPIRNEWGARANDSSD | 29 |
| SLLTEVETPIRNEWGSRSNDSSD | 30 |

(continued)

| Sequence | SEQ ID NO |
|---|---|
| SLLTEVETPIRNEWGSRCNDSSD | 31 |
| SLLTEVETPIRNEWGCRSNDSSD | 32 |
| SLLTEVETPIRNEWGCRANDSSD | 33 |
| SLLTEVETPIRNEWGARCNDSSD | 34 |
| MSLLTEVETPIRNEWGCRCNDSSD | 35 |
| MSLLTEVETPIRNEWGSRSNDSSD | 36 |
| MGISLLTEVETPIRNEWGCRCND-SSDELLGWLWGI | 37 |
| MSLLTEVETPIRNEWGARANDSSD | 38 |
| MSLLTEVETPIRNEWGCRANDSSD | 39 |
| MSLLTEVETPIRNEWGARCNDSSD | 40 |
| MSLLTEVETPIRNEWGCRSNDSSD | 41 |
| MSLLTEVETPIRNEWGSRCNDSSD | 42 |
| SLLTEVETPIRNEWGSRSNDSSDSLL-TEVETPIRNEWGSRSNDSSD | 43 |
| SLLTEVETPIRNEWGSRSNDSSDSLL-TEVETPIRNEWGSRSNDSSDSLL-TEVETPIRNEWGCRCNDSSD | 44 |
| SLLTEVETPIRNEWGARANDSSDSLL-TEVETPIRNEWGCRCNDSSD | 45 |
| SLLTEVETPIRNEWGARANDSSDSLL-TEVETPIRNEWGARANDSSDSLL-TEVETPIRNEWGCRCNDSSD | 46 |
| EVETPIRNEWGSRCNDSSD | 47 |
| EVETPIRNEWGSRCNDSSDEVET-PIRNEWGSRCNDSSD | 48 |
| EVETPIRNEWGSRCNDSSDEVET-PIRNEWGSRCNDSSDEVE-TPIRNEWGCRCNDSSD | 49 |
| SLLTEVETPIRNEWGSRSNDSSDSLL-TEVETPIRNEWGSRSNDSSDSLL-TEVETPIRNEWGSRSNDSSDSLL-TEVETPIRNEWGCRCNDSSD | 50 |
| SLLTEVETPIRNEWGSRSNDSSDSLL-TEVETPIRNEWGSRSNDSSDSLL-TEVETPIRNEWGSRSNDSSDSLL-TEVETPIRNEWGSRSNDSSDSLL-TEVETPIRNEWGCRCNDSSD | 51 |
| SLLTEVETPTRNEWGCRCNDSSD | 52 |
| SLLTEVETPTRNGWGCRCNDSSD | 53 |
| SLLTEVETPIRNEWECRCNGSSD | 54 |
| SLLTEVETPTKNEWECRCNDSSD | 55 |
| SLLTEVETPTRNGWECKCSDSSD | 56 |
| SLLTEVDTLTRNGWGCRCSDSSD | 57 |
| SLLTEVETLTRNGWECKCRDSSD | 58 |
| Influenza B Protein | |
| NNATFNYTNVNPISHIR | 59 |

US Patent No. 4,599,231

Hepatitis B Virus Surface Antigen

The hepatitis B virus surface antigen (HBsAg) provides both B cell and T cell polypeptide epitopes. A number of each epitope type as disclosed in US Patent No. 4,599,231 are set out below in the table along with their peptide denominations, and parenthesized sequence position from the N-terminus, as recited in that patent based on DNA from an *ayw* donor (P49) and an *adw* donor (P72 and P73).

| Sequence | SEQ ID NO |
|---|---|
| B Cell Epitope | |
| P49 (110-137) | |
| FPGSSTTSTGPCRTCMTTAQGTSMYPSC | 60 |
| P49a (125-137) | |
| MTTAQGTSMYPSC | 61 |
| P72 (125-137) | |
| IPGSTTTSTGPCKTCTTPAQGNSMFPSC | 62 |
| P72a (125-137) | |
| TTPAQGNSMFPSC | 63 |
| P73 (107-137) | |
| CPLIPGSTTTSTGPCKTCTTPAQGNSMFPSC | 64 |
| T Cell Epitope | |
| P1 (48-81) | |
| CLGQNSQSPTSNHSPTSCPPTCPGYRWMCLRRF | 65 |
| P5 (38-52) | |
| SLNFLGGTTVCLGQN | 66 |
| P5a (74-52) | |
| VCLGQN | 67 |
| P6 (95-109) | |
| LIFLLVLLDYQGMLPVCPL | 68 |

U.S. Patent No. 5,180,806

Human Papilloma Virus (HPV) Marker Peptides

Papillomaviruses induce benign, dysplastic and malignant hyperproliferations of skin or mucosal epithelium. More than 50 types (strains) of human papillomavirus (HPV) have been identified. In humans, different papillomavirus types are known to cause distinct diseases. For example, HPV types 1 and 2 cause common warts, and types 6 and 11 cause condylomas and genital flat warts. In contrast, HPV types 16, 18 and 33 are carried in a majority of cervical cancers and do not cause the usual condyloma, but rather persist diffusely in the cervical endothelium exhibiting only minimal pathologic changes. It is thought that the HPV types associated with cervical cancer are maintained in a latent state in cervical endothelium tissues for years after initial infection and then progress in some cases to cause cervical cancer.

U.S. Patent No. 5,180,806 discloses several peptide sequences that induce the production of antibodies. Illustrative peptide markers of type 16-related HPV sequences disclosed in US Patent No. 5,180,806 are set out below as illustrative. That patent also discloses peptide sequences from type 18 and type 33, as well as sequences encoded by the E2 ORF of HPV types 6, 11, 18 and 33.

HPV Type 16-related polypeptides

| Sequence | SEQ ID NO |
|---|---|
| MADPAGTNGEEGTGC | 69 |

(continued)

| Sequence | SEQ ID NO |
|---|---|
| HEDEDKENDGDSLPTC | 70 |
| RPFKSNKSTCC | 71 |
| CCDWCIAAFGLTPSI | 72 |
| TYDSEWQRDQFLSQVKIPC | 73 |
| HKSAIVTLTYDSEWQRDQC | 74 |
| CINCQKPLCPEEKQRH | 75 |

A contemplated immunizing composition also typically contains pharmaceutically acceptable salts, buffers and the like excipients that collectively are referred to as pharmaceutically (or physiologically) acceptable diluents or carriers as compared to those that can be present in a composition that is not intended for pharmaceutical use, as in an *in vitro* assay. These compositions are discussed in further detail hereinafter.

A Diprovocim compound useful herein can be provided for use by itself, or as a pharmaceutically acceptable salt. Exemplary salts useful for a contemplated compound include but are not limited to the following: sulfate, hydrochloride, hydro bromides, acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxyethanesulfonate, lactate, maleate, methanesulfonate, nicotinate, 2-naphthalenesulfonate, oxalate, palmoate, pectinate, persulfate, 3-phenyl-propionate, picrate, pivalate, propionate, succinate, tartrate, thiocyanate, tosylate, mesylate and undecanoate. Salts of the carboxylate group include sodium, potassium, magnesium, calcium, aluminum, ammonium, and the many substituted ammonium salts.

The reader is directed to Berge, J. Pharm. Sci. 1977 68(1):1-19 for lists of commonly used pharmaceutically acceptable acids and bases that form pharmaceutically acceptable salts with pharmaceutical compounds.

In some cases, the salts can also be used as an aid in the isolation, purification or resolution of the compounds of this invention. In such uses, the acid or base used and the salt prepared need not be pharmaceutically acceptable.

A contemplated immunizing pharmaceutical composition contains an adjuvant-effective amount of a Compound of Formula **V** or a pharmaceutically acceptable salt thereof dissolved or dispersed in a physiologically (pharmaceutically) acceptable carrier along with the immunogenic marker. Such a composition can be administered to mammalian cells *in vitro* as in a cell culture to contact those cells, or the cells can be contacted *in vivo* as in a living, host mammal in need. As is seen from the data herein, a contemplated Diprovocim compound present at femtomolar to nanomolar amounts provides an adjuvant effect in *in vivo* and in *in vitro* assay studies.

When used as a vaccine adjuvant, a Compound of Formula **V** is preferably administered together with the selected marker immunogen. Both components are preferably present together in a single immunizing pharmaceutical composition as noted above. However, the two ingredients can be present in separately administered immunizing pharmaceutical compositions, and those separate compositions can be administered up to about one to about two hours apart. It is preferred when two separate compositions are administered, that they be administered as close together in time as possible.

In another embodiment, some or all of the Diprovocim compound utilized can be chemically bonded to the immunizing marker compound. That chemical bond can be formed using the Z substituent shown in Formula **V** as where a Z substituent that includes a carboxyl group can be bonded to an amino group of an immunogenic peptide marker compound. Alternatively, where an immunogenic marker compound is utilized as a hapten bonded to a carrier molecule, the Diprovocim compound can also be chemically bonded to the same carrier molecule.

A contemplated immunizing pharmaceutical composition is typically administered *in vivo* to a subject in need thereof a plurality of times within one month, such as daily or weekly, and can be administered over a period of several months to several years. More usually, a contemplated composition is administered a plurality of times over a course of treatment.

A contemplated immunizing pharmaceutical composition is preferably adapted for parenteral administration. Thus, an immunizing pharmaceutical composition is preferably in liquid form when administered, and most preferably, the liquid is an aqueous liquid, although other liquids are contemplated as discussed below, and a presently most preferred composition is an injectable preparation.

Thus, injectable preparations, for example, sterile injectable aqueous or oleaginous solutions or suspensions can be formulated according to the known art using suitable dispersing or wetting agents and suspending agents. The sterile injectable preparation can also be a sterile injectable solution or suspension in a nontoxic parenterally acceptable diluent or solvent, for example, as a solution in 1,3-butanediol. Among the acceptable vehicles and solvents that can be employed are water, Ringer's solution, and isotonic sodium chloride solution, phosphate-buffered saline.

Other liquid pharmaceutical compositions include, for example, solutions suitable for parenteral administration. Sterile water solutions of a Compound of Formula V or sterile solution of a Compound of Formula V in solvents comprising water, ethanol, or propylene glycol are examples of liquid compositions suitable for parenteral administration. In some aspects, a contemplated Compound of Formula V is provided as a dry powder that is to be dissolved in an appropriate liquid medium such as sodium chloride for injection prior to use.

In addition, sterile, fixed oils are conventionally employed as a solvent or suspending medium. For this purpose any bland fixed oil can be employed including synthetic mono- or diglycerides. In addition, fatty acids such as oleic acid find use in the preparation of an injectable composition. Dimethyl acetamide, surfactants including ionic and non-ionic detergents, polyethylene glycols can be used. Mixtures of solvents and wetting agents such as those discussed above are also useful. Sterile solutions can be prepared by dissolving the active component in the desired solvent system, and then passing the resulting solution through a membrane filter to sterilize it or, alternatively, by dissolving the sterile compound in a previously sterilized solvent under sterile conditions.

A mammal in having diseased cells in need of treatment (a subject) and to which a pharmaceutical composition containing at least a Compound of Formula V and a immunogenic marker compound is administered can be a primate such as a human, an ape such as a chimpanzee or gorilla, a monkey such as a cynomolgus monkey or a macaque, a laboratory animal such as a rat, mouse or rabbit, a companion animal such as a dog, cat, horse, or a food animal such as a cow or steer, sheep, lamb, pig, goat, llama or the like.

Preferably, the pharmaceutical composition is in unit dosage form. In such form, the composition is divided into unit doses containing appropriate quantities of the Diprovocim and immunogen. The unit dosage form can be a packaged preparation, the package containing discrete quantities of the preparation, for example, in vials or ampules.

**Results**

**Diprovocim induces cytokine production in both human and mouse cells**

From a chemical library containing approximately 100,000 members, a class of compounds was identified with bilateral symmetry capable of activating TNF biosynthesis in PMA-differentiated human THP-1 myeloid cells. The initial members of the class emerged from an undisclosed compound sublibrary designed to promote cell surface receptor dimerization (18).

Diprovocim-1, below, was developed from this class after extensive structure-activity

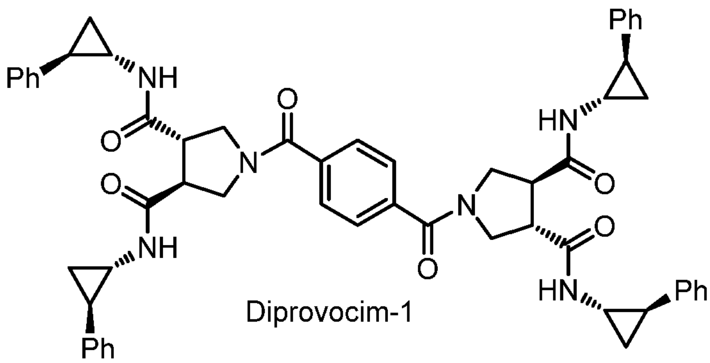

Diprovocim-1

relationship (SAR) studies. It induced dose-dependent TNF production by THP-1 cells ($EC_{50}$ 110 pM) and human PBMC ($EC_{50}$ 875 pM) (Fig. 0A and Fig. 1B), and by mouse peritoneal macrophages ($EC_{50}$ 1.3 nM) and bone marrow-derived dendritic cells (BMDC) ($EC_{50}$ 6.7 nM) (Fig. 0C and Fig. 1D). In addition to TNF, Diprovocim-1 induced IL-6 production by mouse BMDC (Fig. 0E). However, Diprovocim-1 failed to stimulate type I IFN production by mouse peritoneal macrophages (Fig. 7). The other numbered Diprovocims studied and those defined by structural Formula **I** also share these activities.

**Diprovocim targets TLR1/TLR2 and activates downstream MAPK and NF-xB signaling pathway**

To determine the molecular target of the Diprovocims, effects were analyzed on peritoneal macrophages from wild type C57BL/6J mice and C57BL/6J mice deficient in various TLR signaling components. Induction of TNF by Diprovocim was completely absent in TLR1- or TLR2-deficient macrophages, but not in TLR-6 deficient macrophages (Fig. 1A). Diprovocim activity was also dramatically reduced in macrophages from MyD88-, TIRAP-, and IRAK4-deficient cells (Fig. 1A). These data suggested that Diprovocim targets the mouse TLR1/TLR2 heterodimer.

TLR1 or TLR2 antibody significantly reduced the effect of Diprovocim on THP-1 cells, indicating that human TLR1/TLR2 is also a target of Diprovocim molecules (Fig. 1B). Diprovocim induced phosphorylation of IKKα, IKKβ, p38, JNK, and ERK, as well as degradation of IκBα in THP-1 cells and mouse peritoneal macrophages, indicating that Diprovocims activate conventional TLR1/TLR2 signaling, including MAPK and canonical NF-κB signaling (Fig. 1C and Fig. 2D).

**Diprovocim exhibits adjuvant activity *in vivo***

Intramuscular immunization of wild type mice with ovalbumin (OVA) plus either alum or Diprovocim induced similar levels of serum OVA-specific IgG, which were highly elevated compared to levels induced by immunization with OVA plus vehicle (Fig. 2A-Fig. 3C). Whereas immunization with OVA+alum induced primarily the Th2-related Ig subclass IgG1, OVA+Diprovocim induced both IgG1 and the Th1-related IgG2b (Fig. 2B and Fig. 3C).

Dendritic cells (DCs) purified from draining lymph nodes and spleens 24 hours after immunization of mice with OVA+Diprovocim activated OT-I CD8 T cells co-cultured with them, as evidenced by CD69 upregulation on the OT-I cells (Fig. 2D). In contrast, DCs from mice immunized with OVA+vehicle failed to induce CD69 expression on OT-I CD8 T cells (Fig. 2D). This finding suggests that Diprovocims activate antigen cross-presentation by DCs and cross-priming of CD8 T cells *in vivo.*

To further investigate whether cross-priming stimulated by a Diprovocim results in the development of killing ability by CD8+ T cells *in vivo,* a CTL killing assay was performed. Fluorescent marker-labeled and OVA peptide (residues, 257-263)-pulsed target cells were injected i.v. into mice immunized with OVA+Diprovocim and the number of live target cells was measured by flow cytometry two days later. About 70% of target cells had been eliminated in mice immunized with OVA+Diprovocim, compared to about 10% in mice immunized with OVA+vehicle (Fig. 2E). These data demonstrate that Diprovocim exhibits adjuvant activity in antigen-specific antibody production and CTL killing, which was abrogated in TLR1- and/or TLR2-deficient mice (Fig. 2 A-C, F and G).

**Complete inhibition of B16 tumor growth by combined checkpoint blockade and anti-cancer vaccine adjuvanted with Diprovocim**

The adjuvant activity of Diprovocim was examined in preventive immunization of wild type mice against B16 melanoma expressing OVA (B16-OVA) (Fig. 3A). Mice were injected i.m. distal to the tumor cell injection site with OVA with or without Diprovocim on the same day but prior to inoculation with B16-OVA cells. Tumor growth rates and survival times were similar for mice immunized with vehicle alone, Diprovocim alone, or OVA alone (Fig. 3B and Fig. 4C). Relative to OVA alone, immunization with Diprovocim+OVA modestly but significantly slowed tumor growth rate but failed to prolong survival; a similar effect was observed with OVA immunization combined with anti-PD-L1 treatment (Fig. 3B and Fig. 4C).

Strikingly, although without effect by itself (Fig. 8A and Fig 8B) when anti-PD-L1 treatment was added to Diprovocim+OVA immunization, there was complete inhibition of tumor growth and 100% survival through eight weeks of observation (Fig. 3B and Fig. 4C). This dramatic antitumor effect was dependent on OVA immunization since Diprovocim alone combined with anti-PD-L1 treatment had no effect on tumor growth or mouse survival (Fig. 3D and Fig. 4E); this finding is consistent with poor immunogenicity of B16 melanoma (19-21).

To determine whether surviving mice were endowed with specific and long-term memory directed against the cancer antigen, the five-week survivors from Fig. 3C were re-challenged with B16-OVA cells and B16 cells lacking OVA (B16). In the absence of any further therapy, complete failure of B16-OVA tumor growth was observed, whereas B16 tumors grew rapidly (Fig. 3F). Both B16 and B16-OVA tumor cells grew at similar rates in naive C57BL/6J mice (Fig. 3F).

Taken together, these data indicate that when used as an adjuvant in a cancer vaccine, a Diprovocim promotes antigen-specific antitumor immunity, which is greatly enhanced when combined with T cell checkpoint blockade in mice. Immunization with a Diprovocim as an adjuvant produces antigen-specific memory responses that protect the host from relapse of tumor growth.

The antitumor effect of a Diprovocim was examined in therapeutic immunization of mice with already established B16-OVA tumors. C57BL/6J mice were immunized with OVA with or without a Diprovocim on the day of or three days after tumor inoculation and received a booster immunization seven days later (Fig. 3A). In some mice, alum was substituted for Diprovocim to permit direct comparison between these two adjuvants. For all conditions, anti-PD-L1 treatment was initiated on day 3 after tumor inoculation and repeated every three days thereafter for 12 days.

Mice immunized with OVA alone on the day of tumor inoculation survived an average of 24 days, and 100% of mice (8/8) died by 38 days after tumor inoculation. As expected, Diprovocim+OVA administered on the day of tumor inoculation completely inhibited tumor growth, permitting 100% of mice (8/8) to survive through 54 days of observation (Fig. 3G and Fig. 4H). When alum was used instead of a Diprovocim in the same study, tumor growth was partially inhibited, and the average survival time was 37 days, with 25% of mice (2/8) surviving past 54 days (Fig. 3G and Fig. 4H).

When immunization was delayed until three days after tumor inoculation, Diprovocim+OVA still significantly

inhibited tumor growth and prolonged average survival compared to OVA alone (41 days vs. 22 days) (Fig. 3I and Fig. 4J). In contrast, alum+OVA inhibited tumor growth but only slightly increased average survival time (30 days); the survival curve for alum+OVA was not significantly different from the curve for OVA alone (P = 0.082) (Fig. 3I and Fig. 4J). In pre- and post-tumor treatment, the effect of Diprovocim on tumor growth, survival rate, and survival time was superior to that of alum.

**Diprovocim enhances antitumor CTL responses**

The cellular mechanism by which combined anti-PD-L1 treatment and Diprovocim-adjuvanted immunization eliminates tumors was examined. Tumor-infiltrating leukocytes (TILs) of mice immunized with Diprovocim+OVA or alum+OVA were analyzed three days after tumor inoculation (Fig. 4A). Tumors were collected 14 days after inoculation, and single-cell suspensions were antibody stained and analyzed by flow cytometry to detect total leukocytes, CD4 and CD8 T cells, NK cells, DCs, and macrophages. The leukocytes were also stained with antibody to the H-2K$^b$ MHC-class I tetramer bound to the OVA peptide (residues 257-264), as well as antibody against CD8 to identify tumor-specific CD8 T cells.

OVA immunizations containing a Diprovocim significantly increased the frequency of leukocytes in tumors compared to vehicle+OVA (Fig. 4B). Further analysis of these TILs revealed that a Diprovocim increased the frequencies of CD4 and CD8 T cells including activated CD4 and CD8 T cells (CD44$^{high}$) and OVA-specific CD8 T cells, as well as the frequency of NK cells (Fig. 4C - Fig. 5H).

Alum+OVA immunization showed a trend towards increasing TILs (Fig. 4B), which reached statistical significance for total and CD44$^{hign}$ CD8 T cells (Fig. 4E and Fig. 5F). However, the magnitude of the increase was reduced compared to that induced by Diprovocim+OVA. OVA-specific CD8 T cells were not increased by alum+OVA immunization (Fig. 4G) on day 14 after tumor inoculation; neither were total and CD44$^{high}$ CD4 T cells (Fig. 4C and Fig. 5D), nor NK cells compared to vehicle+OVA (Fig. 4H).

The frequencies of intra-tumor DCs and macrophages were similar for mice immunized with vehicle+OVA, Diprovocim+OVA, and alum+OVA (Fig. 4I and Fig. 5J). Overall, these data indicate that the intra-tumor frequencies of CD4 and CD8 T cells, activated CD4 and CD8 T cells, OVA-specific CD8 T cells, and NK cells, but not DCs or macrophages, correlated with the antitumor effects of Diprovocim and alum in immunized mice.

To determine the immune cell population(s) necessary for the antitumor effect of Diprovocim+OVA plus anti-PD-L1, mice were depleted of CD8 T cells, CD4 T cells, NK cells, or all three cell populations using cell type-specific antibodies. The depletion antibodies were administered i.p. on the day of B16-OVA tumor inoculation (day 0) and every three days thereafter for 15 days (Fig. 4K). The effect of Diprovocim+OVA on both tumor growth and mouse survival was abrogated when mice were depleted of CD8 T cells or all three cell types together (CD4 T, CD8 T, NK cells) (Fig. 4L and Fig. 5M). In contrast, depletion of CD4 T cells or NK cells had little effect on the anti-tumor activity of Diprorocim+OVA (Fig. 4L and Fig. 5M).

Interestingly, a slight, statistically significant difference between the effects of CD8 T cell depletion vs. CD4+CD8+NK cell depletion was noted on tumor growth in mice treated with Diprovocim+OVA plus anti-PD-L1, in which tumor growth was greater in mice depleted of all three cell types. However, this difference did not translate to a difference in either survival rate or time. This finding suggests a minor role of either CD4 T cells, NK cells, or both, in mediating the anti-tumor effects of Diprovocim+OVA plus anti-PD-L1. These data demonstrate that CD8 T cells are necessary for tumor eradication by therapeutic Diprovocim+OVA immunization and checkpoint inhibition in mice.

**Discussion**

It is believed that cancer vaccines targeted to tumor neoantigens can boost the success of immune checkpoint inhibition for cancer treatment by increasing the number and activation of tumor-specific CTLs capable of responding to checkpoint inhibitors. However, the type and magnitude of the T cell response to immunization depends critically on the vaccine adjuvant; currently only few adjuvants are approved for use in humans.

Here, the actions of a novel and potent adjuvant, Diprovocim, that engages and activates human and mouse TLR1/TLR2 heterodimers are described. A Diprovocim bears no structural similarity to other reported synthetic chemical ligands, nor to the natural ligands that activate TLR1/TLR2 (22-26). A Diprovocim is more potent and efficacious in activating human TLR1/TLR2 than $Pam_3CSK_4$ (Fig. 9A and Fig. 9B), a well-known ligand.

In mice, a Diprovocim induces strong TLR1- and TLR2-dependent humoral and CTL responses to a co-administered antigen. When combined with checkpoint inhibition, a Diprovocim-adjuvanted immunization causes antigen-specific eradication of a rapidly fatal tumor, and induces memory responses capable of preventing tumor regrowth. Cure of the tumor is observed despite the fact that checkpoint inhibition alone is insufficient to prevent a fatal outcome (Fig. 8A and Fig. 8B), supporting the premise of this combination immunotherapy.

The data support the following key mechanistic events mediating the antitumor effect of Diprovocim-adjuvanted

immunization plus checkpoint inhibition (Fig. 5). Diprovocim binds to TLR1/TLR2 on APCs, activating them to produce pro-inflammatory cytokines and take up the administered tumor-specific antigens for processing and presentation via MHC I and MHC II. Antigen presentation, costimulatory molecule expression, and cytokine secretion by APCs induce proliferation and activation of antigen-specific CD4 T cells and CD8 T cells, which develop cytolytic activity toward tumor cells. NK cells are also activated by pro-inflammatory cytokines and infiltrate the tumor site. The addition of anti-PD-L1 inhibits the major immunosuppressive mechanism active in the tumor microenvironment, permitting uninhibited T cell activation and proliferation in response to TCR/CD28 ligation (27-29), further promoting tumor cell lysis mediated by CD8 T cells.

Numerous reports document both pro- and anti-tumorigenic effects of TLR2 signaling, which may depend on the cell type or type of cancer under study. For example, TLR2 signaling supports tumor growth through induction of immune suppressive cytokines such as IL-10, and activation of myeloid derived suppressor cells and tumor associated macrophages (30-32). In contrast, TLR2 signaling also promotes tumor regression by stimulating DC activation and cross-presentation (33), and downregulating Treg function (34-36).

For a Diprovocim, the overall outcome of systemic TLR2 activation, in the context of OVA immunization combined with immune checkpoint inhibition, was tumor cell lysis and tumor growth inhibition mediated by tumor-infiltrating antigen-specific CD8 T cells.

The therapeutic index of an adjuvant presumably depends upon the efficiency of conjoint targeting of antigen to an APC, and activation of that APC. The mode of interaction between a Diprovocim and TLR2 has been studied by X-ray crystallography, and its contacts with this subunit of the receptor will be reported elsewhere. The structure of a Diprovocim-TLR1/2 complex points to opportunities for a Diprovocim modification to incorporate immunogenic peptides, which might permit optimization of the therapeutic index by assuring that all active Diprovocim molecules are accompanied by antigen. Diprovocim is easy to synthesize and can be rapidly adapted to incorporate tumor-associated antigens and neoantigens. These features make it an attractive candidate for clinical development.

## Materials and methods

### Mice

C57BL/6J, *Tlr2*$^{-/-}$, *Myd88*$^{-/-}$ and OT-I mice were purchased from The Jackson Laboratory. *Ly96*$^{-/-}$ (*MD-2*$^{-/-}$) mice were from RIKEN BRC. *Tlr4*$^{lps3/lps3}$, *Tlr6*$^{int/int}$, *Tlr7*$^{rsq1/resq1}$, *Tirap*$^{tor/tor}$, *Ticam1*$^{Lprs2/Lps2}$, *Ticam1*$^{Lps2/Lps2}$/*Trak4*$^{otiose/otiose}$ mice were generated on a pure C57BL/6J background by ENU mutagenesis and are described at http://mutagenetix.utsouthwestern.edu.

*Tlr1*$^{-/-}$ mice were created by CRISPR/Cas 9 gene targeting. Female C57BL/6J mice were superovulated by injection of 6.5 U pregnant mare serum gonadotropin (PMSG; Millipore), followed by injection of 6.5 U human chorionic gonadotropin (hCG; Sigma-Aldrich) 48 hours later. The superovulated mice were subsequently mated overnight with C57BL/6J male mice. The following day, fertilized eggs were collected from the oviducts and *in* vitro-transcribed Cas9 mRNA (50 ng/μl) and Tlr1 small base-pairing guide RNA (50 ng/ul; 5'-CAAACCGAUCGUAGUGCUGA-3'; SEQ ID NO: 76) were injected into the cytoplasm or pronucleus of the embryos. The injected embryos were cultured in M16 medium (Sigma-Aldrich) at 37 °C in 5% CO2. For the production of mutant mice, two-cell stage embryos were transferred into the ampulla of the oviduct (10-20 embryos per oviduct) of pseudo-pregnant Hsd:ICR (CD-1) female mice (Harlan Laboratories).

All experimental procedures using mice were approved by the Institutional Animal Care and Use Committee (IACUC) of the University of Texas Southwestern Medical Center, and were conducted in accordance with institutionally approved protocols and guidelines for animal care and use. All the mice were maintained at the University of Texas Southwestern Medical Center in accordance with institutionally approved protocols.

### Isolation of peritoneal macrophages, BMDC, human PBMC and cell culture

Thioglycollate-elicited macrophages were recovered 4 days after i.p. injection of 2 ml BBL thioglycollate medium, brewer modified (4% wt/vol; BD Biosciences) by peritoneal lavage with 5 ml phosphate buffered saline (PBS). The peritoneal macrophages were cultured in DMEM cell culture medium [DMEM containing 10% vol/vol FBS (Gemini Bio Products), 1% vol/vol penicillin and streptomycin (Life Technologies)] at 37°C and 95% air/5% $CO_2$.

For murine BMDCs, bone morrow cells were cultured in Petri dishes in 10 ml DMEM cell culture medium containing 10 ng/ml of murine GM-CSF (R&D Systems). On day 3 of culture, this was replaced with fresh GM-CSF medium. Loosely adherent cells were transferred to a fresh Petri dish and cultured for an additional 4 d. Human PBMC were purchased from Stemcell Technologies. THP-1 (ATCC) cells were differentiated by treatment with 100 nM PMA (Sigma) in RPMI cell culture medium [RPMI containing 10% vol/vol FBS (Gemini Bio Products), 1% penicillin and streptomycin (Life Technologies)] for 24 hours. After that, cells were washed with PBS and cultured in fresh RPMI cell culture medium for 24 hours before use in studies.

**Measurement of cytokine production**

Cells were seeded onto 96-well plates at $1x10^5$ cells per well and stimulated with a Diprovocim (dissolved in DMSO, and final DMSO concentrations (≤0.2%) were kept constant in all experiments) for 4 h. Mouse TNF, IL-6, or IFN-β, or human TNF in the supernatants were measured by ELISA kits according to the manufacturer's instructions (eBioscience and PBL Assay Science). Pretreatment with 20 μg/ml anti-TLR1, anti-TLR2 or isotype control antibody (eBioscience) was for 1 hour. Unless otherwise indicated, mouse cells were from wild type C57BL/6J mice.

**Western blotting**

Mouse peritoneal macrophages or human THP-1 cells ($1x10^6$ per well) were stimulated in 12-well plates with a Diprovocim at 500 nM for mouse cells or 5 nM for human cells for the indicated times and lysed directly in sample buffer (Sigma). Cell lysates were separated by SDS-PAGE and transferred to nitrocellulose membranes. Membranes were probed with the following antibodies: phospho-IKKα (Ser176)/IKKβ (Ser177), IκBα, phospho-p38 (Thr180/Tyr182), phospho-JNK (Thr183/Tyr185), phospho-ERK1/2 (Thr202/Tyr204) (Cell Signaling Technology) and β-Actin (Sigma).

**Immunization and measurement**

**of antibody response**

EndoFit ovalbumin (OVA) with ≥98% purity minimum (SDS-PAGE) and endotoxin levels <1 EU/mg was purchased from Invivogen. Mice (4 mice per group) were immunized i.m. with 100 μg OVA mixed with vehicle (DMSO:Tween 80:saline=1:1:8), with 10 mg/kg Diprovocim, or with 2 mg/kg Alum (Alhydrogel adjuvant 2%, Invivogen). After 14 days, serum titers of OVA-specific IgG, IgG1, or IgG2b (SouthernBiotech) were measured by ELISA.

***In vivo* CTL killing assay**

C57BL/6J male mice were injected i.m. with 100 μg OVA plus 10 mg/kg Diprovocim (n=4 mice per group). One week later, naive C57BL/6J mice were killed, and splenocytes were collected. One-half of the splenocytes were left unpulsed, and half were pulsed with OVA257-263 peptides for 2 hours in complete medium [RPMI containing 10% vol/vol FBS, 1% penicillin and streptomycin] at 37 °C. The unpulsed and peptide-pulsed cells were labeled, respectively, with 0.5 μM ("low") or 5 μM ("high") CellTrace Violet (Invitrogen) in serum-free medium for 20 minutes. Equal numbers ($2x10^6$) of CellTrace Violet$^{high}$ (OVA pulsed) and CellTrace Violet$^{low}$ (unpulsed) cells were mixed together and injected intravenously into the immunized mice. After 48 hours, blood from treated mice was collected and subjected to flow cytometry analysis. The numbers of remaining live CellTrace Violet$^{high}$ and CellTrace Violet$^{low}$ cells were determined and used to calculate the percentage of OVA peptide-pulsed target cells killed. Specific killing was defined as

the ratio=CellTrace Violet$^{low}$ cells/ CellTrace Violet$^{high}$ cells.

The percentage of

target cell lysis = [1 - unimmunized ratio/immunized ratio] x 100.

**Tumor inoculation, immunization, and tumor measurement**

B16-OVA cells (B16F10 melanoma cells stably expressing chicken ovalbumin) were grown in DMEM containing 10% vol/vol FBS. A total of $2x10^5$ B16-OVA cells in 100 μL PBS were injected s.c. into the right flank of 8-12 week old male C57BL/6J mice to establish tumors (n=8 mice per group). For pretreatment, 10 mg/kg Diprovocim-1 or 2 mg/kg alum with or without OVA (100 μg) was injected i.m. into mice on the same day as tumor inoculation (day 0). Mice received a booster shot seven days after the first immunization. On day 3, 6 and 9, some groups were injected i.p. with 200 μg checkpoint inhibitor (anti-mPD-L1, BioXcell) in 100 μl saline.

For post-treatment, 10 mg/kg Diprovocim-1 or 2 mg/kg Alum with OVA (100 μg) was injected i.m. into mice on day 3 after tumor inoculation. Mice received a booster shot seven days after the first immunization. On day 3, 6, 9, 12 and 15 after tumor inoculation, mice were also injected i.p. with 200 μg anti-mPD-L1 in 100 μl saline.

For depletion of CD4 T cells, CD8 T cells, and/or NK cells, 300 μg anti-mCD4 (BioXcell), 300 μg anti-mCD8 (BioXcell), 300 μg anti-mNK1.1 (BioXcell), or the three antibodies together in 200 μl saline were injected i.p. into mice on day 0, 3, 6, 9, 12, and 15 after tumor inoculation. 10 mg/kg Diprovocim with OVA (100 μg) or vehicle was injected i.m. into mice on day 3 after tumor inoculation. Mice received a booster shot seven days after the first immunization. On day 3, 6, 9,

12 and 15 after tumor inoculation, mice were also injected i.p. with 200 μg anti-mPD-L1 in 100 μl saline.

Tumors were measured with a digital caliper (Fisher) and the tumor sizes were calculated using the following formula: volume=0.5 x length x width$^2$. Mice were sacrificed when the tumor length or width reached 2 cm.

### Tumor-infiltrating leukocyte separation and staining

A total of 2x105 B16-OVA cells in 100 μL PBS were injected s.c. into the flank of each mouse to establish tumors (n=6 mice per treatment). 10 mg/kg Diprovocim-1 or 2 mg/kg alum with OVA (100 μg) was injected i.m. into mice on day 3 after tumor inoculation. Mice received a booster shot seven days after the first immunization. On day 3, 6, 9, and 12 after tumor inoculation, mice were also injected i.p. with 200 μg anti-mPD-L1.

On day 14 after tumor inoculation, tumors were harvested, minced and filtered through a 40-μm strainer to obtain single-cell suspensions. Red blood cells were lysed with RBC lysis buffer (Sigma). After pelleting, cells were stained with a mixture of antibodies for 45 minutes, including anti-mouse CD45.2-PE or CD45.2-APC (BioLegend), anti-mouse CD3-FITC (BD Biosciences), anti-mouse CD4-BV786 (BD Biosciences), anti-mouse CD8-BV510 (BioLegend), anti-mouse CD44-PE-CF594 (BioLegend), APC-conjugated H-2Kb/OVA (SIINFEKL; SEQ ID NO: 77) tetramer (Baylor College of Medicine), anti-mouse F4/80-PE (Tonbo Bioscience), anti-mouse CD11b-BV605 (BioLegend), anti-CD11c-BV711 (BD Biosciences), anti-NK1.1-BV650 (BD Biosciences). Then, the cells were washed twice with PBS. Stained cells were analyzed with an LSRII instrument (BD Biosciences) and the flow cytometry data were analyzed using FlowJo software.

### Measurement of cross-priming of CD8 T cells

C57BL/6J male mice were injected i.m. with 100 ng OVA mixed with vehicle or with 10 mg/kg Diprovocim(n=4 mice per treatment). 24 hours later, DCs from draining lymph nodes and spleen were purified by Mouse Pan Dendritic Cell Isolation Kit (Miltenyi Biotech). CD8 T cells from OT-I transgenic mice were purified by Mouse CD8+ T Cell Isolation Kit (Miltenyi Biotech). 3x10$^5$ DCs were co-cultured with 3x10$^5$ OT-I CD8 T cells in RPMI medium containing 10% vol/vol FBS and 1% vol/vol penicillin and streptomycin for 24 hours. Then, cells were collected and stained with anti-mouse CD3-FITC, anti-mouse CD8-BV510, and anti-mouse CD69-PE-CF594 (BioLegend) for 45 minutes. Then, the cells were washed twice with PBS. Stained cells were analyzed with an LSRII instrument and the flow cytometry data were analyzed using FlowJo software.

### Statistical analyses

Data represent means ± SEM in all graphs depicting error bars. The statistical significance of differences between experimental groups was determined using GraphPad Prism 7 and the indicated statistical tests. For comparisons of differences between two unpaired experimental groups, an unpaired Student's t test was used and two-tailed P values are reported. P values are indicated by * $P \le 0.05$; ** $P \le 0.01$; *** $P \le 0.001$; **** $P \le 0.0001$. $P \le 0.05$ was considered statistically significant.

### Methods

### Synthesis of Diprovocim-1

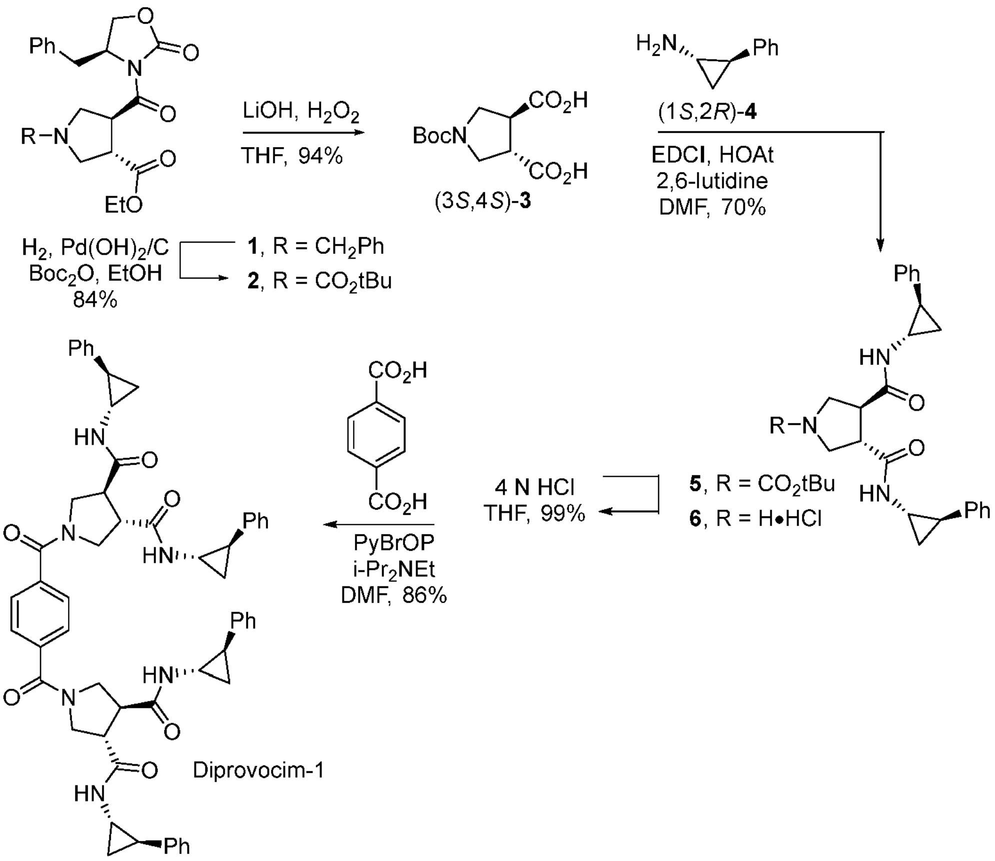


The syntheses of Diprovocim molecules and intermediates are shown and discussed in detail in WO 2018/005812 1A, published on January 04, 2018. Illustrative syntheses are also set out hereinbelow.

(3S,4S)-1-*tert*-Butyl 3-Ethyl 4-((S)-4-Benzyl-2-oxooxazolidine-3-carbonyl)pyrrolidine-1,3-dicarboxylate **(2).** (3*S*,4*S*)-Ethyl 1-benzyl-4-((S)-4-benzyl-2-oxooxazolidine-3-carbonyl)pyrrolidine-3-carboxylate[1] **(1,** 3.43 g, 7.86 mmol) and $Boc_2O$ (1.80 g, 8.25 mmol, 1.05 equiv) were dissolved in ethanol (EtOH, 50 mL) at room temperature. $Pd(OH)_2/C$ (500 mg) was added and the reaction mixture was sparged with nitrogen ($N_2$) for 15 minutes.

A 3-way flushing adapter, equipped with a hydrogen ($H_2$) filled balloon and vacuum source, was attached. The headspace above the reaction mixture was evacuated until the solvent began to boil, then backfilled with $H_2$. This vacuum/fill process was repeated 10-15 times to maximize $H_2$ in the headspace. After stirring for 18 hours, the reaction mixture was filtered through a 6 cm Celite plug, rinsing with EtOH aliquots (3 × 15 mL) thoroughly, and concentrated. Flash column chromatography ($SiO_2$, 25% EtOAc/hexanes) provided 2.93 g (84%) of **2** as a clear, viscous oil.
$^1H$ NMR (400 MHz, $CDCl_3$) δ 7.40 - 7.19 (m, 5H), 4.69 (dd, *J* = 9.0, 4.5 Hz, 1H), 4.52 (q, *J* = 7.7 Hz, 1H), 4.29 - 4.14 (m, 4H), 3.95 - 3.75 (m, 2H), 3.60 (m, 2H), 3.52 - 3.27 (m, 2H), 2.86 - 2.71 (m, 1H), 1.46 (s, 9H), 1.28 (t, *J* = 7.5 Hz, 3H). HRMS (ESI-TOF) *m/z* calcd for $C_{23}H_{31}N_2O_7$ $[M+H]^+$ 447.2126, found 447.2126.

[1] Prepared as a single stereoisomer according to: Bao et al., U.S. Patent 6,489,354 B1.

(3S,4S)-1-(tert-Butoxycarbonyl)pyrrolidine-3,4-dicarboxylic Acid (**3**).[2] (3S,4S)-1-tert-Butyl 3-ethyl 4-((S)-4-benzyl-2-oxooxazolidine-3-carbonyl)-pyrrolidine-1,3-dicarboxylate ((3*S*,4*S*)-**2**, 2.06 g, 4.63 mmol) was dissolved in anhydrous tetrahydrofuran (THF, 20 mL) and cooled to 0 °C. Hydrogen peroxide (2.10 mL, ca. 18.5 mmol, 4.0 equiv, 30% w/v) was added dropwise to the stirred reaction solution. After 3-5 minutes, LiOH•$H_2O$ (500 mg, 11.9 mmol) was added. After 2 hours, additional LiOH (470 mg, 11.2 mmol) was added, along with $H_2O$ (10 mL) and THF (15 mL).

[2] Modified procedure from: Ma et al., Tetrahedron Asymm. 8, 883-887 (1997).

The aqueous THF reaction mixture was stirred 3 hours, warming to room temperature. Saturated aqueous $Na_2SO_3$ (10 mL) was added, and the THF was removed under a $N_2$ stream. The resulting mixture was poured into $H_2O$ (200 mL) and extracted with methylene chloride ($CH_2Cl_2$, 2 × 100 mL) to remove the oxazolidinone.

The aqueous phase was acidified with the addition of aqueous 1 N HCl to pH 2 (ca. 75 mL). The aqueous phase was extracted with ethyl acetate (EtOAc, 3 × 125 mL), and the organic extracts were dried over $Na_2SO_4$, filtered and concentrated to provide 1.13 g (94%) of (*S*,*S*)-**3** as a white solid. $^1H$ NMR (500 MHz, DMSO-$d_6$) δ 3.59 - 3.48 (m, 2H), 3.41 - 3.31 (m, 2H), 3.30 - 3.18 (m, 2H), 1.39 (s, 9H).

(3*S*,4*S*)-*tert*-Butyl 3,4-Bis(((1*S*,2*R*)-2-phenylcyclopropyl)carbamoyl)pyrrolidine-1-carboxylate (**5**). (3*S*,4*S*)-1-(*tert*-Butoxycarbonyl)pyrrolidine-3,4-dicarboxylic acid ((*S*,*S*)-**3**, 775 mg, 2.99 mmol), (1S,2R)-trans-2-phenylcyclopropylamine ((1*S,2R*)-**4**, 816 mg, 6.13 mmol, 2.05 equiv, commercially available from D-L Chiral Chemicals), and 1-hydroxy-7-azabenzotriazole (HOAt, 895 mg, 6.58 mmol, 2.20 equiv) were dissolved in anhydrous dimethylformamide (DMF, (15 mL) under a $N_2$ atmosphere. 2,6-Lutidine (1.75 mL, 14.9 mmol, 5.00 equiv) was added slowly. Upon dissolution of the reagents (about 15 minutes), 1-ethyl-3-(3-dimethylaminopropyl)carbodiimide hydrochloride (EDCI•HCl, 1.43 g, 7.47 mmol, 2.50 equiv) was added in one portion, and the reaction mixture was stirred for 18 hours, after which it was poured into aqueous 1 N HCl (150 mL) and EtOAc (100 mL).

The aqueous phase was extracted with EtOAc (2 × 75 mL), and the combined organic phases were washed with aqueous 1 N HCl (75 mL), saturated aqueous $NaHCO_3$ (75 mL), and saturated aqueous NaCl (50 mL) sequentially. The organic phase was dried over $Na_2SO_4$, filtered and concentrated. Flash column chromatography ($SiO_2$, 50% EtOAc/hexanes) provided 1.02 g (70%) of **5**.

$^1$H NMR (400 MHz, $CDCl_3$) δ 7.33 - 7.24 (m, 5H), 7.23 - 7.09 (m, 5H), 6.61 (s, 1H), 6.43 (s, 1H), 3.85 (t, *J* = 9.7 Hz, 1H), 3.68 (m, 1H), 3.60 (t, *J* = 10.5 Hz, 1H), 3.42 (t, *J* = 10.4 Hz, 1H), 3.27 (q, *J* = 10.0, 9.3 Hz, 1H), 3.12 (t, *J* = 9.7 Hz, 1H), 2.88 (m, 2H), 2.05 (ddt, *J* = 9.8, 6.4, 3.4 Hz, 2H), 1.46 (s, 9H), 1.24 (q, *J* = 6.6 Hz, 2H), 1.13 (dt, *J* = 10.1, 5.3 Hz, 2H). HRMS (ESI-TOF) *m/z* calcd for $C_{29}H_{36}N_3O_4$ $[M+H]^+$ 490.2700, found 490.2705.

(3*S*,4*S*)-*N*$^3$,*N*$^4$-Bis((1*S*,2*R*)-2-phenylcyclopropyl)pyrrolidine-3,4-dicarboxamide Hydrochloride (**6**). (3S,4S)-*tert*-Butyl 3,4-bis(((1*S*,2*R*)-2-phenylcyclopropyl)carbamoyl)-pyrrolidine-1-carboxylate (**5**, 998 mg, 2.04 mmol) was suspended in anhydrous THF (2 mL) at room temperature. 4 N HCl (8 mL, 4.0 M solution in dioxane) was added dropwise to the vigorously stirred reaction solution. After stirring 3 hours at room temperature, during which some product had precipitated from the reaction mixture, the solvents were removed by $N_2$ stream over 16 hours. The residual solids were suspended in anhydrous THF and reconcentrated *in vacuo* (3 × 5 mL) to ensure complete removal of the dioxane and excess HCl. This process was repeated with anhydrous $Et_2O$ (3 × 5 mL) to provide 870 mg (99%) of **6** as an amorphous white solid.

$^1$H NMR (500 MHz, DMSO-*d*$_6$) δ 9.35 (s, 2H), 8.76 (d, J = 4.4 Hz, 2H), 7.26 (t, *J* = 7.6 Hz, 4H), 7.20 - 7.06 (m, 6H), 3.76 - 3.62 (m, 1H), 3.55 - 3.42 (m, 1H), 3.26 (t, *J* = 8.2 Hz, 2H), 3.21 - 3.11 (m, 2H), 2.90 - 2.78 (m, 2H), 1.99 (ddd, *J* = 9.6, 6.3, 3.4 Hz, 2H), 1.26 - 1.13 (m, 4H). HRMS (ESI-TOF) *m/z* calcd for $C_{24}H_{28}N_3O_2$ $[M+H]^+$ 390.2176, found 390.2178.

Diprovocim-1: (3S,3'S,4S,4'S)-1,1'-Terephthaloylbis(*N*$^3$,*N*$^4$-bis((1*S*,2*R*)-2-phenylcyclopropyl)pyrrolidine-3,4-dicarboxamide). (3*S*,4*S*)-*N*$^3$,*N*$^4$-Bis((1*S*,2*R*)-2-phenylcyclopropyl)-pyrrolidine-3,4-dicarboxamide hydrochloride **(6,** 500 mg, 1.17 mmol, 2.20 equiv) and terephthalic acid (benzene-1,4-dicarboxylic acid, 89 mg, 0.53 mmol, 1.00 equiv) were dissolved in anhydrous DMF (6 mL) at room temperature. *i*-$Pr_2$NEt (0.280 mL, 1.60 mmol, 3.00 equiv) was added, followed by bromo-tris-pyrrolidinophosphonium hexafluorophosphate (PyBrOP, 497 mg, 1.07 mmol, 2.00 equiv) after 5 minutes and the mixture was stirred at 23 °C for 18 hours. After 18 hours, the reaction mixture was diluted with EtOAc (300 mL) and washed with aqueous 0.5 N HCl (2 × 150 mL). The aqueous phase was extracted with EtOAc (1 × 50 mL).

The combined organic phases were washed with saturated aqueous $NaHCO_3$ (100 mL) and saturated aqueous NaCl (75 mL). The organic phase was dried over $Na_2SO_4$, decanted and concentrated. Flash column chromatography ($SiO_2$, 5-8% MeOH/$CH_2Cl_2$) provided Diprovocim-1. Diprovocim-1 could be further purified by trituration with cold (0 °C) 1:1 $Et_2O$/EtOAc (3 ×5 mL), decanting off the liquid phase to provide 421 mg (86%) of pure diprovocim. $[\alpha]_D^{26}$ +57 (*c* 0.33, EtOH). IR (neat) $v_{max}$ 3259, 1633, 1539, 1426, 1386, 1073, 695 $cm^{-1}$.

$^1$H NMR (600 MHz, DMSO-*d*$_6$) δ 8.42 (d, *J* = 4.3 Hz, 2H), 8.29 (d, *J* = 4.3 Hz, 2H), 7.56 (s, 4H), 7.27 - 7.21 (m, 8H), 7.19 - 7.09 (m, 8H), 7.09 - 7.03 (m, 4H), 3.80 (dd, *J* = 12.0, 8.6 Hz, 2H), 3.71 - 3.58 (m, 2H), 3.51 (ddd, *J* = 15.6, 11.2, 8.2 Hz, 4H), 3.19 (q, *J* = 8.4 Hz, 2H), 3.10 (q, *J* = 8.1 Hz, 2H), 2.90 - 2.80 (m, 2H), 2.80 - 2.73 (m, 2H), 1.97 (ddd, *J* = 9.6, 6.4, 3.4 Hz, 2H), 1.86 (ddd, *J* = 9.5, 6.3, 3.4 Hz, 2H), 1.21 - 1.13 (m, 4H), 1.13 - 1.05 (m, 4H). $^{13}$C NMR (151 MHz, DMSO-*d*$_6$) δ 171.65, 170.93, 167.46, 141.28, 141.19, 137.71, 128.17, 128.14, 127.09, 125.83, 125.79, 125.60, 51.48, 48.74, 46.95, 45.83, 45.07, 32.54, 32.45, 25.87, 23.90, 23.81, 15.33, 15.24. HRMS (ESI-TOF) *m/z* calcd for $C_{56}H_{57}N_6O_6$ $[M+H]^+$ 909.4334, found 909.4334.

## Citations

1. Hou B, Reizis B & DeFranco AL (2008) Toll-like receptors activate innate and adaptive immunity by using dendritic cell-intrinsic and - extrinsic mechanisms. Immunity 29(2): 272-282.
2. MacLeod H & Wetzler LM (2007) T cell activation by TLRs: A role for TLRs in the adaptive immune response. Sci STKE 2007(402): pe48.
3. Coffman RL, Sher A & Seder RA (2010) Vaccine adjuvants: Putting innate immunity to work. Immunity 33(4): 492-503.
4. Haanen JBAG (2017) Converting cold into hot tumors by combining immunotherapies. Cell 170(6): 1055-1056.

5. Sharma P, Hu-Lieskovan S, Wargo JA & Ribas A (2017) Primary, adaptive, and acquired resistance to cancer immunotherapy. Cell 168(4): 707-723.

6. Wang H, et al (2017) cGAS is essential for the antitumor effect of immune checkpoint blockade. Proc Natl Acad Sci U S A 114(7): 1637-1642.

7. Zou W, Wolchok JD & Chen L (2016) PD-L1 (B7-H1) and PD-1 pathway blockade for cancer therapy: Mechanisms, response biomarkers, and combinations. Sci Transl Med 8(328): 328rv4.

8. Topalian SL, Drake CG & Pardoll DM (2015) Immune checkpoint blockade: A common denominator approach to cancer therapy. Cancer Cell 27(4): 450-461.

9. Takeda Y, et al (2017) A TLR3-specific adjuvant relieves innate resistance to PD-L1 blockade without cytokine toxicity in tumor vaccine immunotherapy. Cell Rep 19(9): 1874-1887.

10. Shah RR, Hassett KJ & Brito LA (2017) Overview of vaccine adjuvants: Introduction, history, and current status. Methods Mol Biol 1494: 1-13.

11. Reed SG, Orr MT & Fox CB (2013) Key roles of adjuvants in modern vaccines. Nat Med 19(12): 1597-1608.

12. Kawai T & Akira S (2010) The role of pattern-recognition receptors in innate immunity: Update on toll-like receptors. Nat Immunol 11(5): 373-384.

13. Kawasaki T & Kawai T (2014) Toll-like receptor signaling pathways. Front Immunol 5: 461.

14. Beutler BA (2009) TLRs and innate immunity. Blood 113(7): 1399-1407.

15. Lim KH & Staudt LM (2013) Toll-like receptor signaling. Cold Spring Harb Perspect Biol 5(1): a011247.

16. Wang S, et al (2016) Intratumoral injection of a CpG oligonucleotide reverts resistance to PD-1 blockade by expanding multifunctional CD8+ T cells. Proc Natl Acad Sci U S A 113(46): E7240-E7249.

17. Dowling JK & Mansell A (2016) Toll-like receptors: The swiss army knife of immunity and vaccine development. Clin Transl Immunology 5(5): e85.

18. Goldberg J, et al (2002) Erythropoietin mimetics derived from solution phase combinatorial libraries. J Am Chem Soc 124(4): 544-555.

19. Ueha S, et al (2015) Robust antitumor effects of combined anti-CD4-depleting antibody and anti-PD-1/PD-L1 immune checkpoint antibody treatment in mice. Cancer Immunol Res 3(6): 631-640.

20. Lechner MG, et al (2013) Immunogenicity of murine solid tumor models as a defining feature of in vivo behavior and response to immunotherapy. J Immunother 36(9): 477-489.

21. Chen L, et al (1994) Tumor immunogenicity determines the effect of B7 costimulation on T cell-mediated tumor immunity. J Exp Med 179(2): 523-532.

22. Murgueitio MS, et al (2017) Enhanced immunostimulatory activity of in silico discovered agonists of toll-like receptor 2 (TLR2). Biochim Biophys Acta 1861(11 Pt A): 2680-2689.

23. Guo X, et al (2017) The novel toll-like receptor 2 agonist SUP3 enhances antigen presentation and T cell activation by dendritic cells. Front Immunol 8: 158.

24. Guan Y, Omueti-Ayoade K, Mutha SK, Hergenrother PJ & Tapping RI (2010) Identification of novel synthetic toll-like receptor 2 agonists by high throughput screening. J Biol Chem 285(31): 23755-23762.

25. Cheng K, et al (2015) Specific activation of the TLR1-TLR2 heterodimer by small-molecule agonists. Sci Adv 1(3): 10.1126/sciadv.1400139.

26. Jin MS, et al (2007) Crystal structure of the TLR1-TLR2 heterodimer induced by binding of a tri-acylated lipopeptide. Cell 130(6): 1071-1082.

27. Freeman GJ, et al (2000) Engagement of the PD-1 immunoinhibitory receptor by a novel B7 family member leads to negative regulation of lymphocyte activation. J Exp Med 192(7): 1027-1034.

28. Nishimura H, Nose M, Hiai H, Minato N & Honjo T (1999) Development of lupus-like autoimmune diseases by disruption of the PD-1 gene encoding an ITIM motif-carrying immunoreceptor. Immunity 11(2): 141-151.

29. Dong H, Zhu G, Tamada K & Chen L (1999) B7-H1, a third member of the B7 family, co-stimulates T-cell proliferation and interleukin-10 secretion. Nat Med 5(12): 1365-1369.

30. Tang M, et al (2015) Toll-like receptor 2 activation promotes tumor dendritic cell dysfunction by regulating IL-6 and IL-10 receptor signaling. Cell Rep 13(12): 2851-2864.

31. Yamazaki S, et al (2011) TLR2-dependent induction of IL-10 and Foxp3+ CD25+ CD4+ regulatory T cells prevents effective anti-tumor immunity induced by Pam2 lipopeptides in vivo. PLoS One 6(4): e18833.

32. Shime H, et al (2017) Toll-like receptor 2 ligand and interferon-gamma suppress anti-tumor T cell responses by enhancing the immunosuppressive activity of monocytic myeloid-derived suppressor cells. Oncoimmunology 7(1): e1373231.

33. Shen KY, et al (2014) Molecular mechanisms of TLR2-mediated antigen cross-presentation in dendritic cells. J Immunol 192(9): 4233-4241.

34. Nyirenda MH, et al (2011) TLR2 stimulation drives human naive and effector regulatory T cells into a Th17-like phenotype with reduced suppressive function. J Immunol 187(5): 2278-2290.

35. Zhang Y, et al (2011) TLR1/TLR2 agonist induces tumor regression by reciprocal modulation of effector and

regulatory T cells. J Immunol 186(4): 1963-1969.
36. Amiset L, et al (2012) TLR2 ligation protects effector T cells from regulatory T-cell mediated suppression and repolarizes T helper responses following MVA-based cancer immunotherapy. Oncoimmunology 1(8): 1271-1280.

The foregoing description and the examples are intended as illustrative and are not to be taken as limiting.

## Claims

1. An immunizing pharmacological composition for use in inhibiting the growth of cancerous or pathogen-infected cells in a mammal, which diseased cells exhibit a marker molecule that is absent on cells of the same type that are free of said disease or is present on the disease-free cells in significantly reduced numbers compared to said diseased cells, the pharmaceutical composition comprising:
(i) an adjuvant-sufficient amount of a Diprovocim compound, (ii) a T cell-stimulating amount of an immune checkpoint inhibitor, and (iii) an immunizing amount of said marker molecule or portion thereof; and
wherein said Diprovocim compound corresponds in structure to structural Formula **V,**

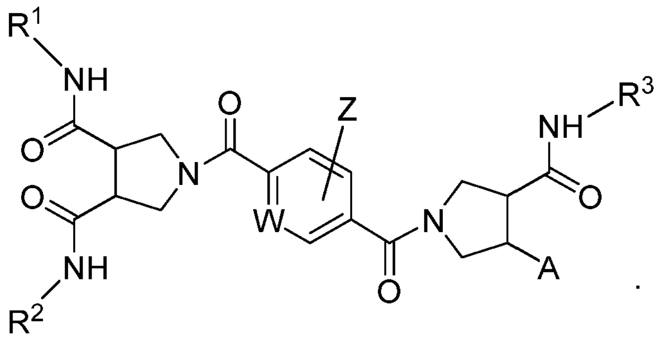

**V**

.

wherein

**-A** is -H (hydrido) or -C(O)NH-$R^4$;
$R^1$, $R^2$, $R^3$ and $R^4$ are the same or different and are a 2-(4-fluorophenyl)ethyl, a trans-2-phenylcyclopropyl, a trans-2-(4-fluorophenyl)-cyclopropyl or a $C_2$-$C_{18}$ hydrocarbyl group with the provisos that:

1) at least two of $R^1$, $R^2$, $R^3$ and $R^4$ ($R^{1-4}$) or at least two of $R^1$, $R^2$, and $R^3$ ($R^{1-3}$) are a trans-2-phenylcyclopropyl, a trans-2-(4-fluorophenyl)-cyclopropyl group or a mixture thereof, or each of $R^{1-4}$ is a 2-(4-fluorophenyl)ethyl group,
2) at least one depicted pyrrolidinyldicarboxamido group has the (*S,S*) configuration, and each depicted R substituent other than a $C_2$-$C_{18}$ hydrocarbyl group is a trans-2-phenylcyclopropyl, a trans-2-(4-fluorophenyl)-cyclopropyl group or a mixture thereof when each of $R^{1-4}$ is other than 2-(4-fluorophenyl)ethyl,
3) no more than two of $R^{1-4}$ are $C_2$-$C_{18}$ hydrocarbyl groups when **-A** is -C(O)NH-$R^4$, and
4) when ***A*** is hydrido, one of $R^{1-3}$ can be a $C_2$-$C_{18}$ hydrocarbyl group and the depicted $R^3$-containing pyrrolidinylcarboxamido group can have either the R or S configurations, or a mixture of both configurations;

-Z is one or more of halogen -H, $-NH_2$, -OH, $-OCH_3$, $-NO_2$, $-OCH_2CO_2H$, $-O(CH_2CH_20)_nCH_2CH_2CO_2H$, $-OCH_2CONH(CH_2CH_2O)_nCH_2CH_2CO_2H$, $-NHCOCH_2O-(CH_2CH_2O)_nCH_2CO_2H$, $-OCH_2CONHCH_2CONHCH(CHOH)CO_2H$, $-OCH_2CONHCH_2CONHCHCO_2H(CH_2CO_2H)$, $-OCH_2CONHCH_2CONHCH(CHOH)(CH_2CH_2O)_nCH_2CH_2CO_2H$, $-OCH_2CONHCH_2CONHCH[(CH_2)_4NH_2]CO_2H$, $-OCH_2CONHCH_2CONHCH(CH_2OH)CO\{NHCH[(CH_2)_4NH_2]CO\}_mNHCH-[(CH_2)_4NH_2]CO_2H$ (SEQ ID NOs: 3-8), $-OCH_2CONHCH_2CO\{NHCH[(CH_2)_4NH_2]CO\}_pNHCH[(CH_2)_4NH]CO_2H$ (SEQ ID NOs: 9-13) and $-OCH_2CONHCH_2CO\{NHCH(CH_2OH)CO\}_qNHCH(CH_2OH)CO_2H$ (SEQ ID NOs: 14-18);
W is nitrogen (N) or CH;
"n" is a number whose average value is one to about eight;
"m" is a number whose value is 1 to about 6; "p" is a number whose value is 1 to about 6; and
"q" is a number whose value is 1 to about 6.

2. The immunizing pharmaceutical composition for use according to claim 1, wherein said Diprovocim compound,

immune checkpoint inhibitor, and marker molecule or portion thereof are dissolved or dispersed within the same immunizing pharmaceutical composition, or within separate immunizing pharmaceutical compositions.

**3.** The immunizing pharmaceutical composition for use according to claim 2, wherein said immunizing composition is an aqueous medium.

**4.** The immunizing pharmaceutical composition for use according to claim 1, wherein said immune checkpoint inhibitor is a paratope-containing molecule.

**5.** The immunizing pharmaceutical composition for use according to claim 1, wherein the substituents of each depicted pyrrolidinyldicarboxamido group in Formula **V** has the (*S*,*S*) configuration.

**6.** The immunizing pharmaceutical composition for use according to claim 1, wherein W is CH, said Diprovocim compound has the structural Formula **Ia**, and wherein the $R^{1-4}$ and Z moieties are

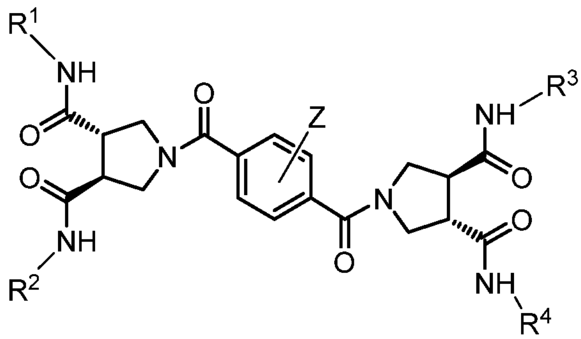

**Ia**

as described above.

**7.** The immunizing pharmaceutical composition for use according to claim 1, wherein -Z is -H; preferably wherein each of $R^{1-4}$ is a trans-2-phenylcyclopropyl group or a trans-2-(4-fluorophenyl)cyclopropyl group having the (1*S*,2*R*) configuration.

**8.** The immunizing pharmaceutical composition for use according to claim 1, wherein said diseased cells are cancerous solid tumor cells.

**9.** The immunizing pharmaceutical composition for use according to claim 8, wherein said marker molecule is one or more of CD96, CD20, DLL4, CD55, TIM-3, CXCR1, CD54, CD114, LGR5, CD105, CD56, CD13, CD271, CD34, CXCR4, CD26, CD117, CD10, CD146, Notch2, CD49f, CD24, ABCG2, PODXL-2, Cripto-1, CD326, CD90, CD133, SSEA1, TRA-1-81, TRA-1-60, SSEA4, SSEA3, CD151, CD340 and CD44.

**10.** The immunizing pharmaceutical composition for use according to claim 9, wherein said cancerous solid tumor cells are selected from the group consisting of one or more of osteosarcoma cells, Kaposi's sarcoma cells, melanoma cells, prostate cancer cells, glioblastoma cells, small cell lung carcinoma cells, breast cancer cells, liver cancer cells, colon cancer cells, ovarian cancer cells, renal cancer cells, gastric cancer cells, neuroblastoma cells, pancreatic cancer cells, and Hodgkin's lymphoma.

**11.** The immunizing pharmaceutical composition for use according to claim 1, wherein said diseased cells are pathogen-infected.

**12.** The immunizing pharmaceutical composition for use according to claim 11, wherein the infecting pathogen is one or more of a virus, a bacterium, a fungus, and an unicellular parasite.

**13.** The immunizing pharmaceutical composition for use according to claim 12, wherein said marker molecule is one or more of the circumsporozoite protein *of P. falciparum, P. vivax, P. bergeii* or *P. yoelli;* the M2e protein, hemagglutinin protein or neuraminidase protein of influenza virus.

**14.** The immunizing pharmaceutical composition for use according to claim 1, wherein said Diprovocim compound has a

structural formula selected from the group consisting of one or more of Formulas **A, B, C, D, E, F, G, H** and **I**

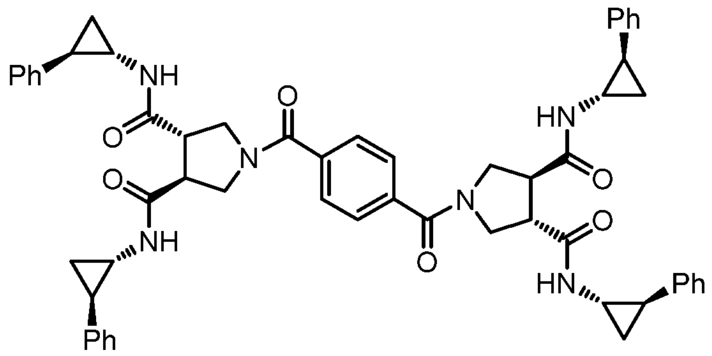

**A**

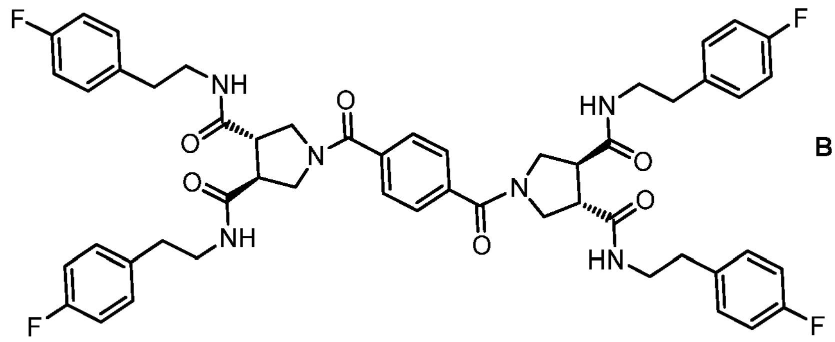

**B**

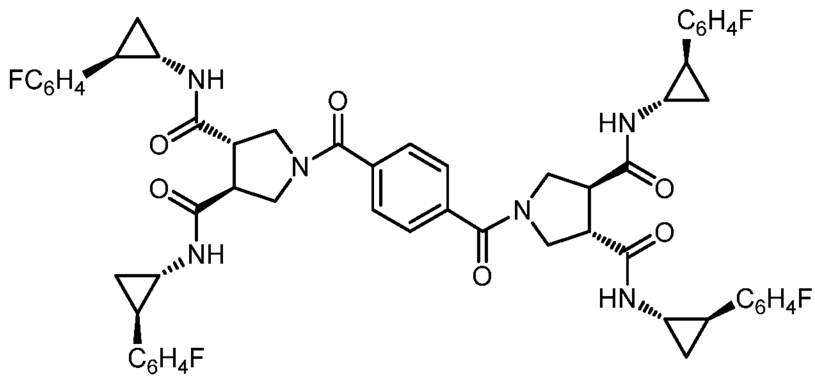

**C**

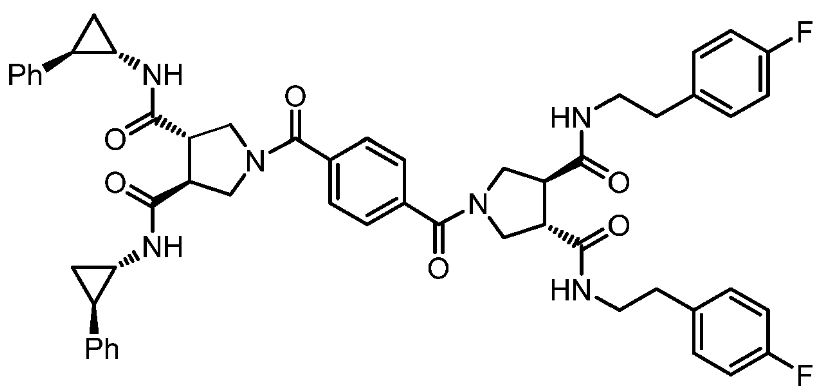

**D**

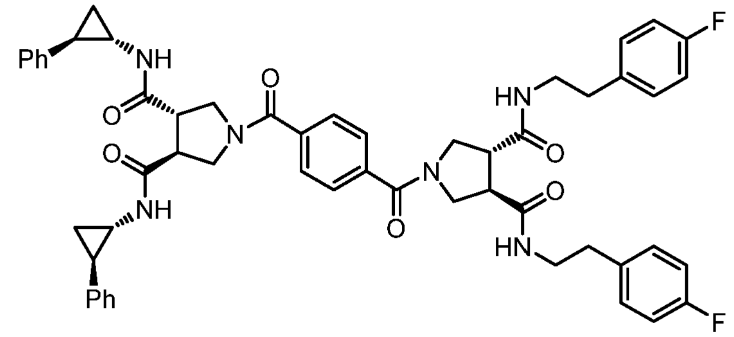

E

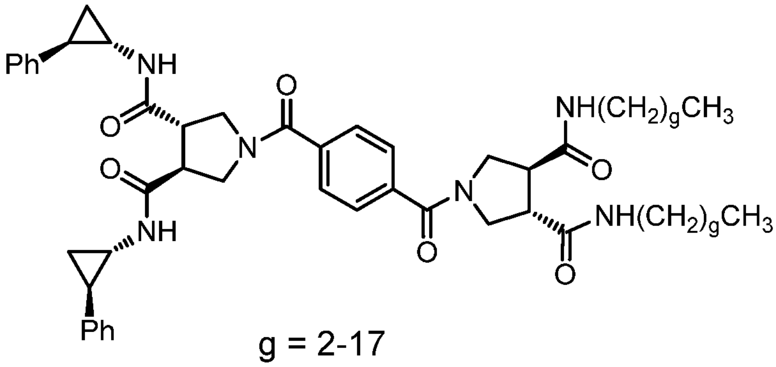

g = 2-17

F

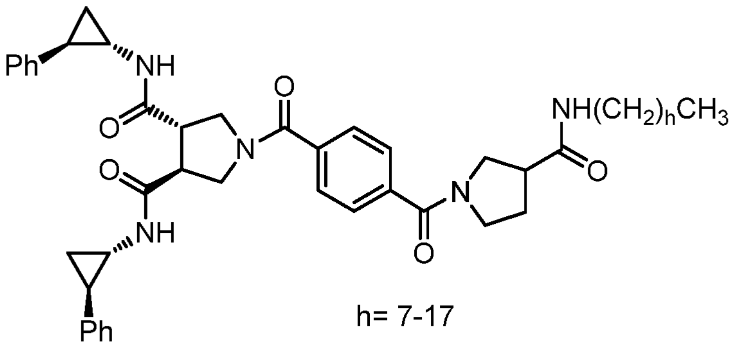

h= 7-17

G

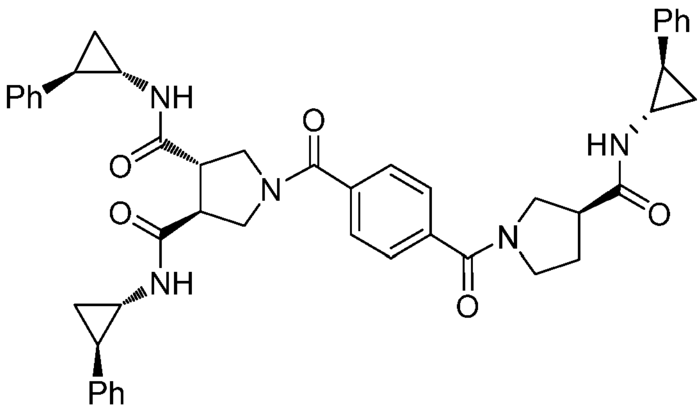

H

and

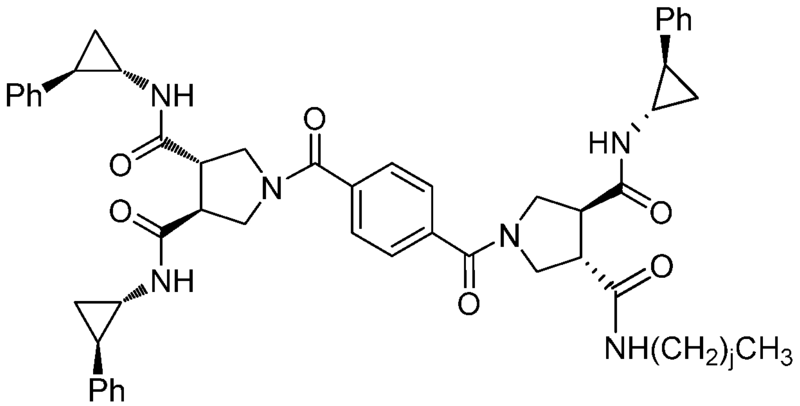

I

j = 7-17

## Patentansprüche

1. Immunisierende pharmakologische Zusammensetzung zur Hemmung des Wachstums von Krebszellen oder pathogeninfizierten Zellen in einem Säugetier, wobei die erkrankten Zellen ein Markermolekül aufweisen, das an Zellen desselben Typs, die frei von der Krankheit sind, fehlt oder an den krankheitsfreien Zellen in deutlich geringerer Anzahl als den erkrankten Zellen vorhanden ist, die pharmazeutische Zusammensetzung umfassend:
(i) eine adjuvant ausreichende Menge einer Diprovocim-Verbindung, (ii) eine T-Zellen stimulierende Menge eines Immun-Checkpoint-Inhibitors und (iii) eine immunisierende Menge des Markermoleküls oder eines Teils davon und wobei die Diprovocim-Verbindung in ihrer Struktur der Strukturformel **V** entspricht,

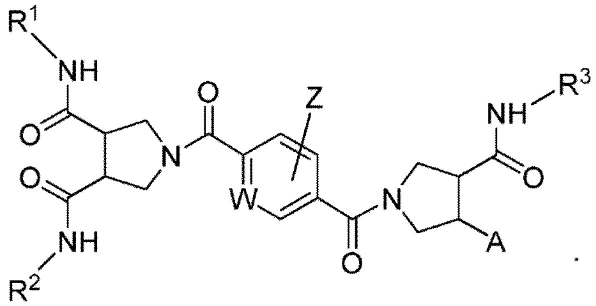

**V**

wobei

A -H (Hydrido) oder -C(O)NH-$R^4$ ist,
$R^1$, $R^2$, $R^3$ und $R^4$ gleich oder verschieden sind und eine 2-(4-Fluorphenyl)ethyl-, eine trans-2-Phenylcyclopropyl-, eine trans-2-(4-Fluorphenyl)cyclopropyl- oder eine $C_2$-$C_{18}$-Hydrocarbylgruppe sind, mit den Maßgaben, dass:

1) mindestens zwei von $R^1$, $R^2$, $R^3$ und $R^4$ ($R^{1-4}$) oder mindestens zwei von $R_1$, $R_2$ und $R^3$ ($R^{1-3}$) eine trans-2-Phenylcyclopropyl-, eine trans-2-(4-Fluorphenyl)cyclopropylgruppe oder eine Mischung davon sind oder jedes von $R^{1-4}$ eine 2-(4-Fluorphenyl)ethylgruppe ist,
2) mindestens eine abgebildete Pyrrolidinyldicarboxamidgruppe die (*S,S*)-Konfiguration aufweist und jeder abgebildete R-Substituent außer einer $C_2$-$C_{18}$-Hydrocarbylgruppe eine trans-2-Phenylcyclopropyl-, eine trans-2-(4-Fluorphenyl)cyclopropylgruppe oder eine Mischung davon ist, wenn jedes der $R^{1-4}$ nicht 2-(4-Fluorphenyl)ethyl ist,
3) nicht mehr als zwei von $R^{1-4}$ $C_2$-$C_{18}$-Hydrocarbylgruppen sind, wenn -A -C(O)NH-$R_4$ ist, und
4) wenn A Hydrido ist, eines von $R^{1-3}$ eine $C_2$-$C_{18}$-Hydrocarbylgruppe sein kann und die abgebildete $R^3$-enthaltende Pyrrolidinylcarboxamidgruppe entweder die *R*- oder *S*-Konfiguration oder eine Mischung beider Konfigurationen aufweisen kann,

-Z ein oder mehrere von Halogen, -H, $-NH_2$, -OH, $-OCH_3$, $-NO_2$, $-OCH_2CO_2H$, $-O(CH_2CH_2O)_nCH_2CH_2CO_2H$,

$-OCH_2CONH(CH_2CH_2O)_nCH_2CH_2CO_2H$, $-NHCOCH_2O-(CH_2CH_2O)_nCH_2CO_2H$, $-OCH_2CONHCH_2CONHCH(CHOH)CO_2H$, $-OCH_2CONHCH_2CONHCHCO_2H(CH_2CO_2H)$, $-OCH_2CONHCH_2CONHCH(CHOH)(CH_2CH_2O)_nCH_2CH_2CO_2H$, $-OCH_2CONHCH_2CONHCH[(CH_2)_4NH_2]CO_2H$, $-OCH_2CONHCH_2CONHCH(CH_2OH)CO\{NHCH[(CH_2)_4NH_2]CO\}_mNHCH-[(CH_2)_4NH_2]CO_2H$ (SEQ ID Nr: 3-8), $-OCH_2CONHCH_2CO\{NHCH[(CH_2)_4NH_2]CO\}_pNHCH[(CH_2)_4NH]CO_2H$ (SEQ ID Nr: 9-13) und $-OCH_2CONHCH_2CO\{NHCH(CH_2OH)CO\}_qNHCH(CH_2OH)CO_2H$ (SEQ ID Nr: 14-18) ist,
W Stickstoff (N) oder CH ist,
"n" eine Zahl ist, deren Durchschnittswert eins bis etwa acht ist,
"m" eine Zahl ist, deren Wert 1 bis etwa 6 ist,
"p" eine Zahl ist, deren Wert 1 bis etwa 6 ist,
"q" eine Zahl ist, deren Wert 1 bis etwa 6 ist.

**2.** Immunisierende pharmazeutische Zusammensetzung zur Verwendung nach Anspruch 1, wobei die Diprovocim-Verbindung, der Immun-Checkpoint-Inhibitor und das Markermolekül oder ein Teil davon in derselben immunisierenden pharmazeutischen Zusammensetzung oder in getrennten immunisierenden pharmazeutischen Zusammensetzungen gelöst oder dispergiert sind.

**3.** Immunisierende pharmazeutische Zusammensetzung zur Verwendung nach Anspruch 2, wobei die immunisierende Zusammensetzung ein wässriges Medium ist.

**4.** Immunisierende pharmazeutische Zusammensetzung zur Verwendung nach Anspruch 1, wobei der Immun-Checkpoint-Inhibitor ein Paratop-haltiges Molekül ist.

**5.** Immunisierende pharmazeutische Zusammensetzung zur Verwendung nach Anspruch 1, wobei die Substituenten jeder abgebildeten Pyrrolidinyldicarboxamidgruppe in Formel V die (S,S)-Konfiguration aufweisen.

**6.** Immunisierende pharmazeutische Zusammensetzung zur Verwendung nach Anspruch 1, wobei W CH ist, die Diprovocim-Verbindung die Strukturformel **Ia** aufweist

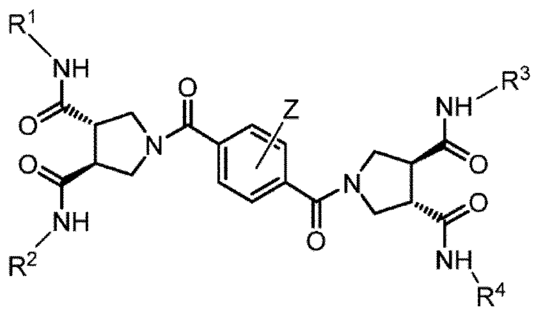

Ia

und wobei die $R^{1-4}$- und Z-Reste wie oben beschrieben sind.

**7.** Immunisierende pharmazeutische Zusammensetzung zur Verwendung nach Anspruch 1, wobei -Z H ist, wobei vorzugsweise jedes von $R^{1-4}$ eine trans-2-Phenylcyclopropylgruppe oder eine trans-2-(4-Fluorphenyl)cyclopropylgruppe ist, die eine (1S,2R)-Konfiguration aufweist.

**8.** Immunisierende pharmazeutische Zusammensetzung zur Verwendung nach Anspruch 1, wobei die erkrankten Zellen krebsartige solide Tumorzellen sind.

**9.** Immunisierende pharmazeutische Zusammensetzung zur Verwendung nach Anspruch 8, wobei das Markermolekül eines oder mehrere von CD96, CD20, DLL4, CD55, TIM-3, CXCR1, CD54, CD114, LGR5, CD105, CD56, CD13, CD271, CD34, CXCR4, CD26, CD117, CD10, CD146, Notch2, CD49f, CD24, ABCG2, PODXL-2, Cripto-1, CD326, CD90, CD133, SSEA1, TRA-1-81, TRA-1-60, SSEA4, SSEA3, CD151, CD340 und CD44 ist.

**10.** Immunisierende pharmazeutische Zusammensetzung zur Verwendung nach Anspruch 9, wobei die krebsartigen soliden Tumorzellen aus der Gruppe ausgewählt sind, bestehend aus Osteosarkom-Zellen, Kaposi-Sarkom-Zellen, Melanom-Zellen, Prostatakrebs-Zellen, Glioblastom-Zellen, kleinzelligen Lungenkarzinom-Zellen, Brustkrebs-Zellen, Leberkrebs-Zellen, Dickdarmkrebs-Zellen, Eierstockkrebs-Zellen, Nierenkrebs-Zellen, Magenkrebs-Zellen,

Neuroblastom-Zellen, Bauchspeicheldrüsenkrebs-Zellen und Hodgkin-Lymphom.

**11.** Immunisierende pharmazeutische Zusammensetzung zur Verwendung nach Anspruch 1, wobei die erkrankten Zellen pathogeninfiziert sind.

**12.** Immunisierende pharmazeutische Zusammensetzung zur Verwendung nach Anspruch 11, wobei das infizierende Pathogen ein oder mehrere eines Virus, eines Bakteriums, eines Pilzes und eines einzelligen Parasites ist.

**13.** Immunisierende pharmazeutische Zusammensetzung zur Verwendung nach Anspruch 12, wobei das Markermolekül eines oder mehrere des Circumsporozoitenproteins von *P. falciparum, P. vivax, P. bergeii* oder *P. yoelli,* des M2e-Proteins, des Hämagglutininproteins oder des Neuraminidaseproteins des Grippevirus ist.

**14.** Immunisierende pharmazeutische Zusammensetzung zur Verwendung nach Anspruch 1, wobei die Diprovocim-Verbindung eine Strukturformel aufweist, die aus der Gruppe ausgewählt ist, die aus einer oder mehreren der Formeln A, B, C, D, E, F, G, H und I besteht

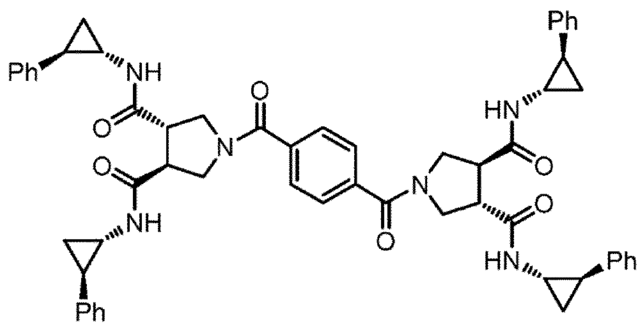

A

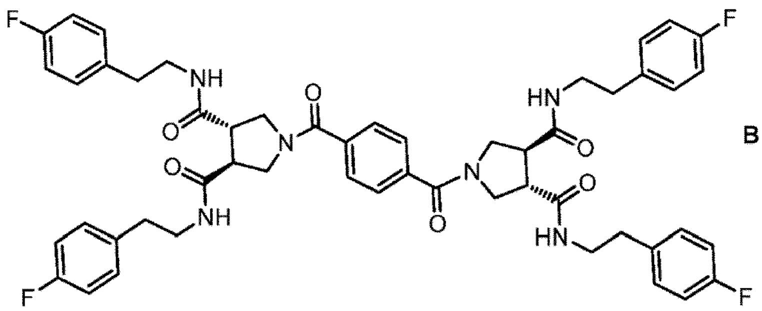

B

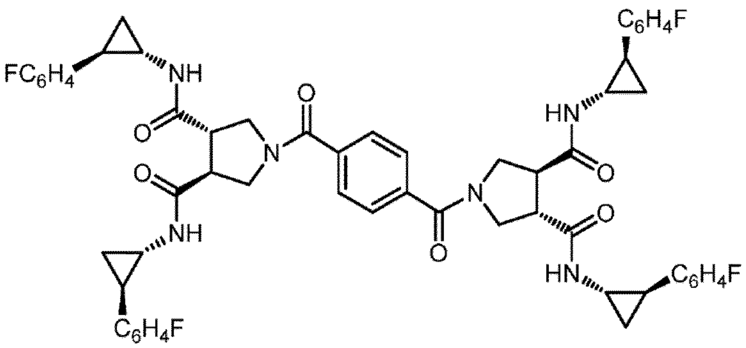

C

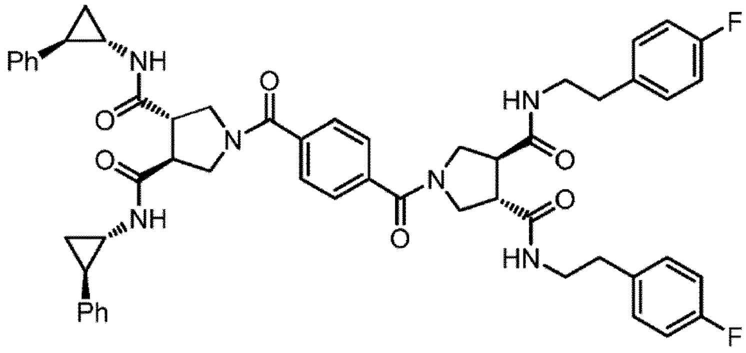

**D**

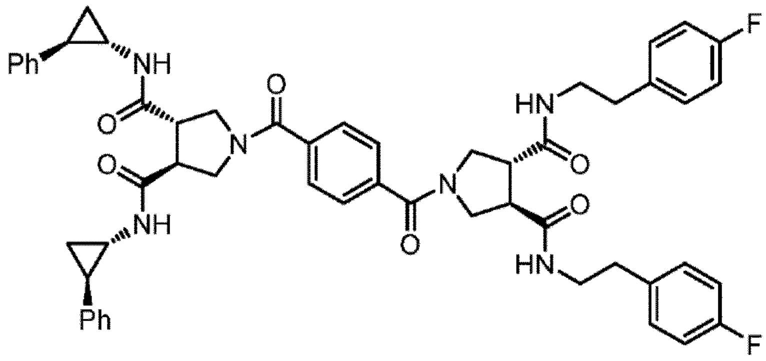

**E**

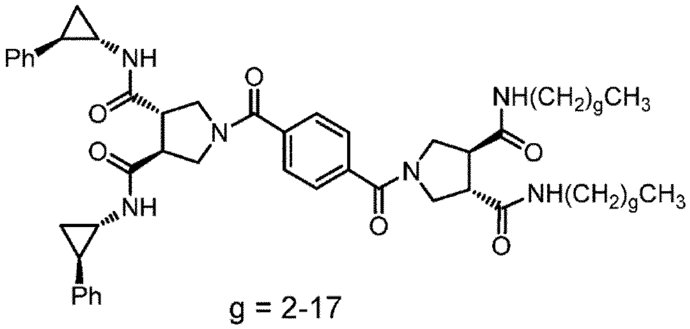

**F**

g = 2-17

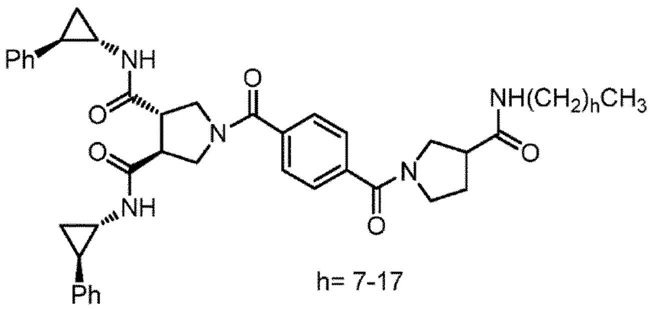

**G**

h= 7-17

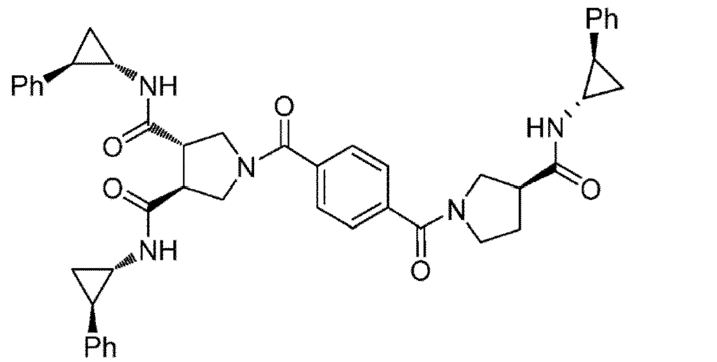

H

und

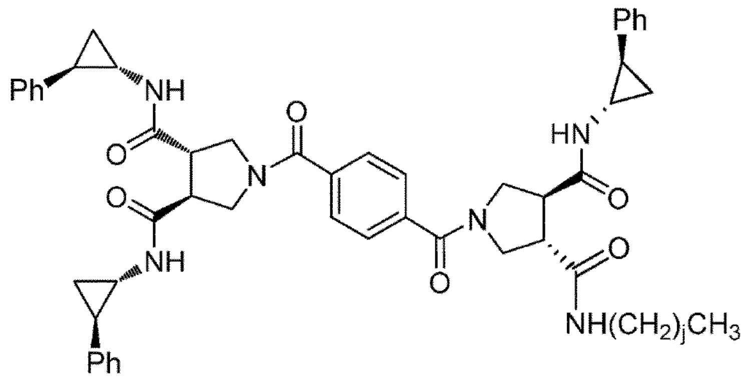

I

j = 7-17

.

**Revendications**

1. Une composition pharmacologique immunisante destinée à être utilisée pour inhiber, chez un mammifère, la croissance de cellules cancéreuses ou infectées par des agents pathogènes, lesquelles cellules malades comportent une molécule marqueur qui est absente sur les cellules du même type exemptes de ladite maladie ou qui est présente en nombre significativement réduit sur les cellules non malades par comparaison avec lesdites cellules malades, la composition pharmaceutique comprenant :

   (i) une quantité suffisante d''adjuvant d'un composé consistant en Diprovocim,
   (ii) une quantité stimulant les cellules T inhibitrice du point de contrôle immunitaire et
   (iii) une quantité immunisante de ladite molécule marqueur ou d'une partie de celle-ci ; et

   dans laquelle ledit composé Diprovocim correspond structurellement à la formule structurale V,

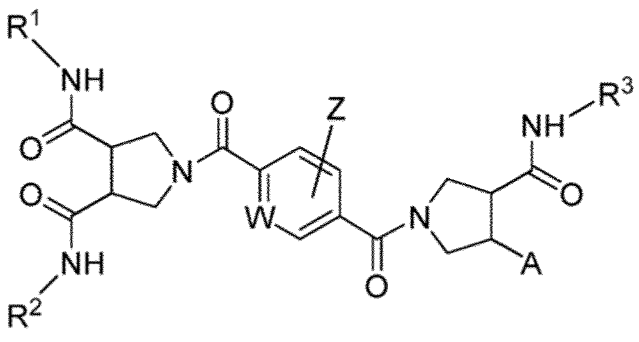

V

dans laquelle

-**A** est -H (hydrido) ou -C (O) NH-$R^4$ ;
$R^1$, $R^2$, $R^3$ et $R^4$, identiques ou différents, représentent un groupe 2-(4-fluorophényl)éthyle, un trans-2-phénylcyclopropyle, un trans-2-(4-fluorophényl)-cyclopropyle ou un groupe hydrocarbyle en $C_2$-$C_{18}$ à condition que :

1) au moins deux des $R^1$, $R^2$, $R^3$ et $R^4$ ($R^{1-4}$) ou au moins deux des $R^1$, $R^2$ et $R^3$ ($R^{1-3}$) sont un groupe trans-2-phénylcyclopropyle, un groupe trans-2-(4-fluorophényl)-cyclopropyle ou un mélange de ceux-ci, ou chacun des $R^{1-4}$ est un groupe 2-(4-fluorophényl)-éthyle,
2) au moins un groupe pyrrolidinyldicarboxamido représenté présente la configuration en (*S, S*), et chaque substituant *R* représenté autre qu'un groupe hydrocarbyle en $C_2$-$C_{18}$ est un groupe trans-2-phénylcyclopropyle, un groupe trans-2-(4-fluorophényl)-cyclopropyle ou un mélange de ceux-ci lorsque chacun des $R^{1-4}$ est autre que 2-(4-fluorophényl)éthyle,
3) pas plus de deux des $R^{1-4}$ sont des groupes hydrocarbyles en $C_2$-$C_{18}$ lorsque -**A** est -C(O)NH-$R^4$, et
4) lorsque A est hydrido, l'un des $R^{1-3}$ peut être un groupe hydrocarbyle en $C_2$-$C_{18}$ et le groupe pyrrolidinylcarboxamido contenant $R^3$ représenté peut avoir soit les configurations *R* ou *S*, soit un mélange des deux configurations ;

-z est un ou plusieurs halogènes, -H, $-NH_2$, -OH, $-OCH_3$, $-NO_2$, $-OCH_2CO_2H$, - $O(CH_2CH_2O)_nCH_2CH_2CO_2H$, $-OCH_2CONH(CH_2CH_2O)_nCH_2CH_2CO_2H$, $-NHCOCH_2O(CH_2CH_2O)_nCH_2CO_2H$, $-OCH_2CONHCH_2CONHCH(CHOH)CO_2H$, $-OCH_2CONHCH_2CONHCHCO_2H(CH_2CO_2H)$ , $-OCH_2CONHCH_2CONHCH(CHOH)(CH_2CH_2O)_nCH_2CH_2CO_2H$, $-OCH_2CONHCH_2CONHCHC[(CH_2)_4NH_2]CO_2H$, $-OCH_2CONHCH_2CONHCH(CH_2OH)CO\{NHCH[(CH_2)_4NH_2]CO\}_mNHCH-[(CH_2)_4NH_2]CO_2H$ (SEQ ID NOs: 3-8), $-OCH_2CONHCH_2CO\{NHCH[(CH_2)_4NH_2]CO\}_pNHCH[(CH_2)_4NH]CO_2H$ (SEQ ID NOs: 9-13) , et $-OCH_2CONHCH_2CO\{NHCH(CH_2OH)CO\}_qNHCH(CH_2OH)CO_2H$ (SEQ ID Nos:14-18) ;
W est l'azote (N) ou CH ;
«n» est un nombre dont la valeur moyenne est comprise entre un et environ huit ; «m» est un nombre dont la valeur est comprise entre 1 et environ 6 ;
«p» est un nombre dont la valeur est comprise entre 1 et environ 6 ; et
«q» est un nombre dont la valeur est comprise entre 1 et environ 6.

**2.** La composition pharmaceutique immunisante destinée à être utilisée selon la revendication 1, dans laquelle ledit composé Diprovocim, inhibiteur de point de contrôle immunitaire, et la molécule marqueur ou une partie de ceux-ci sont dissous ou dispersés dans la même composition pharmaceutique immunisante ou dans des compositions pharmaceutiques immunisantes distinctes.

**3.** La composition pharmaceutique immunisante destinée à être utilisée selon la revendication 2, dans laquelle ladite composition immunisante est un milieu aqueux.

**4.** La composition pharmaceutique immunisante destinée à être utilisée selon la revendication 1, dans laquelle ledit inhibiteur de point de contrôle immunitaire est une molécule contenant un paratope.

**5.** La composition pharmaceutique immunisante destinée à être utilisée selon la revendication 1, dans laquelle les substituants de chaque groupe pyrrolidinyldicarboxamido représenté dans la formule **V** ont la configuration (S, S).

**6.** La composition pharmaceutique immunisante destinée à être utilisée selon la revendication 1, dans laquelle W est CH, ledit composé Diprovocim présente la formule structurale **Ia**, et dans laquelle les fragments $R^{1-4}$ et z sont

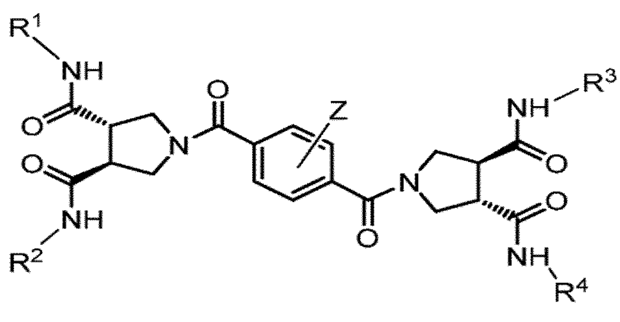

**Ia**

tels que décrits ci-dessus.

7. La composition pharmaceutique immunisante destinée à être utilisée selon la revendication 1, où -z est -H; de préférence dans laquelle chacun des $R^{1-4}$ est un groupe trans-2-(4-phénylcyclopropyle ou un groupe trans-2-(4-fluorophényl)cyclo-propyle ayant une configuration (I*S*, 2*R*).

8. La composition pharmaceutique immunisante destinée à être utilisée selon la revendication 1, dans laquelle lesdites cellules malades sont des cellules tumorales solides cancéreuses.

9. La composition pharmaceutique immunisante destinée à être utilisée selon la revendication 8, dans laquelle ladite molécule marqueur est une ou plusieurs des molécules CD96, CD20, DLL4, CD55, TIM-3, CXCR1, CD54, CD114, LGR5, CD105, CD56, CD13, CD271, CD34, CXCR4, CD26, CD117, CD1O, CD146, Notch2, CD49f, CD24, ABCG2, PODXL-2, Cripto-1, CD326, CD90, CD133, SSEA1, TRA-1-81, TRA-1-60, SSEA4, SSEA3, CD151, CD340 et CD44.

10. La composition pharmaceutique immunisante destinée à être utilisée selon la revendication 9, dans laquelle lesdites cellules tumorales solides cancéreuses sont sélectionnées dans le groupe constitué d'une ou plusieurs cellules parmi les cellules d'ostéosarcome, les cellules de sarcome de Kaposi, les cellules de mélanome, les cellules cancéreuses de la prostate, les cellules de glioblastome, les cellules de carcinome pulmonaire à petites cellules, les cellules cancéreuses du sein, les cellules cancéreuses du foie, les cellules cancéreuses du côlon, les cellules cancéreuses de l'ovaire, les cellules cancéreuses du rein, les cellules cancéreuses gastriques, les cellules de neuroblastome, les cellules cancéreuses du pancréas et le lymphome de Hodgkin.

11. La composition pharmaceutique immunisante destinée à être utilisée selon la revendication 1, dans laquelle lesdites cellules malades sont infectées par un agent pathogène.

12. La composition pharmaceutique immunisante destinée à être utilisée selon la revendication 11, dans laquelle l'agent pathogène infectant est un ou plusieurs des virus, bactérie, champignon et parasite unicellulaire.

13. La composition pharmaceutique immunisante destinée à être utilisée selon la revendication 12, dans laquelle ladite molécule marqueur est une ou plusieurs parmi la protéine circumsporozoïte de *P. falciparum, P. vivax, P. bergeii ou P. yoelli* ; la protéine M2e, la protéine hémagglutinine ou la protéine neuraminidase du virus de la grippe.

14. La composition pharmaceutique immunisante destinée à être utilisée selon la revendication 1, dans laquelle ledit composé Diprovocim présente une formule structurale choisie dans le groupe constitué d'une ou plusieurs des formules A, B, C, D, E, F, G, H et 1

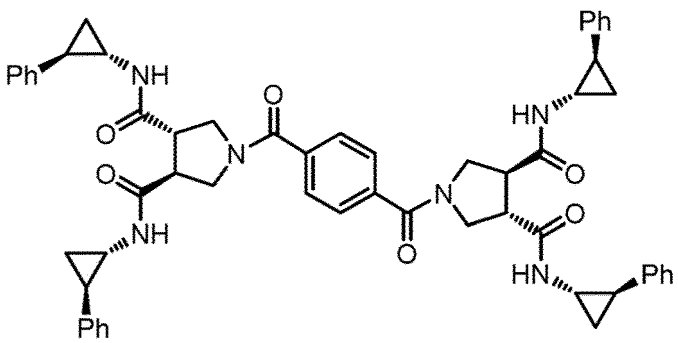

A

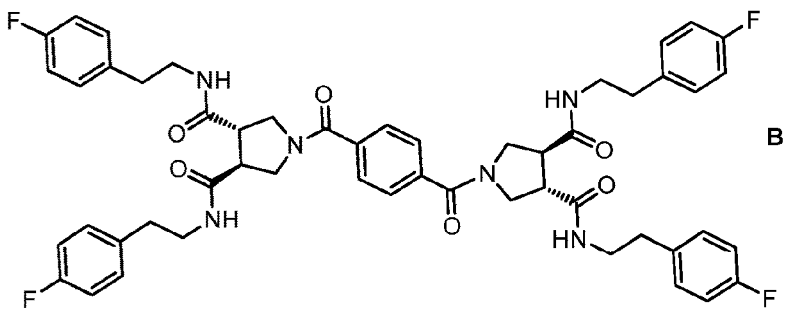

B

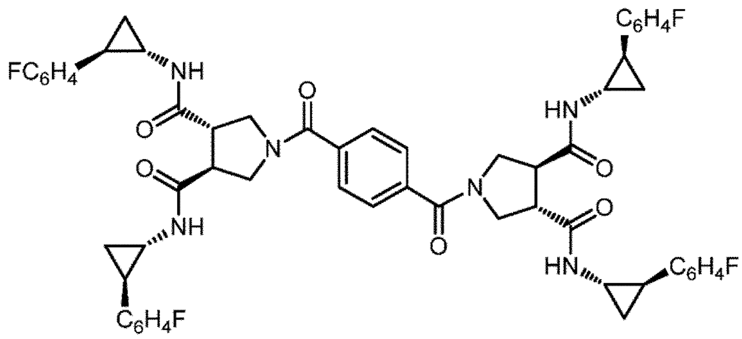

C

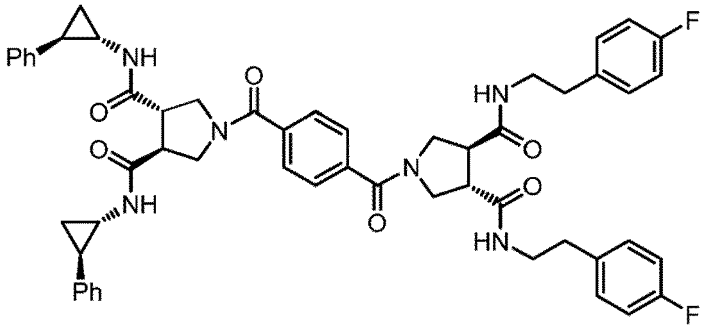

D

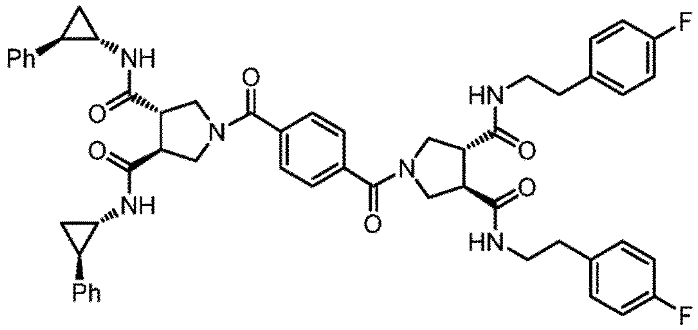

E

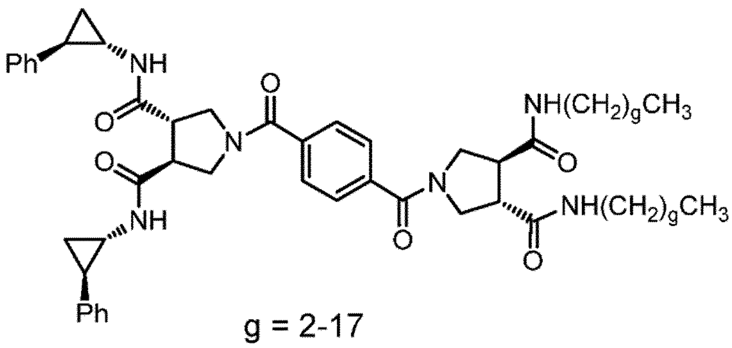

F

g = 2-17

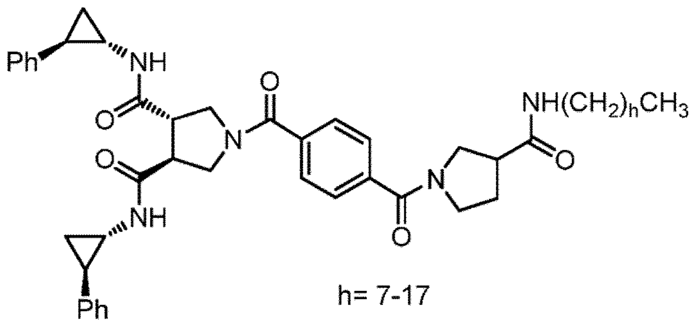

h= 7-17

**G**

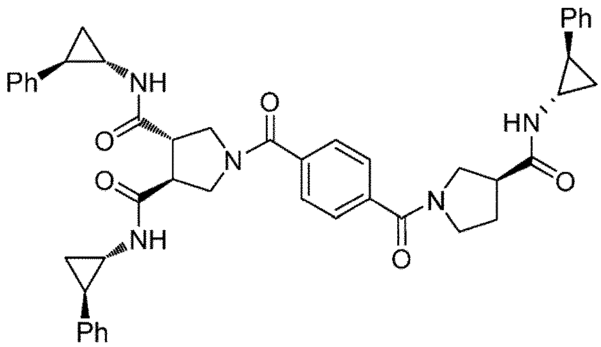

**H**

et

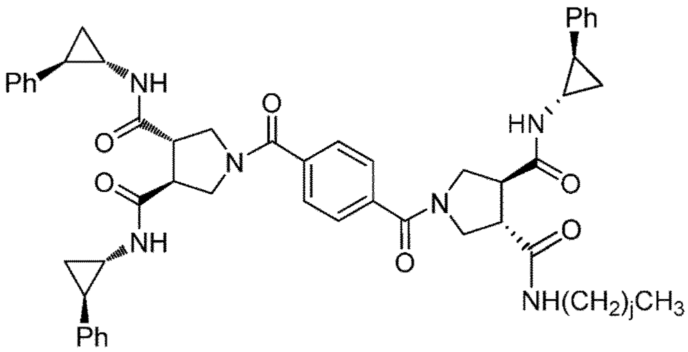

j = 7-17

**I**

Fig. 1A

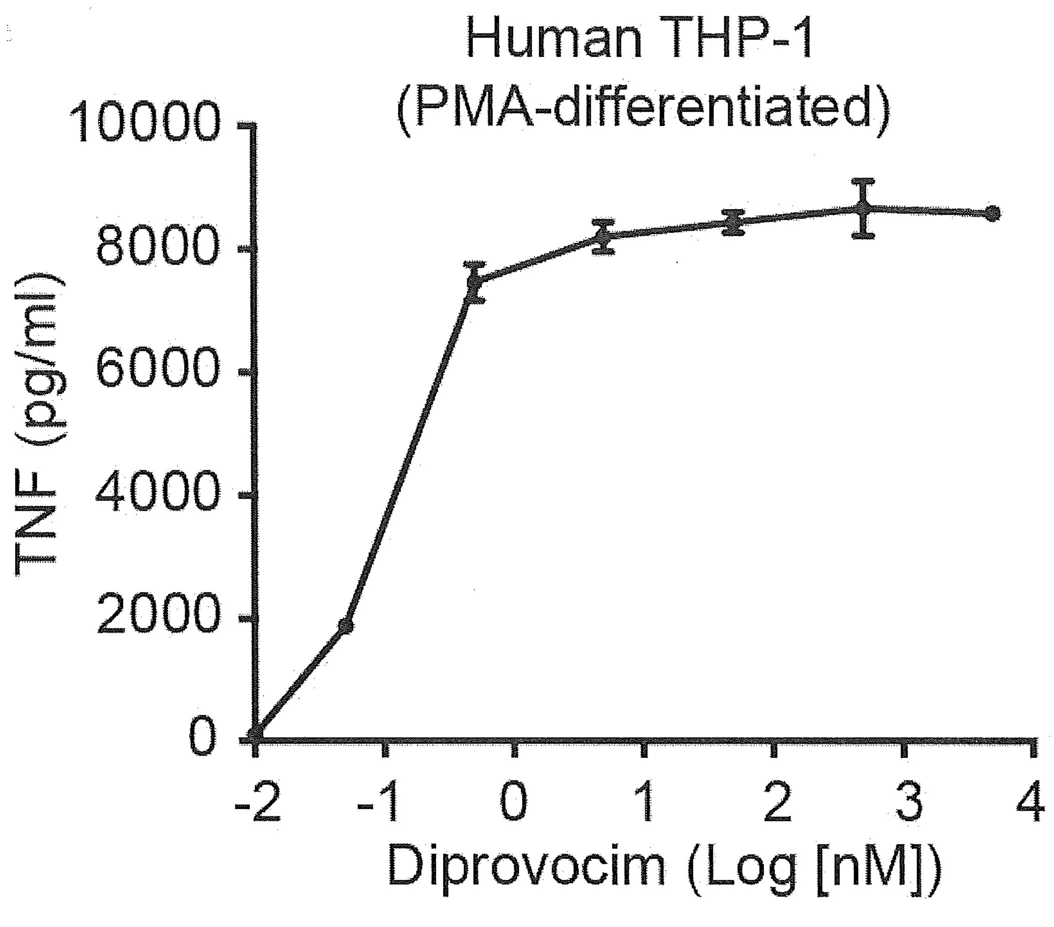
Human THP-1
(PMA-differentiated)
TNF (pg/ml)
10000
8000
6000
4000
2000
0
-2
-1
0
1
2
3
4
Diprovocim (Log [nM])

Fig. 1B

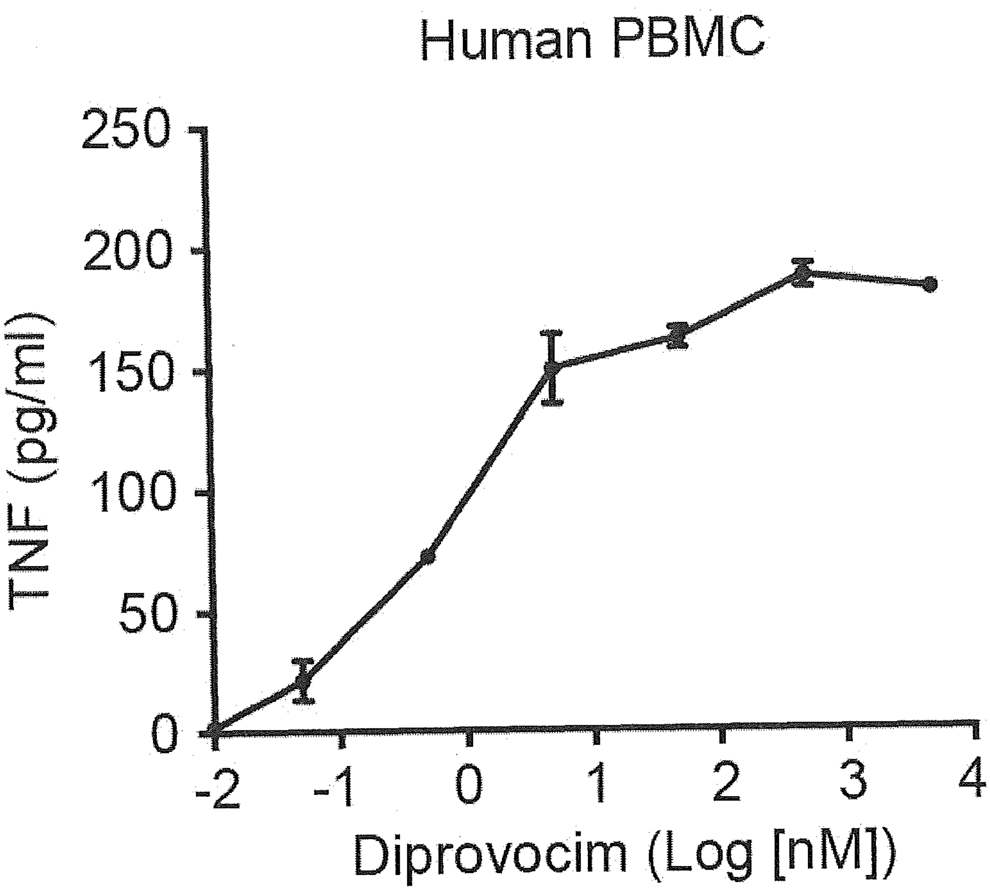
Human PBMC
250
200
150
100
50
0
TNF (pg/ml)
-2
-1
0
1
2
3
4
Diprovocim (Log [nM])

Fig. 1C

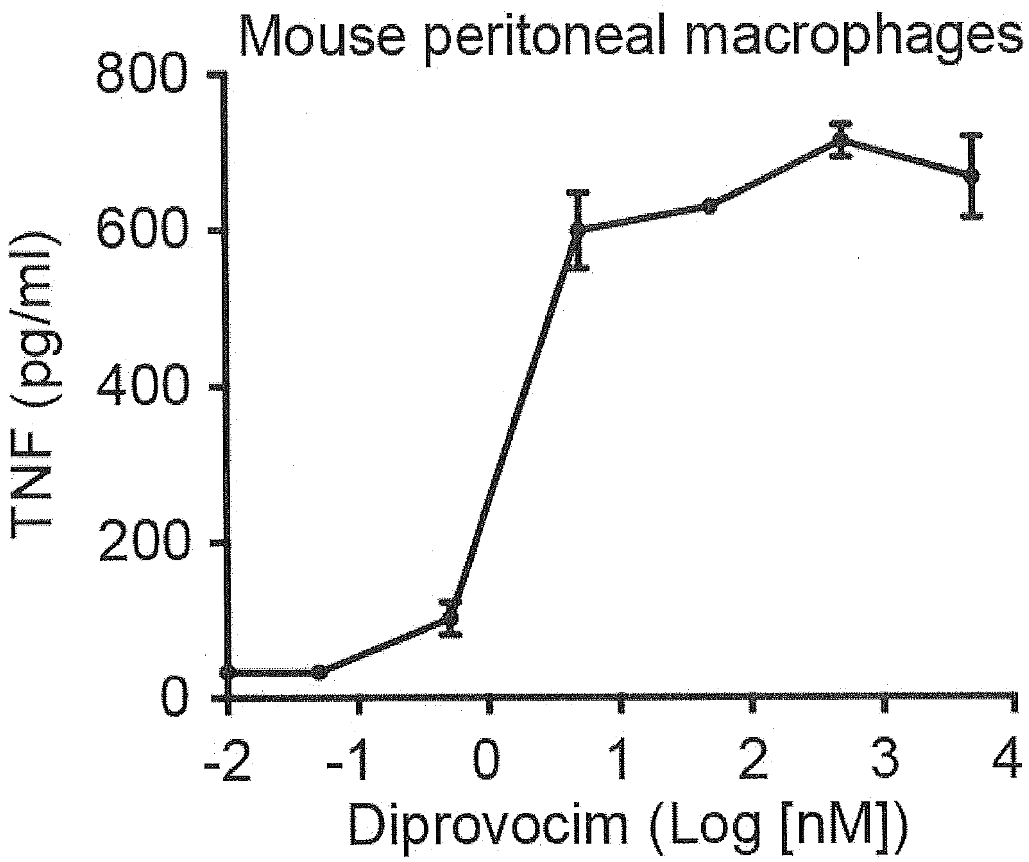
Mouse peritoneal macrophages
800
600
400
200
0
TNF (pg/ml)
-2
-1
0
1
2
3
4
Diprovocim (Log [nM])

Fig. 1D

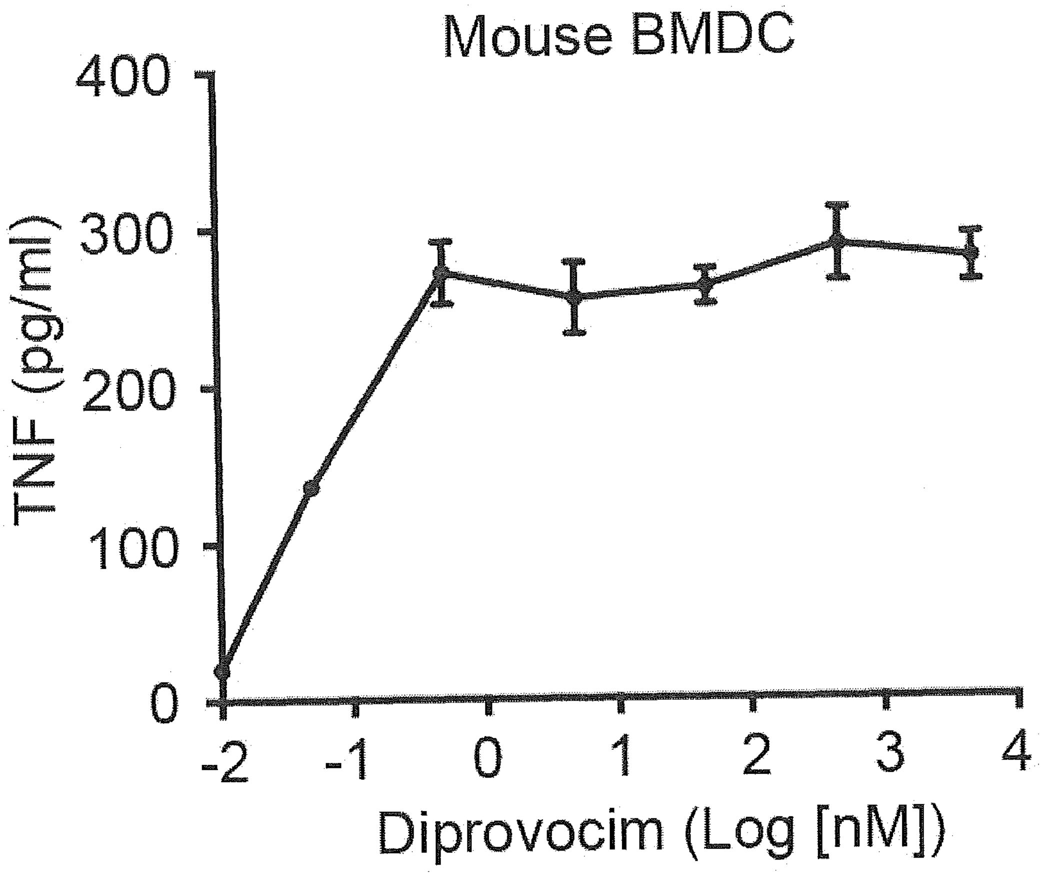
Mouse BMDC
TNF (pg/ml)
400
300
200
100
0
-2
-1
0
1
2
3
4
Diprovocim (Log [nM])

Fig. 1E

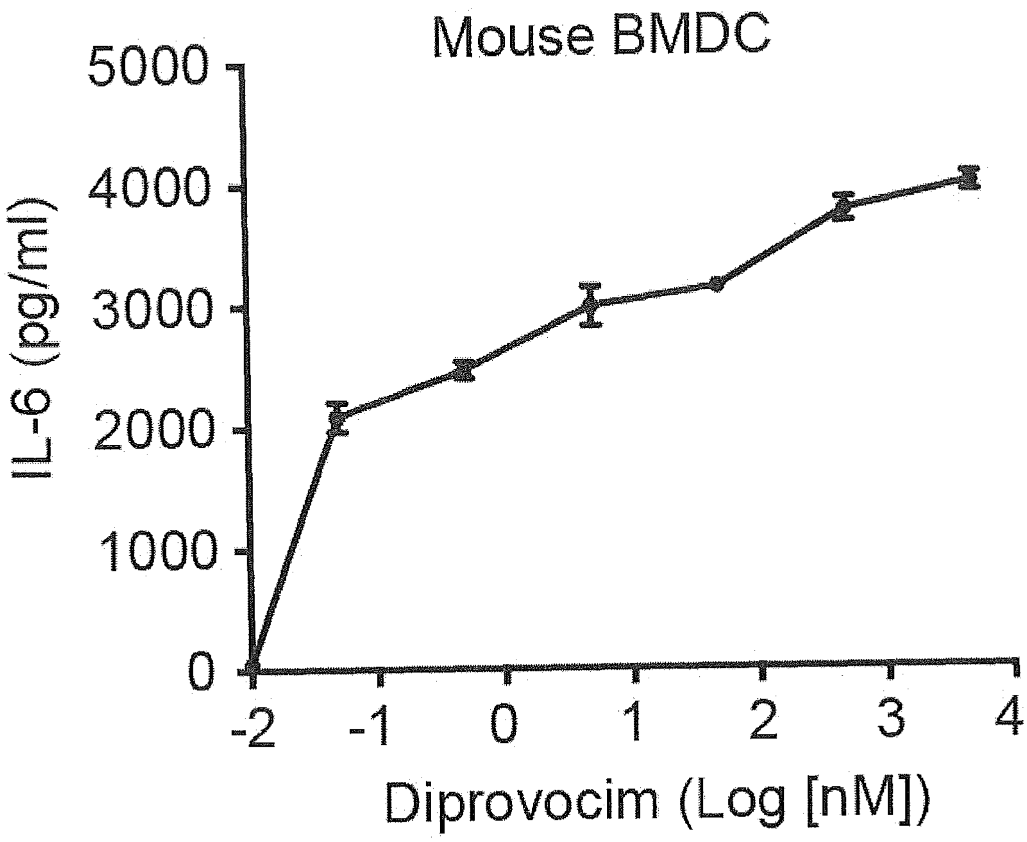
Mouse BMDC
IL-6 (pg/ml)
5000
4000
3000
2000
1000
0
-2
-1
0
1
2
3
4
Diprovocim (Log [nM])

Fig. 2A

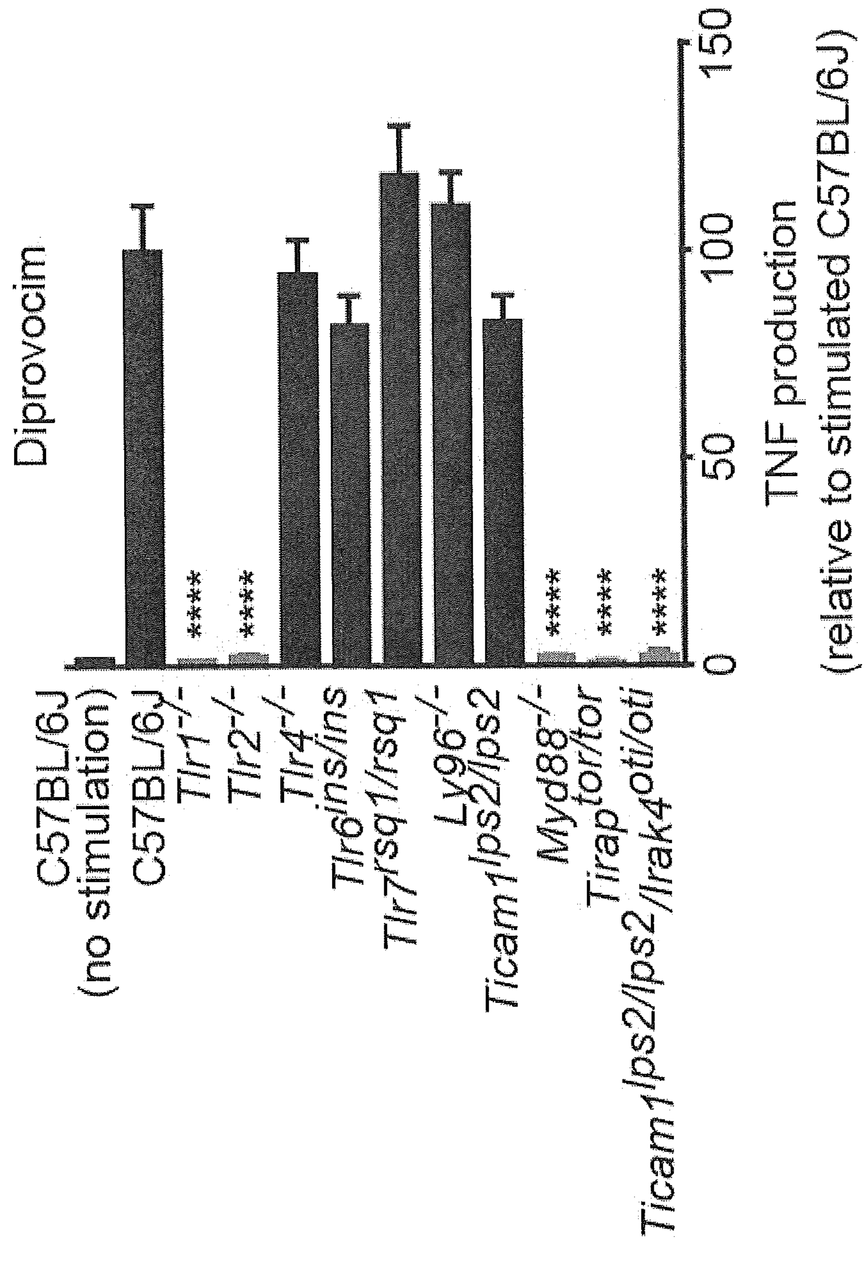
Diprovocim
C57BL/6J (no stimulation)
C57BL/6J
Tlr1-/-
Tlr2-/-
Tlr4-/-
Tlr6ins/ins
Tlr7rsq1/rsq1
Ly96-/-
Ticam1lps2/lps2
Myd88-/-
Tiraptor/tor
Ticam1lps2/lps2/Irak4oti/oti
****
0
50
100
150
TNF production
(relative to stimulated C57BL/6J)

Fig. 2B

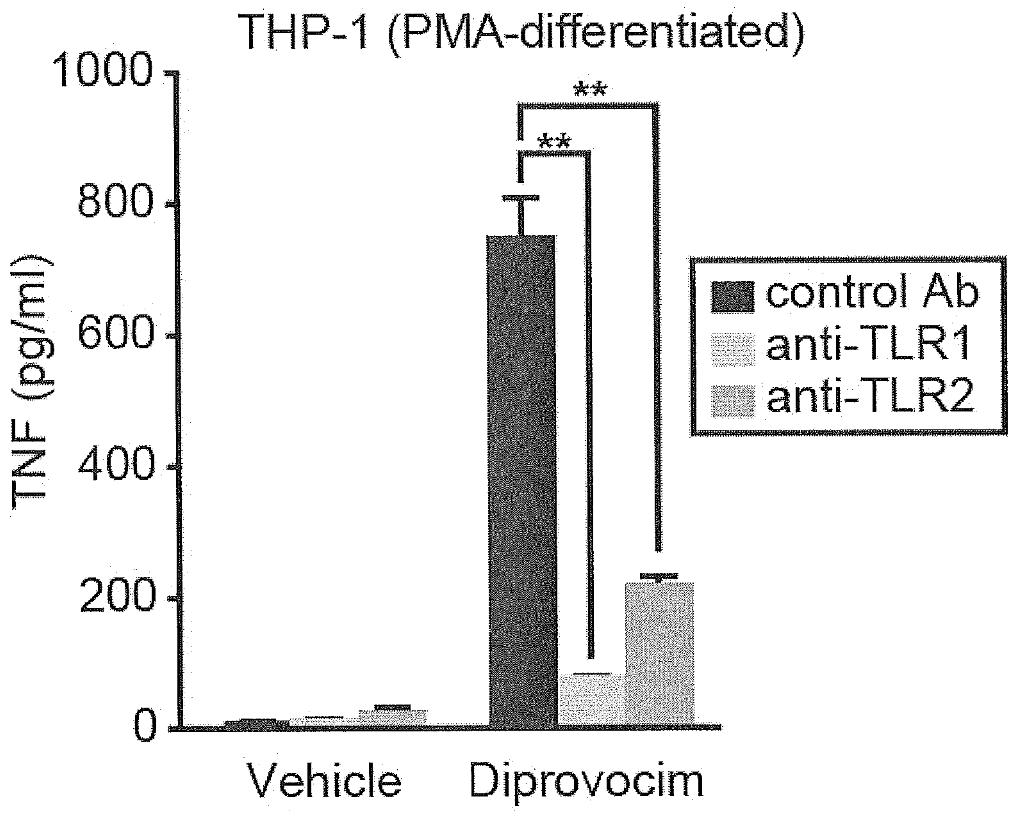
THP-1 (PMA-differentiated)
1000
800
600
400
200
0
TNF (pg/ml)
**
**
control Ab
anti-TLR1
anti-TLR2
Vehicle
Diprovocim

Fig. 2C

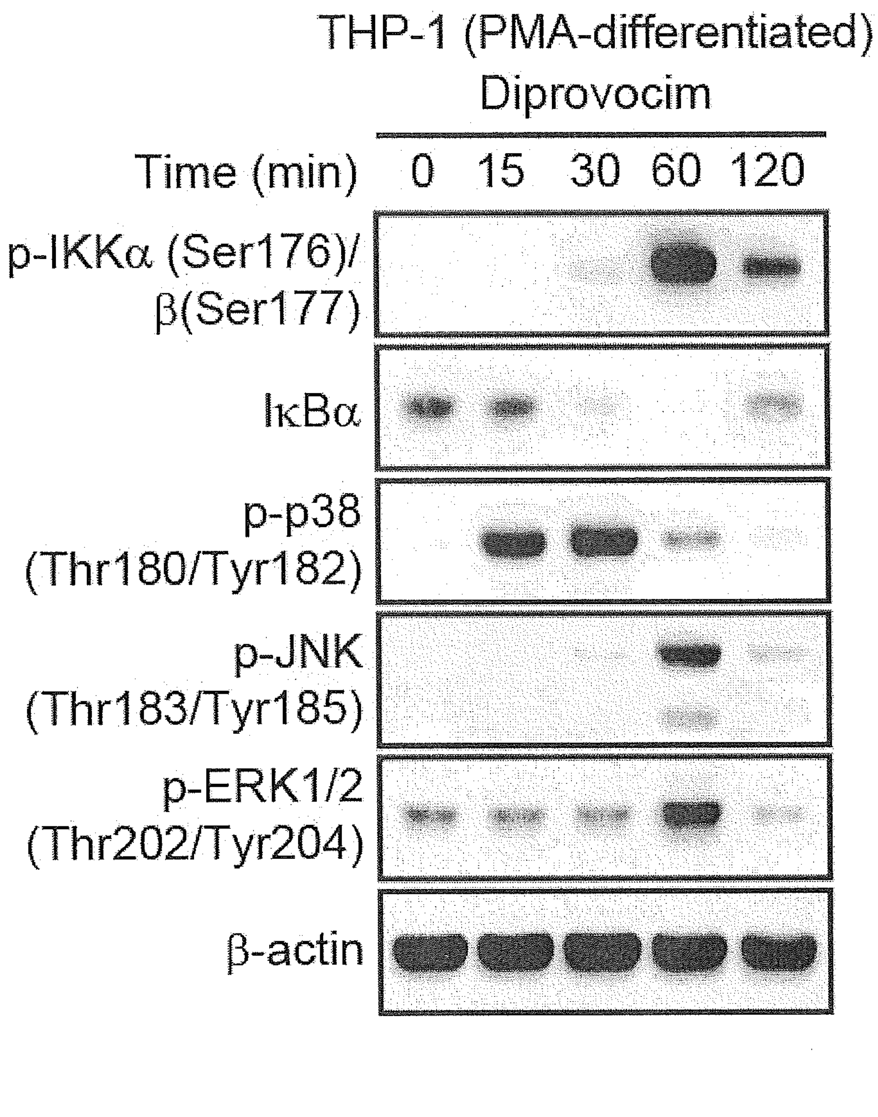
THP-1 (PMA-differentiated)
Diprovocim
Time (min) 0 15 30 60 120
p-IKKα (Ser176)/ β(Ser177)
IκBα
p-p38 (Thr180/Tyr182)
p-JNK (Thr183/Tyr185)
p-ERK1/2 (Thr202/Tyr204)
β-actin

Fig. 2D

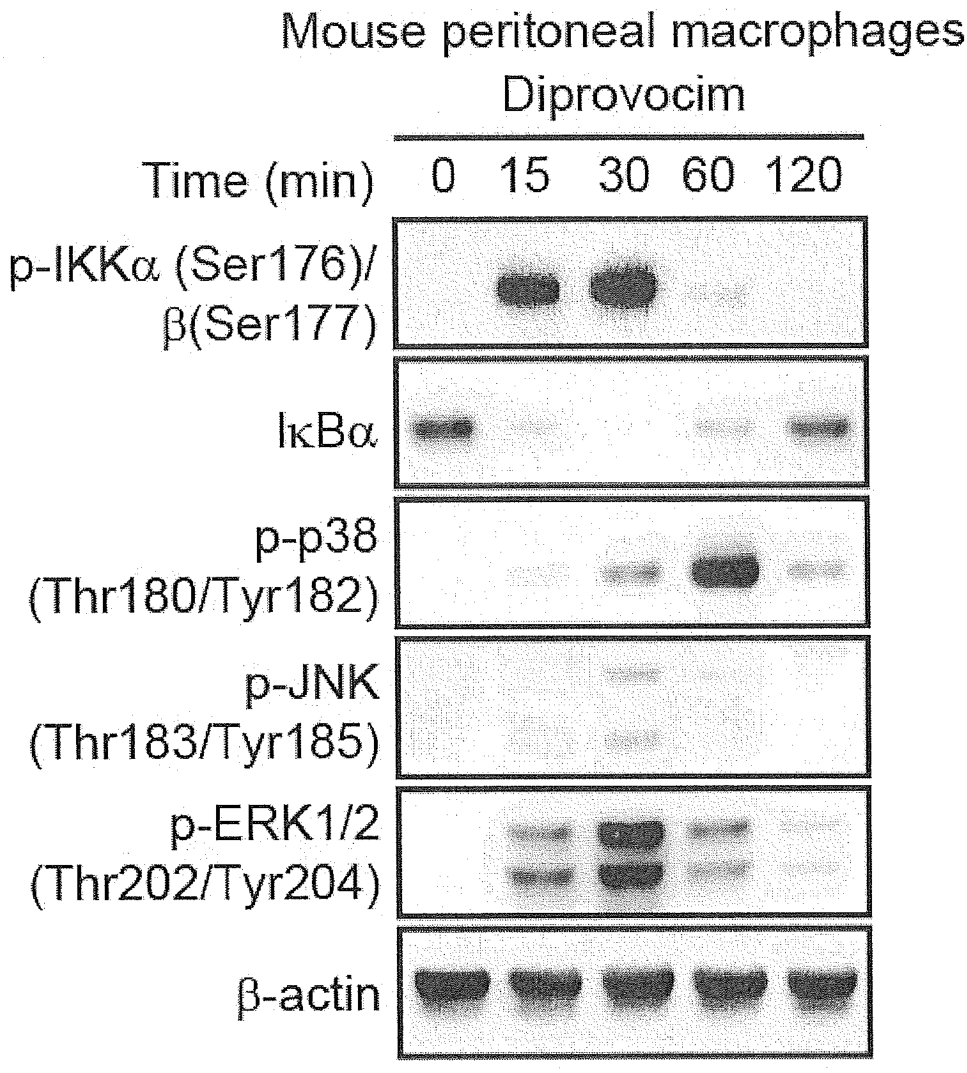
Mouse peritoneal macrophages
Diprovocim
Time (min) 0 15 30 60 120
p-IKKα (Ser176)/
β(Ser177)
IκBα
p-p38
(Thr180/Tyr182)
p-JNK
(Thr183/Tyr185)
p-ERK1/2
(Thr202/Tyr204)
β-actin

Fig. 3A

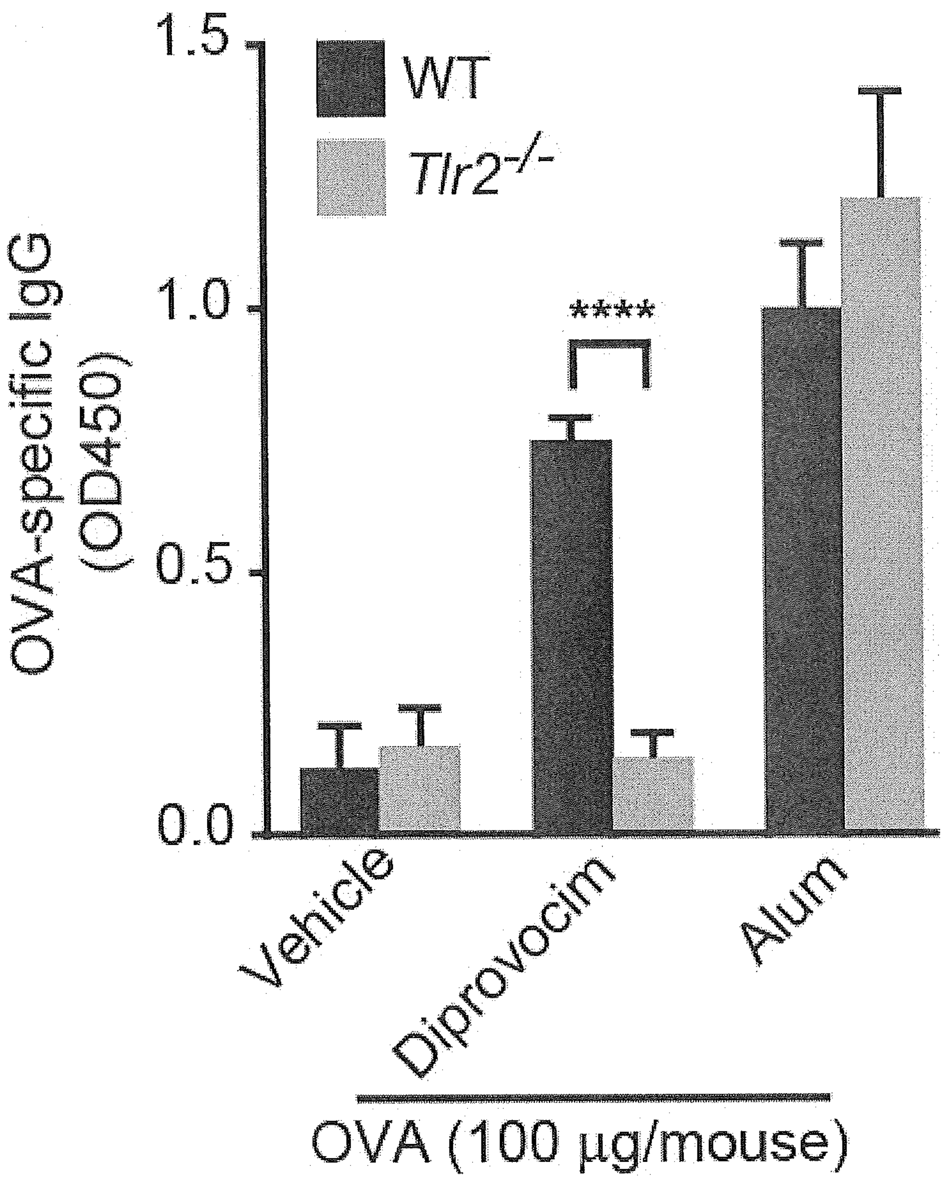
1.5
1.0
0.5
0.0
OVA-specific IgG
(OD450)
WT
Tlr2-/-
****
Vehicle
Diprovocim
Alum
OVA (100 μg/mouse)

Fig. 3B

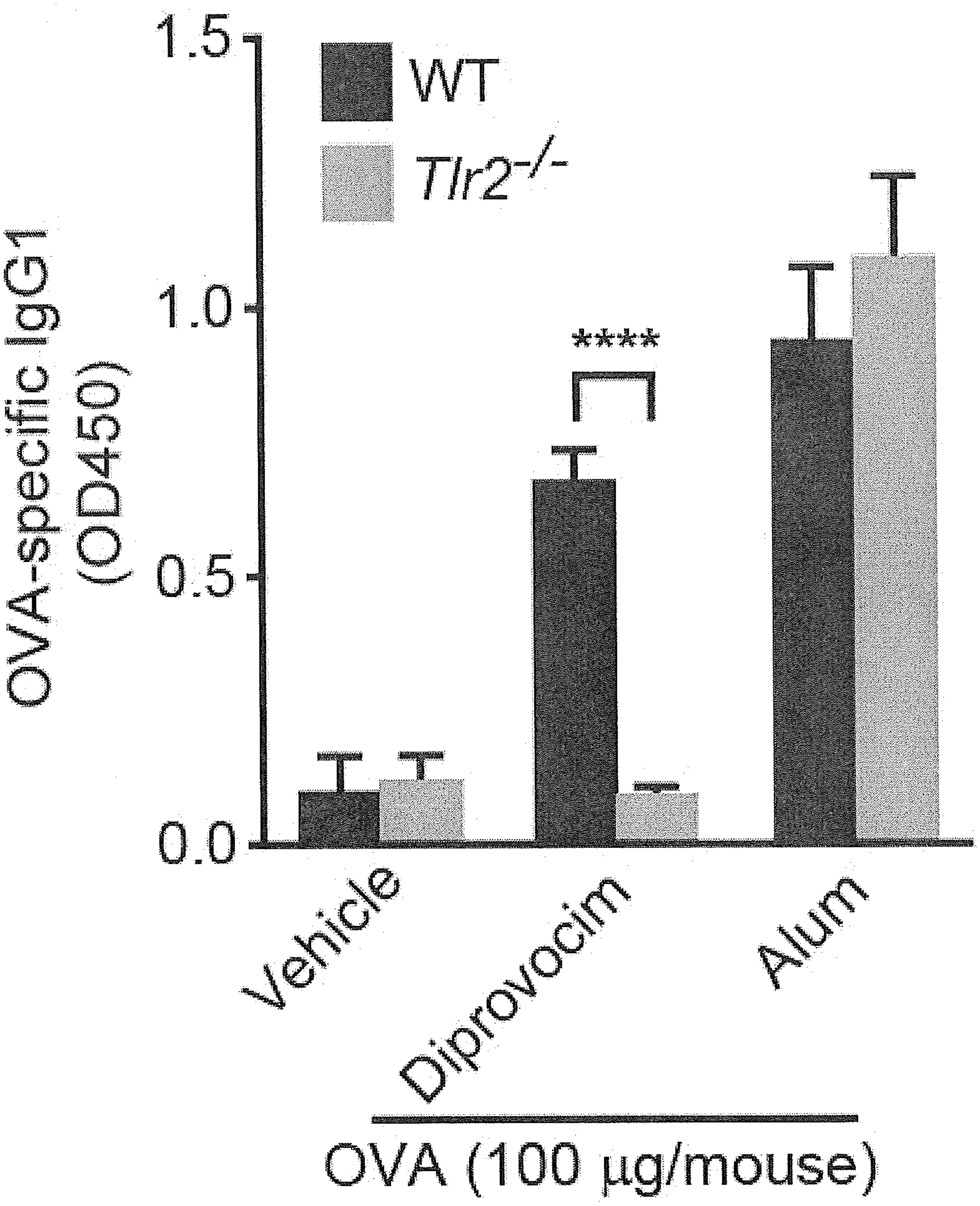
1.5
1.0
0.5
0.0
OVA-specific IgG1 (OD450)
WT
Tlr2-/-
****
Vehicle
Diprovocim
Alum
OVA (100 μg/mouse)

Fig. 3C

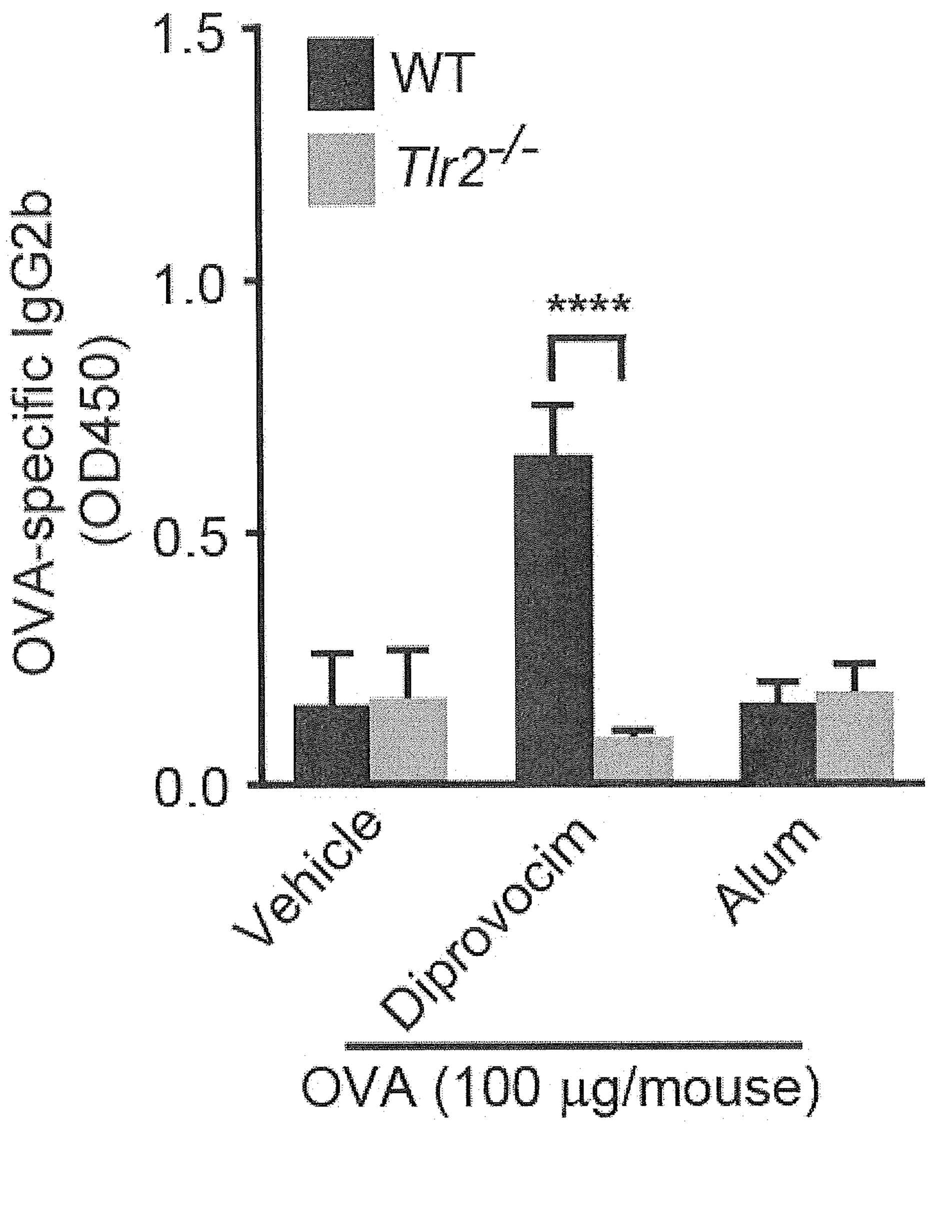
1.5
1.0
0.5
0.0
WT
Tlr2-/-
OVA-specific IgG2b (OD450)
****
Vehicle
Diprovocim
Alum
OVA (100 µg/mouse)

# Fig. 3D

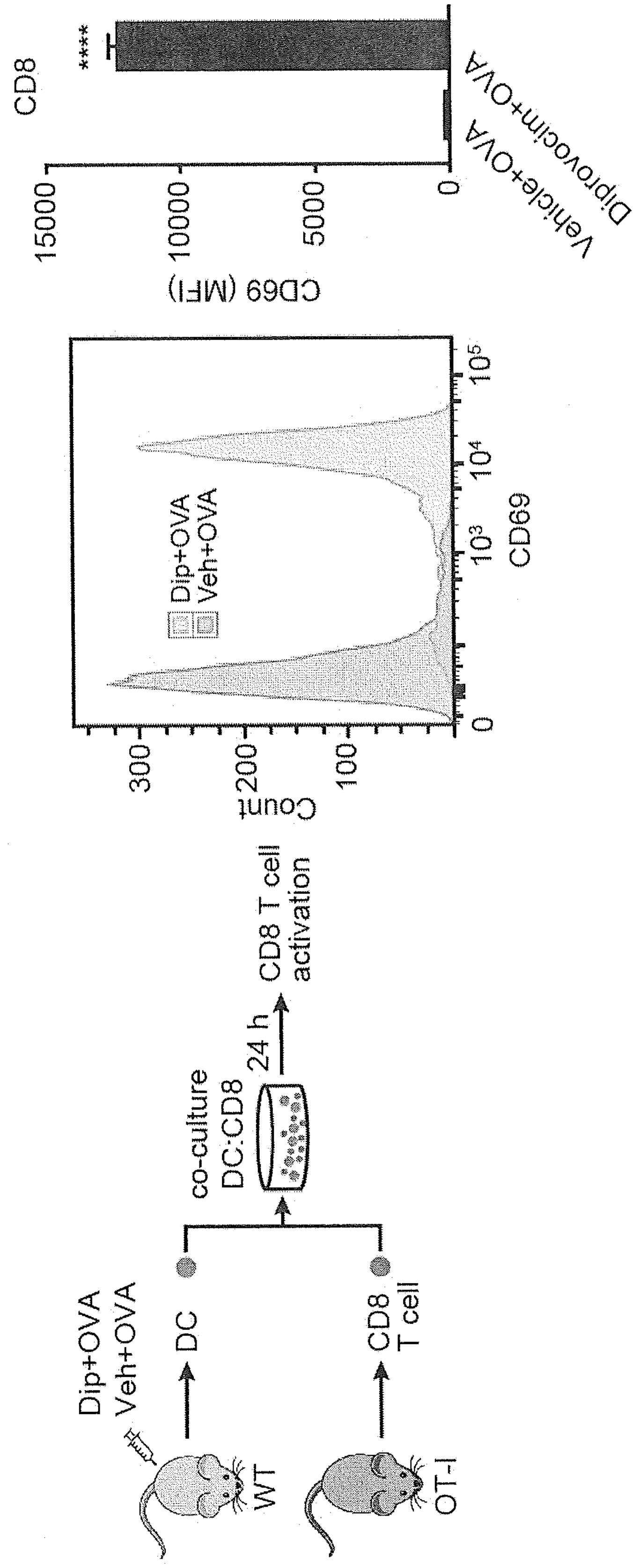

Dip+OVA
Veh+OVA
WT
DC
OT-I
CD8
T cell
co-culture
DC:CD8
24 h
CD8 T cell
activation
Count
300
200
100
0
CD69
Dip+OVA
Veh+OVA
CD8
CD69 (MFI)
15000
10000
5000
0
****
Vehicle+OVA
Diprovocim+OVA

Fig. 3E

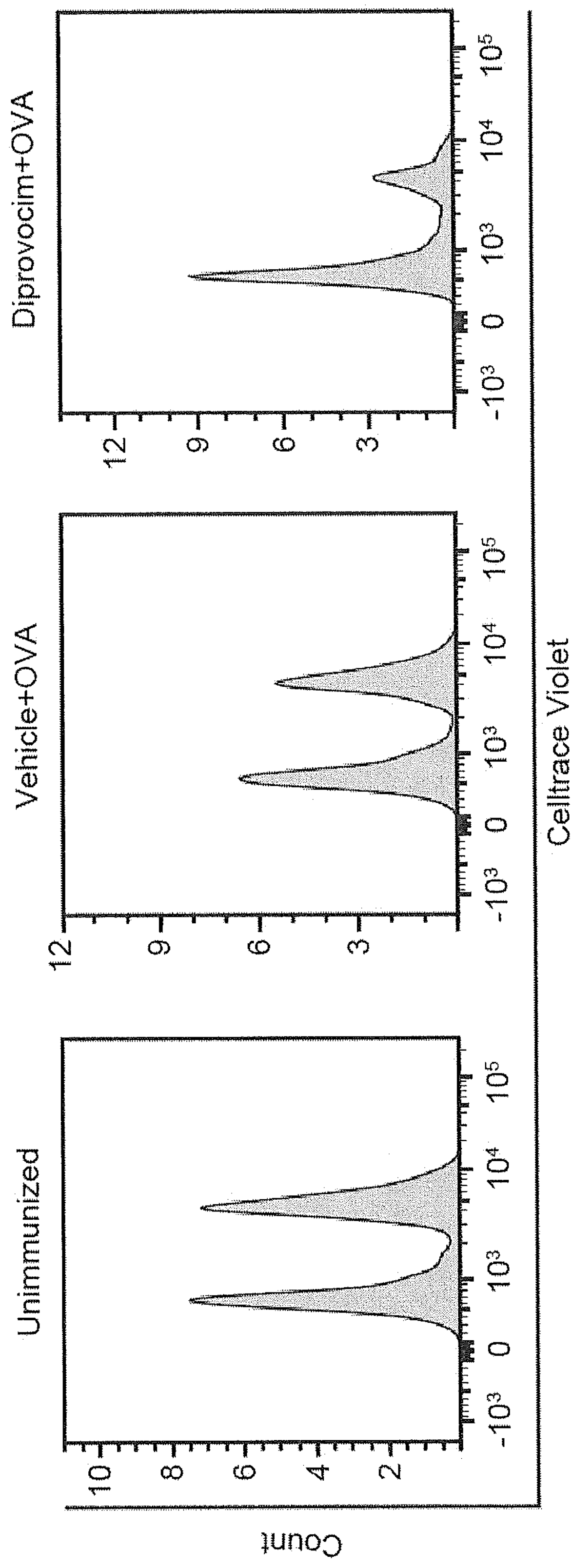
Unimmunized
Vehicle+OVA
Diprovocim+OVA
Count
Celltrace Violet
10
8
6
4
2
12
9
6
3
-10³
0
10³
10⁴
10⁵

Fig. 3F

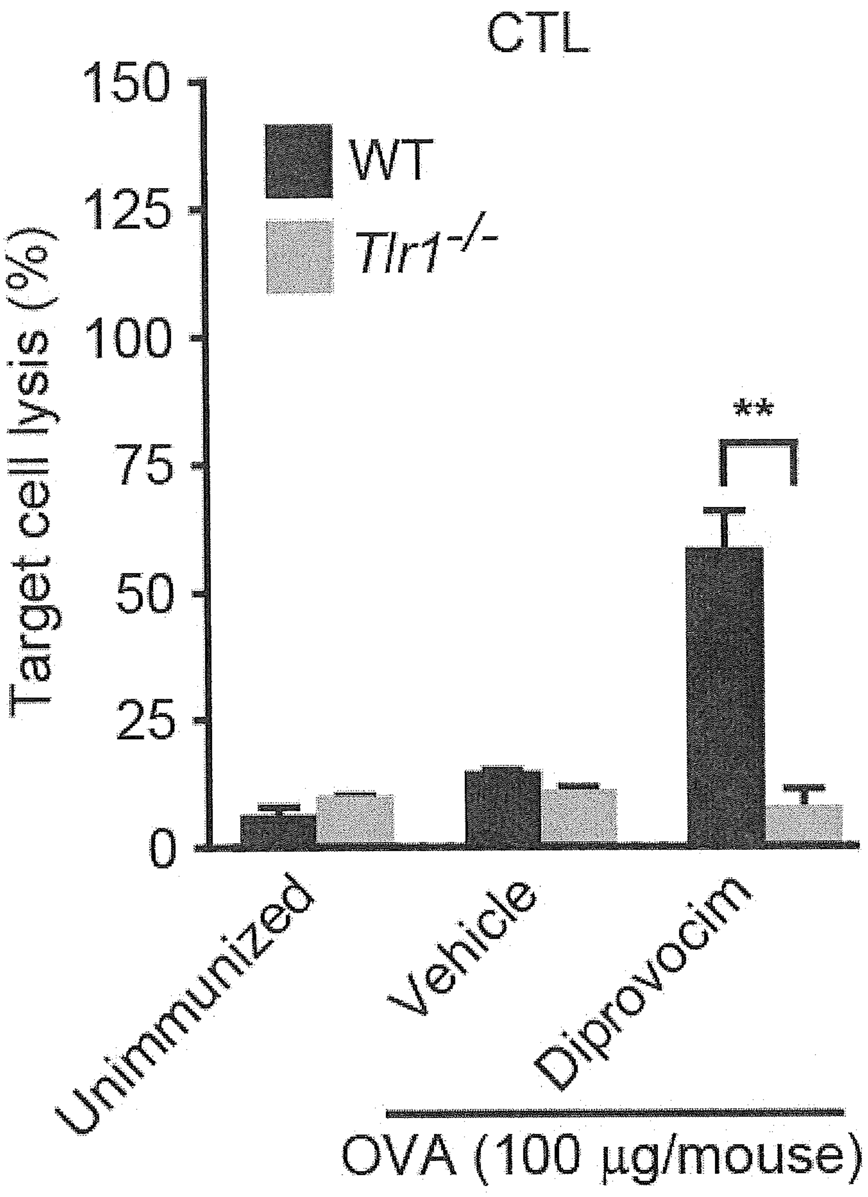
CTL
150
125
100
75
50
25
0
Target cell lysis (%)
WT
Tlr1-/-
**
Unimmunized
Vehicle
Diprovocim
OVA (100 µg/mouse)

Fig. 3G

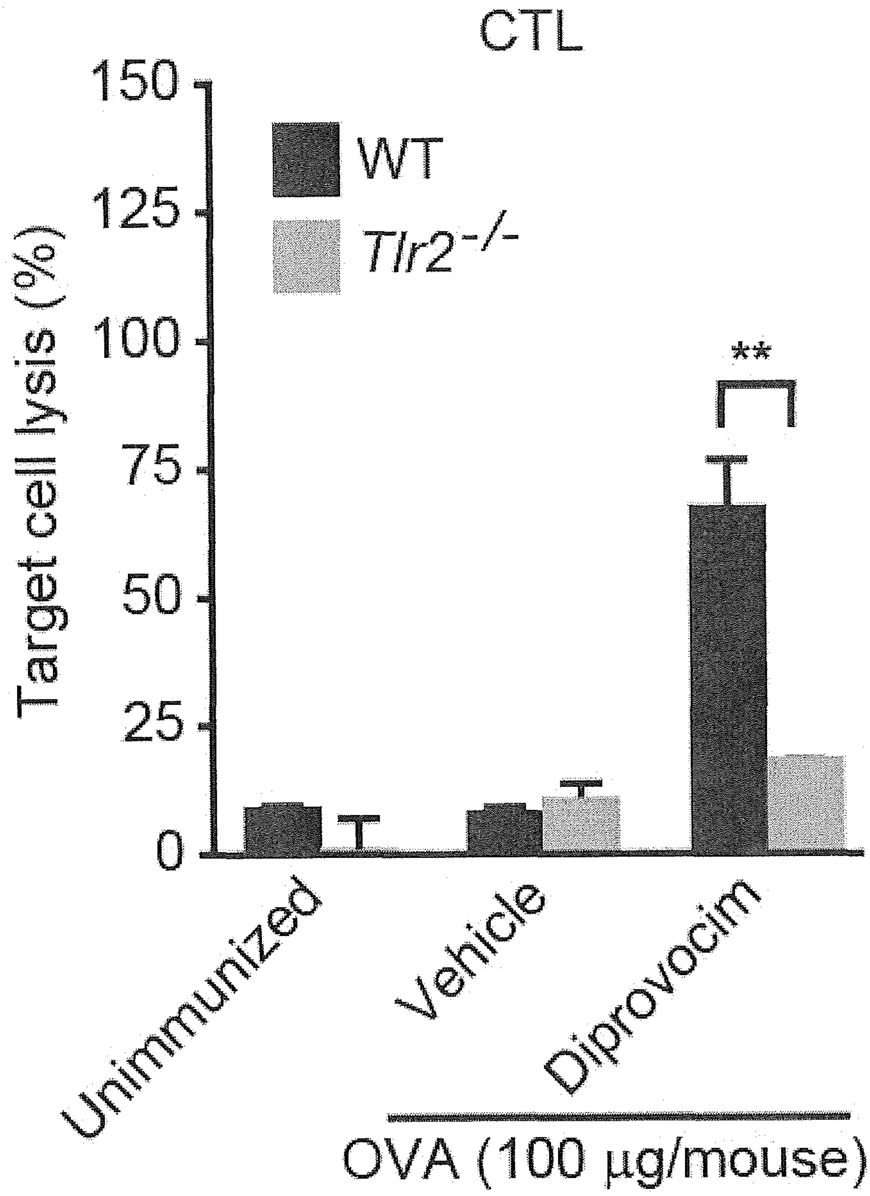
CTL
Target cell lysis (%)
150
125
100
75
50
25
0
WT
Tlr2-/-
**
Unimmunized
Vehicle
Diprovocim
OVA (100 µg/mouse)

Fig. 4A

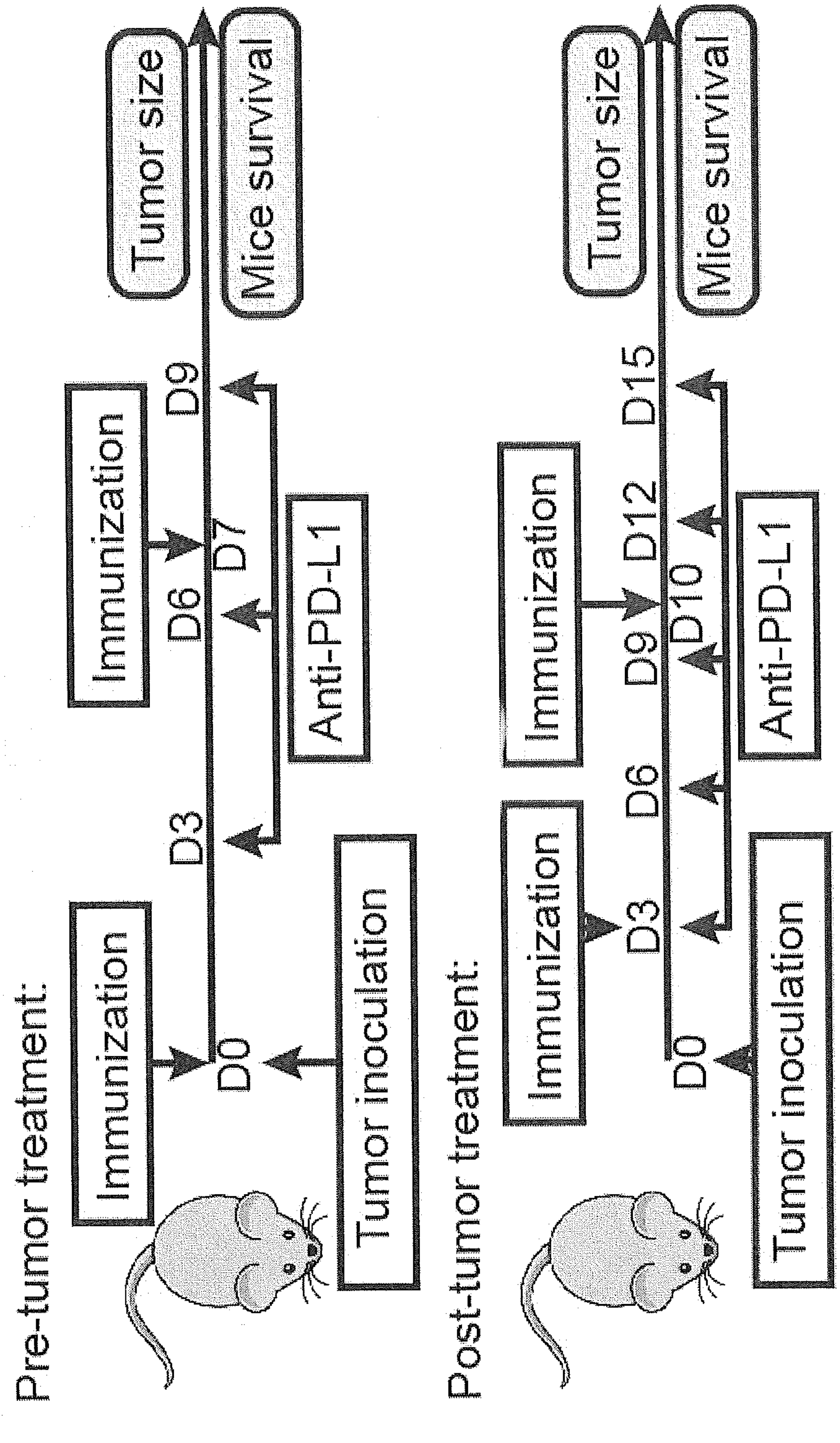
Pre-tumor treatment:
Immunization
Tumor inoculation
D0
D3
D6
D7
D9
Immunization
Anti-PD-L1
Tumor size
Mice survival
Post-tumor treatment:
Immunization
Tumor inoculation
D0
D3
D6
D9
D10
D12
D15
Immunization
Immunization
Anti-PD-L1
Tumor size
Mice survival

Fig. 4B

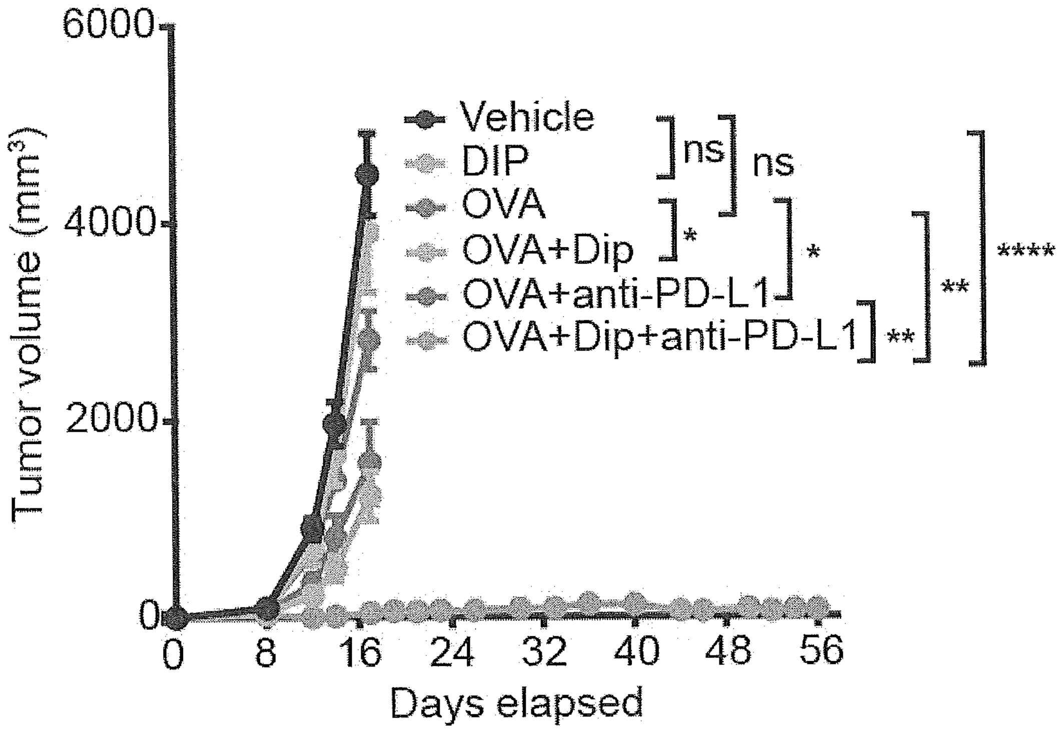
6000
4000
2000
0
Tumor volume (mm3)
0
8
16
24
32
40
48
56
Days elapsed
Vehicle
DIP
OVA
OVA+Dip
OVA+anti-PD-L1
OVA+Dip+anti-PD-L1
ns
ns
*
*
**
**
****

Fig. 4C

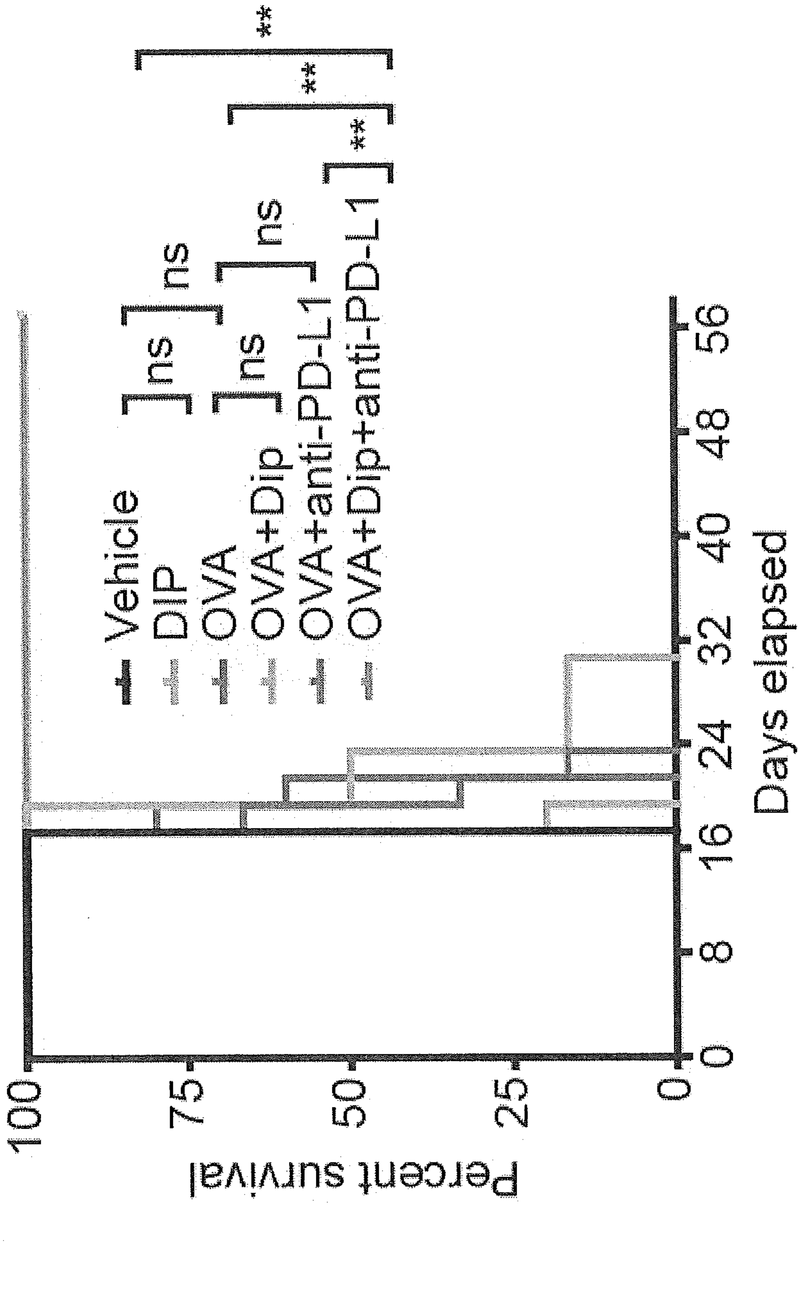
Percent survival
100
75
50
25
0
0
8
16
24
32
40
48
56
Days elapsed
Vehicle
DIP
OVA
OVA+Dip
OVA+anti-PD-L1
OVA+Dip+anti-PD-L1
ns
ns
ns
ns
**
**
**

Fig. 4D

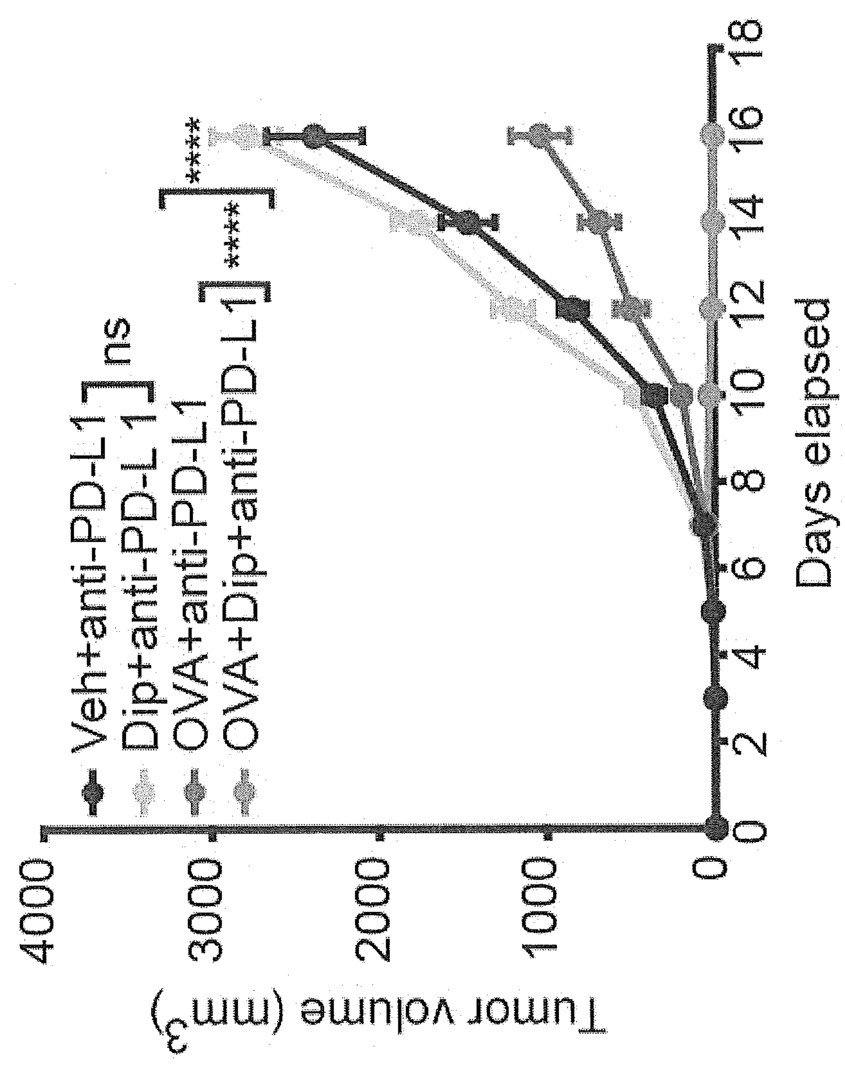
Veh+anti-PD-L1
Dip+anti-PD-L 1
OVA+anti-PD-L1
OVA+Dip+anti-PD-L1
ns
****
****
Tumor volume (mm³)
4000
3000
2000
1000
0
0
2
4
6
8
10
12
14
16
18
Days elapsed

Fig. 4E

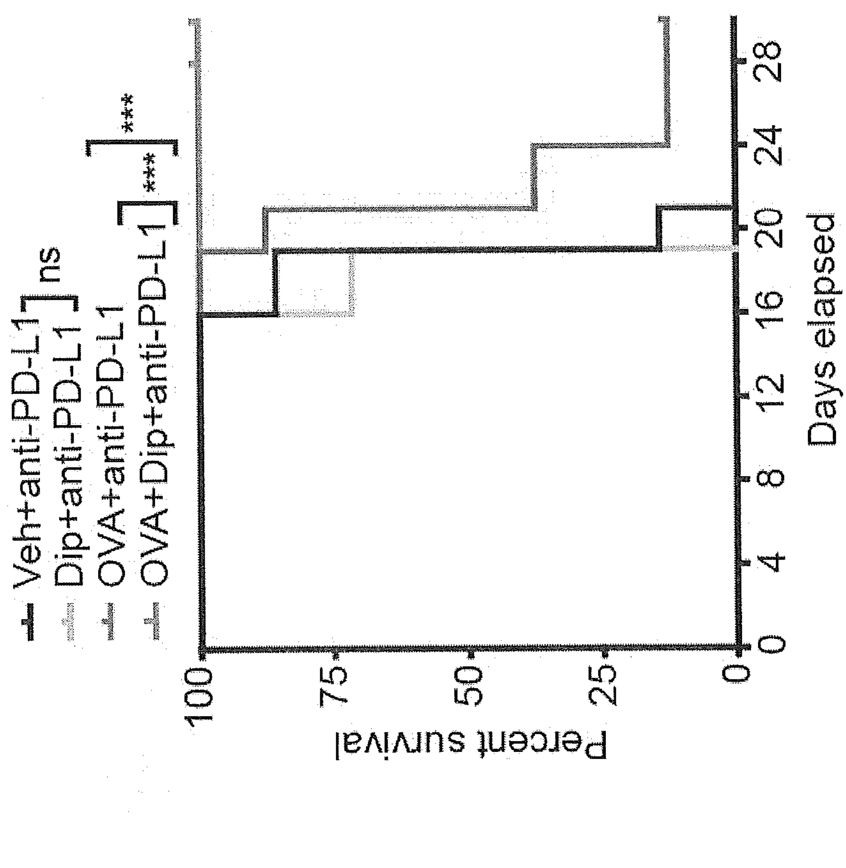

Veh+anti-PD-L1
Dip+anti-PD-L1
ns
OVA+anti-PD-L1
OVA+Dip+anti-PD-L1
***
***
Percent survival
100
75
50
25
0
0
4
8
12
16
20
24
28
Days elapsed

Fig. 4F

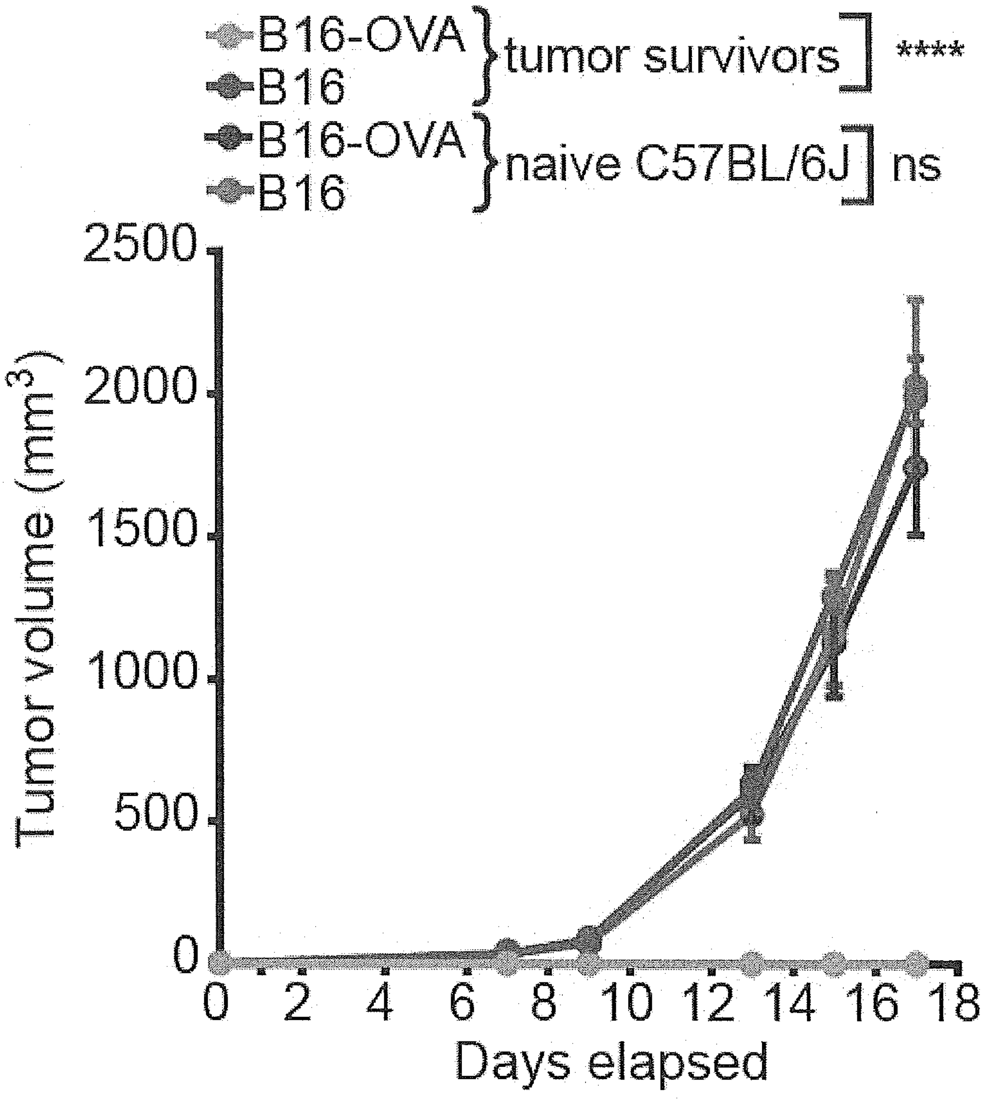
B16-OVA
B16
tumor survivors
****
B16-OVA
B16
naive C57BL/6J
ns
Tumor volume (mm3)
2500
2000
1500
1000
500
0
0 2 4 6 8 10 12 14 16 18
Days elapsed

Fig. 4G

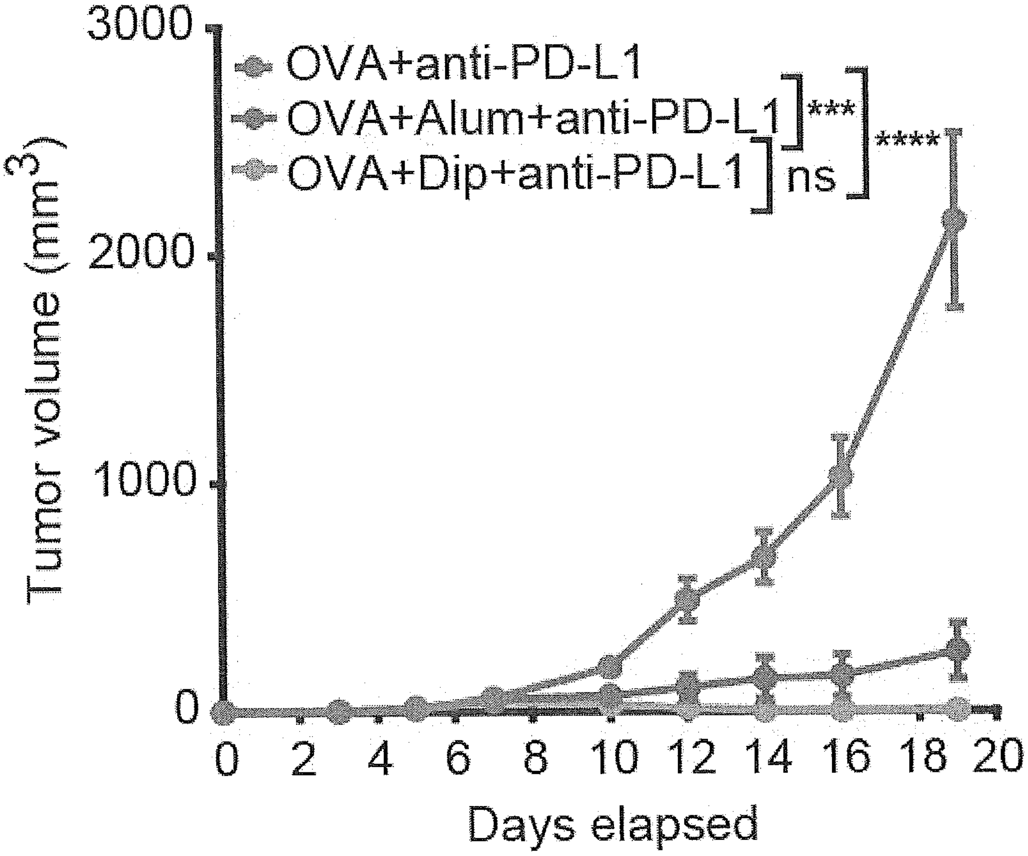
3000
2000
1000
0
Tumor volume (mm3)
OVA+anti-PD-L1
OVA+Alum+anti-PD-L1
OVA+Dip+anti-PD-L1
***
****
ns
0 2 4 6 8 10 12 14 16 18 20
Days elapsed

Fig. 4H

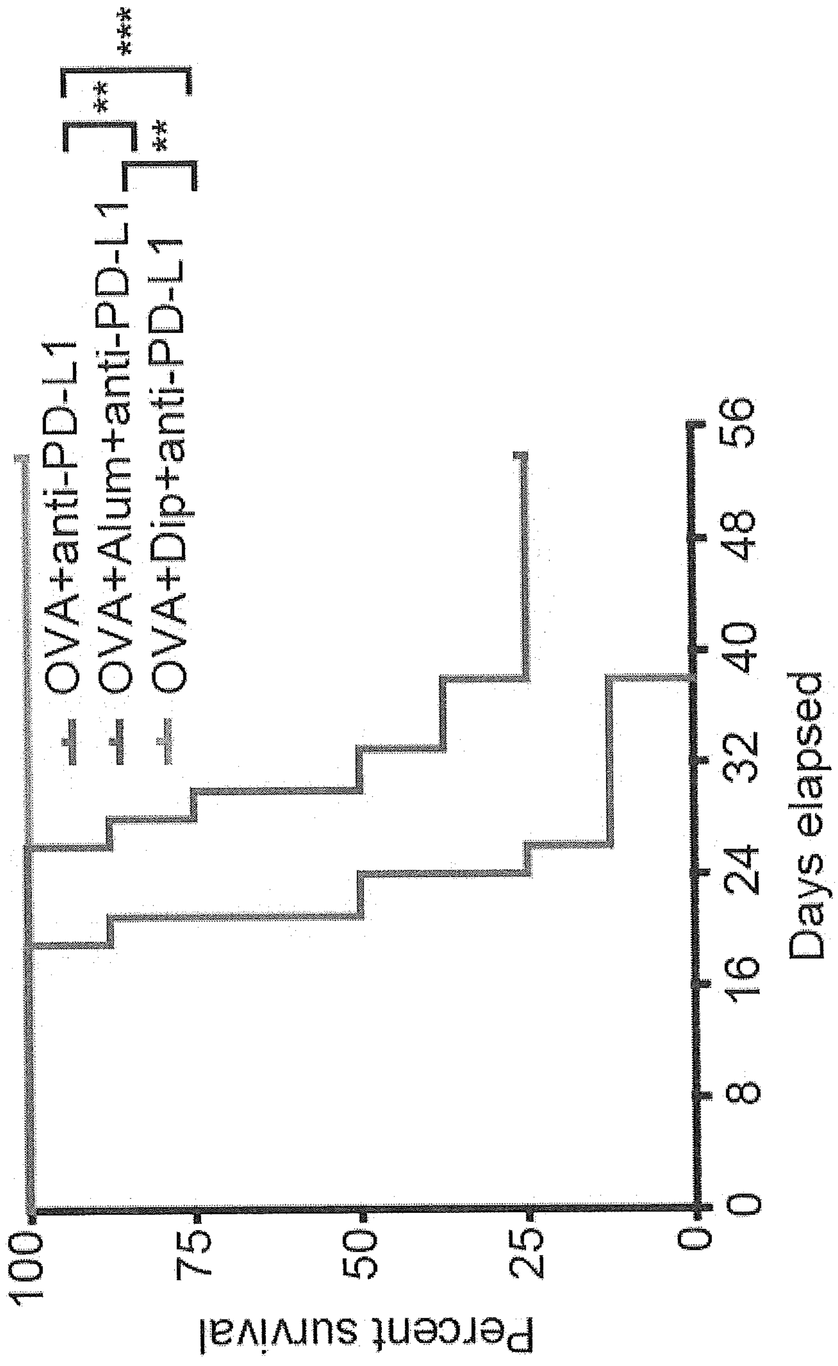

OVA+anti-PD-L1
OVA+Alum+anti-PD-L1
OVA+Dip+anti-PD-L1
**
**
***
Percent survival
100
75
50
25
0
0
8
16
24
32
40
48
56
Days elapsed

Fig. 41

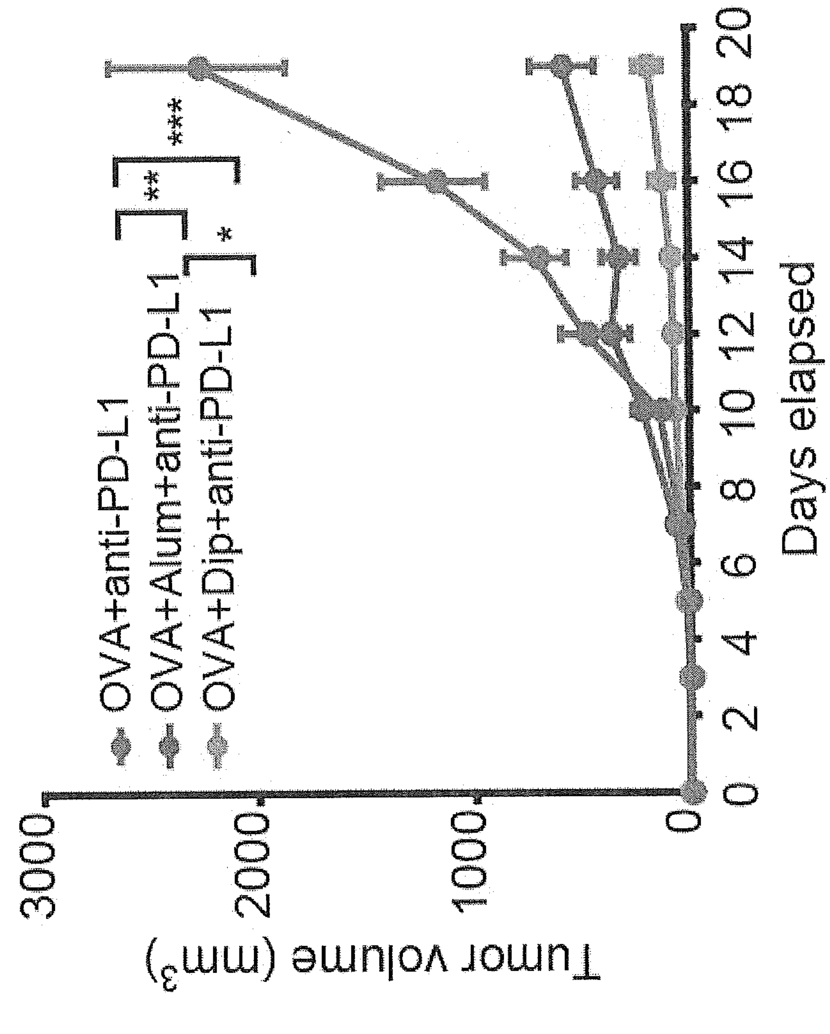
OVA+anti-PD-L1
OVA+Alum+anti-PD-L1
OVA+Dip+anti-PD-L1
**
***
*
Tumor volume (mm3)
3000
2000
1000
0
0
2
4
6
8
10
12
14
16
18
20
Days elapsed

Fig. 4J

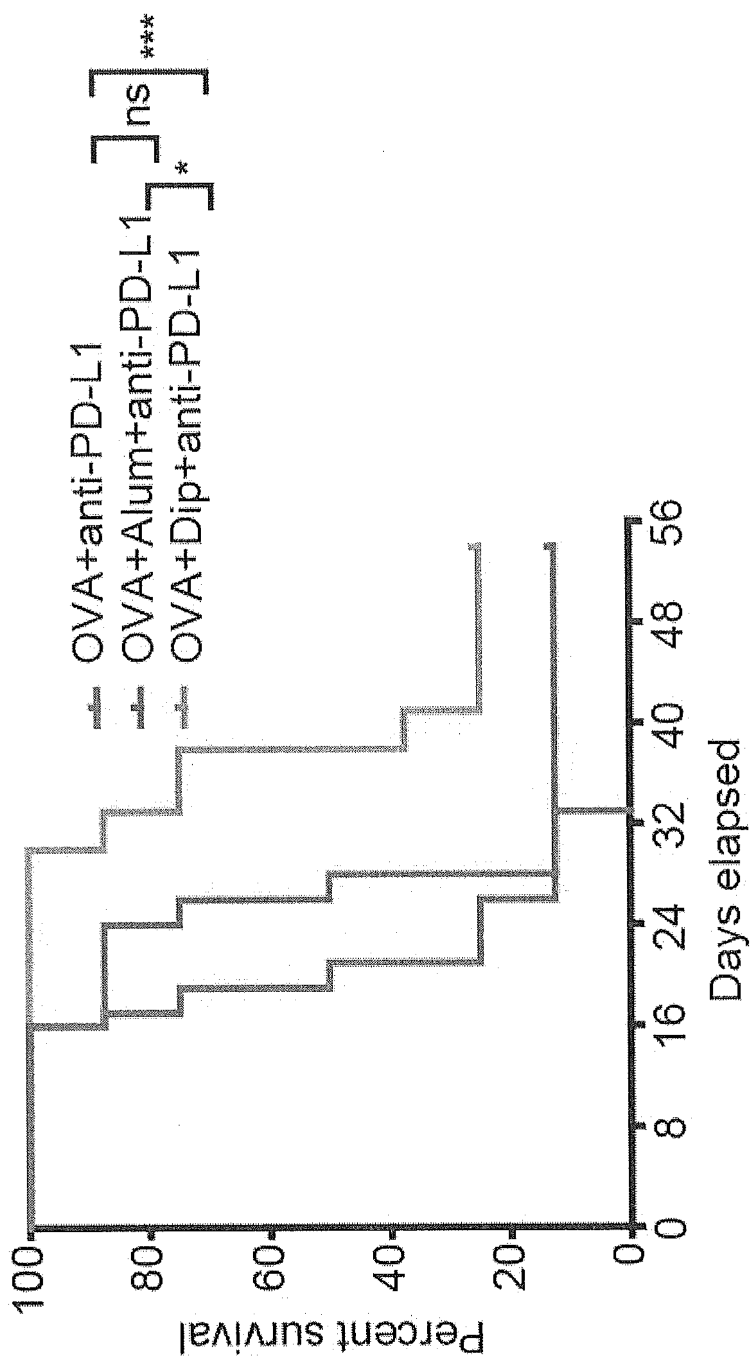
OVA+anti-PD-L1
OVA+Alum+anti-PD-L1
OVA+Dip+anti-PD-L1
ns
*
***
Percent survival
100
80
60
40
20
0
0
8
16
24
32
40
48
56
Days elapsed

Fig. 5A

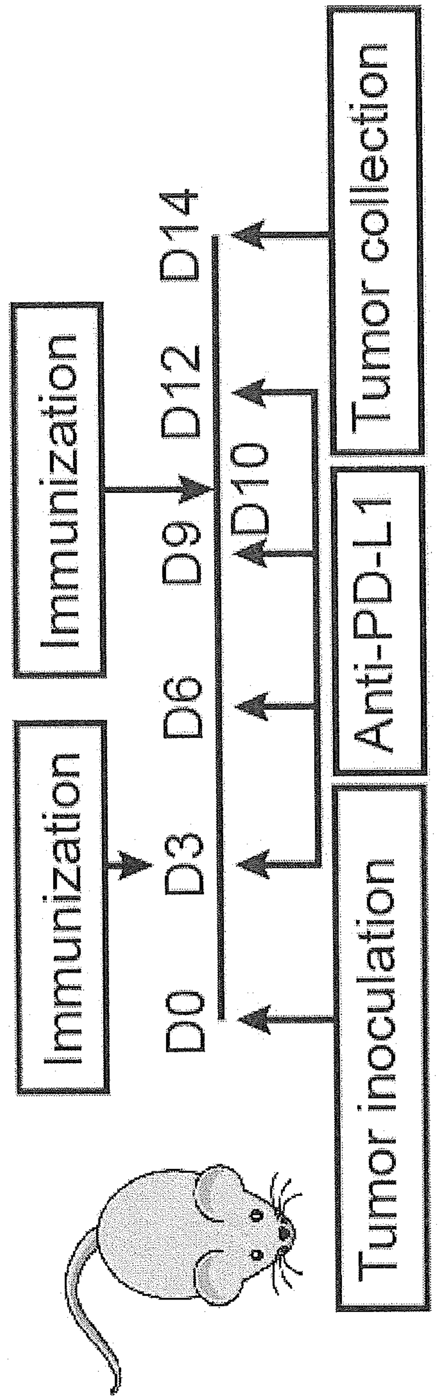
Immunization
Immunization
D0
D3
D6
D9
D10
D12
D14
Tumor inoculation
Anti-PD-L1
Tumor collection

Fig. 5B

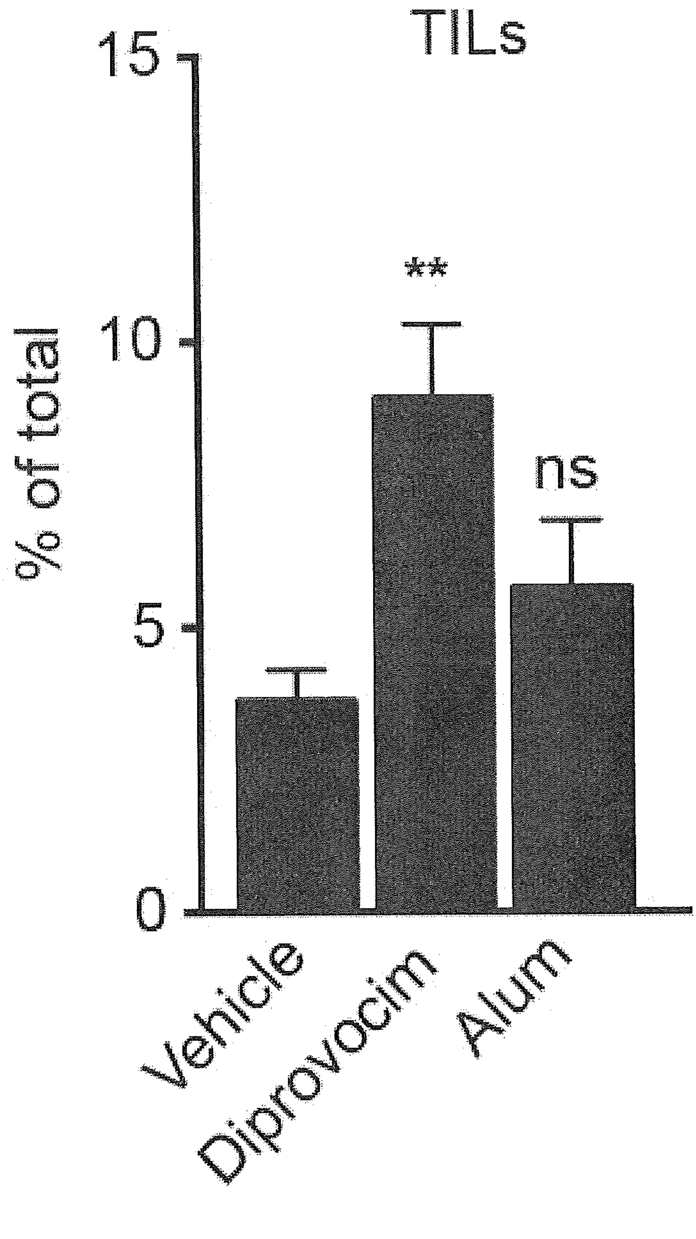

Fig. 5C

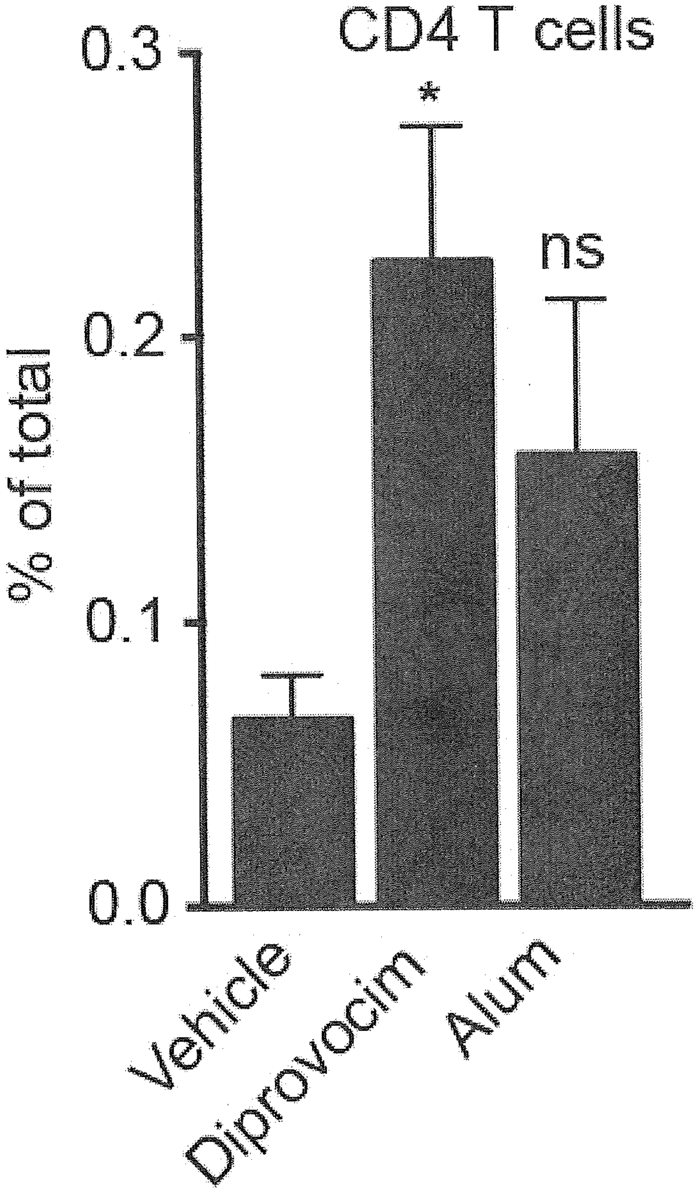

Fig. 5D

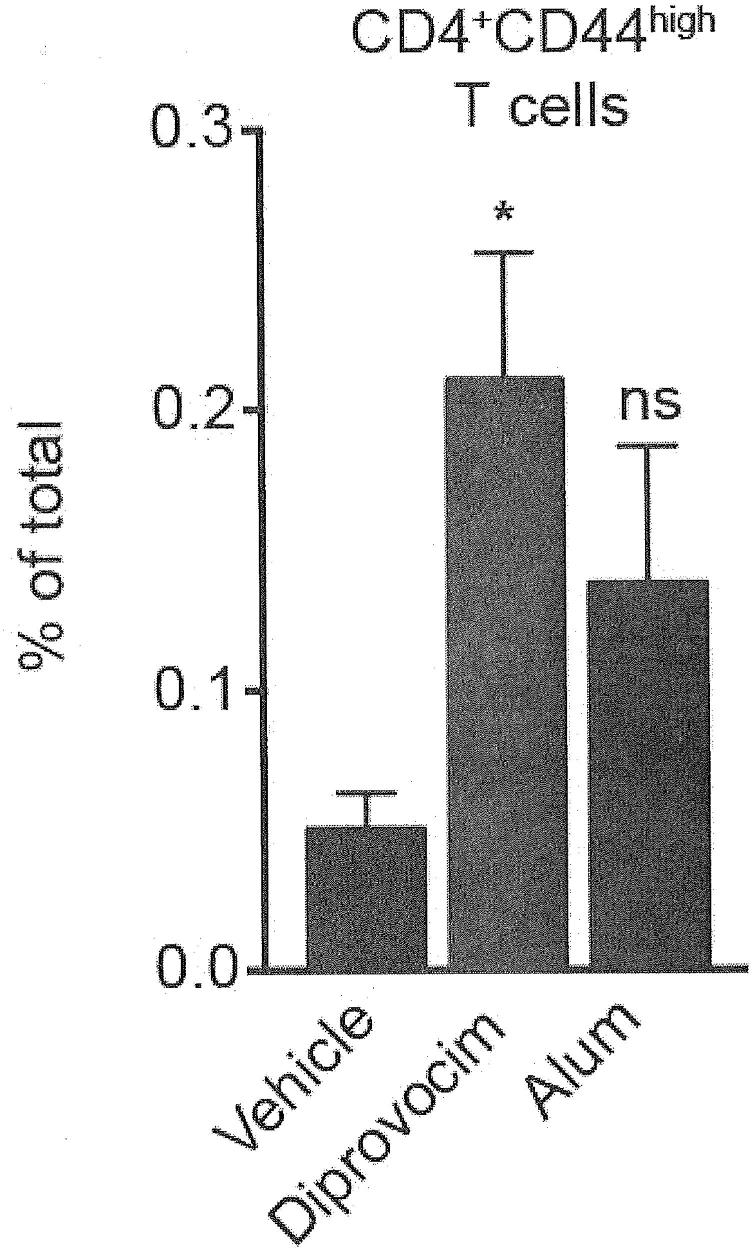
CD4+CD44high
T cells
% of total
0.3
0.2
0.1
0.0
*
ns
Vehicle
Diprovocim
Alum

Fig. 5E

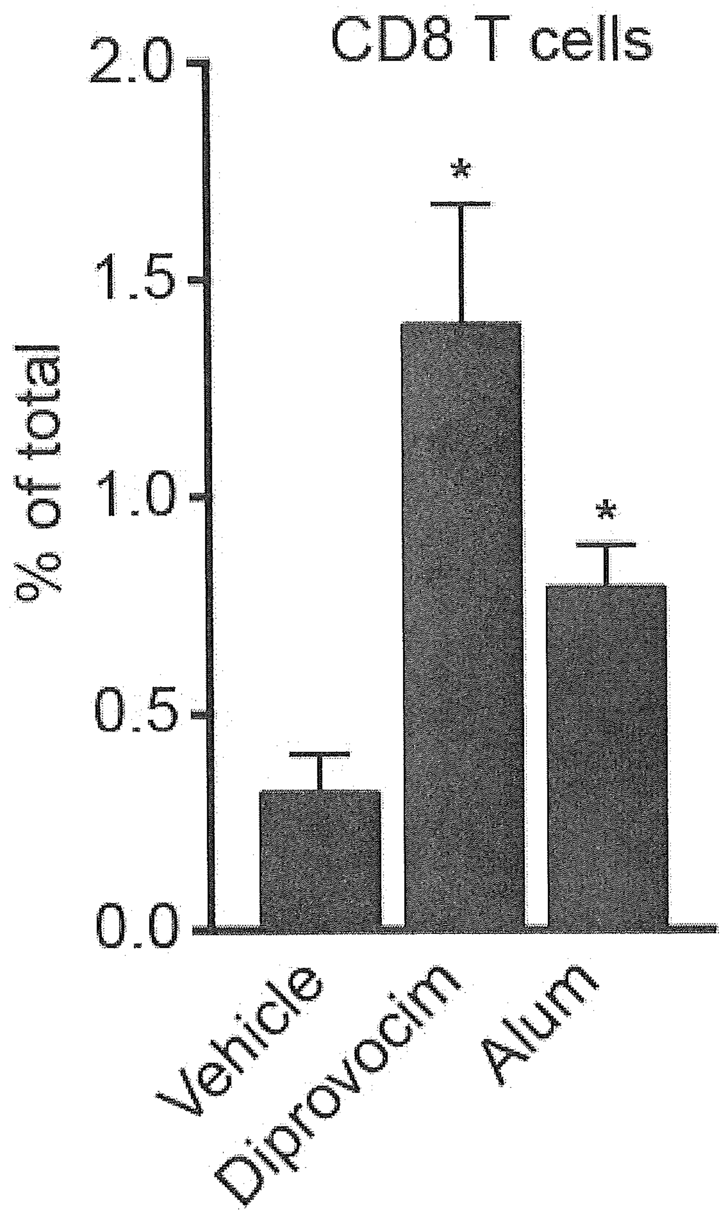

Fig. 5F

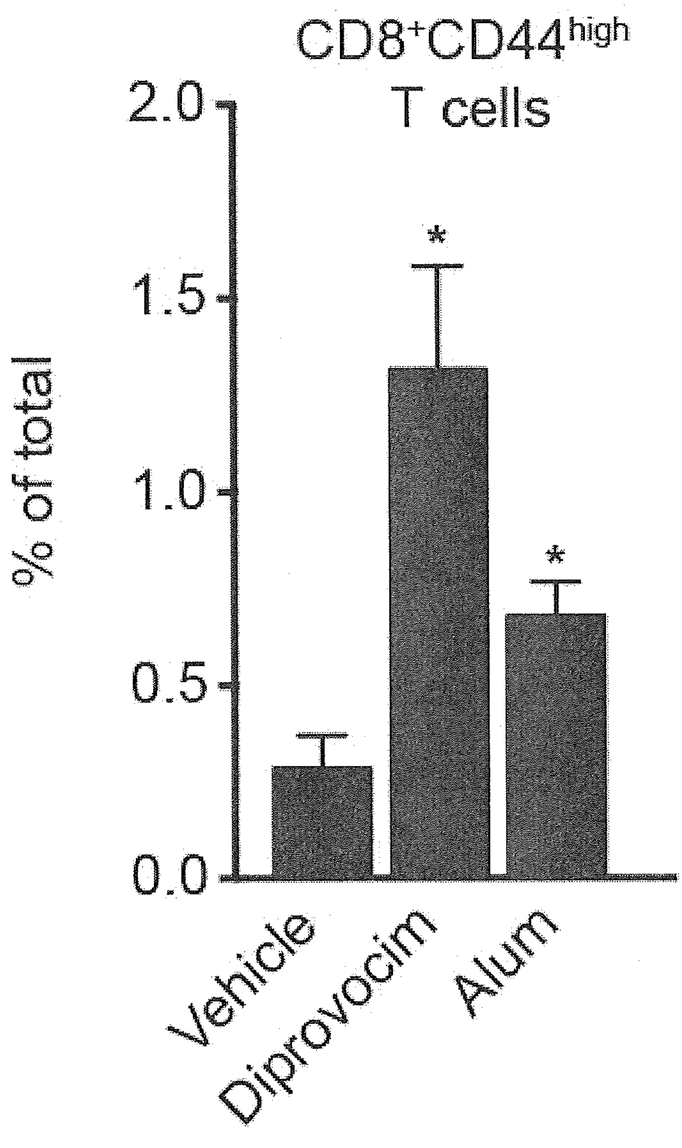
CD8+CD44high
T cells
2.0
1.5
1.0
0.5
0.0
% of total
*
*
Vehicle
Diprovocim
Alum

Fig. 5G

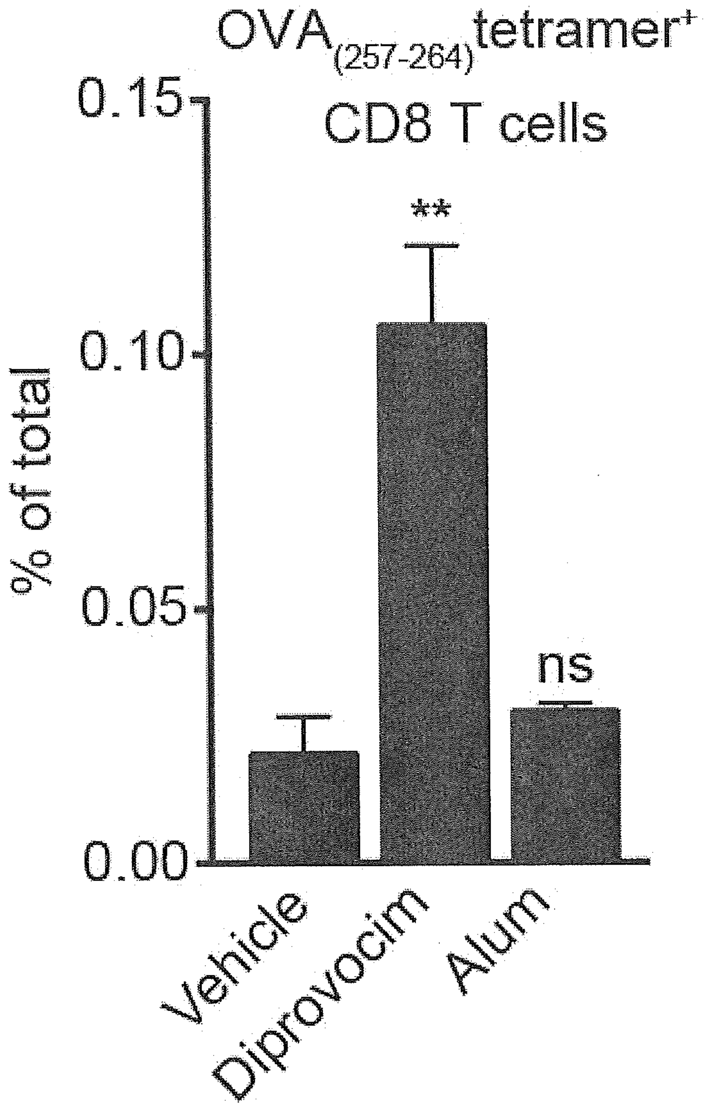
OVA(257-264)tetramer+
CD8 T cells
0.15
0.10
0.05
0.00
% of total
**
ns
Vehicle
Diprovocim
Alum

Fig. 5H

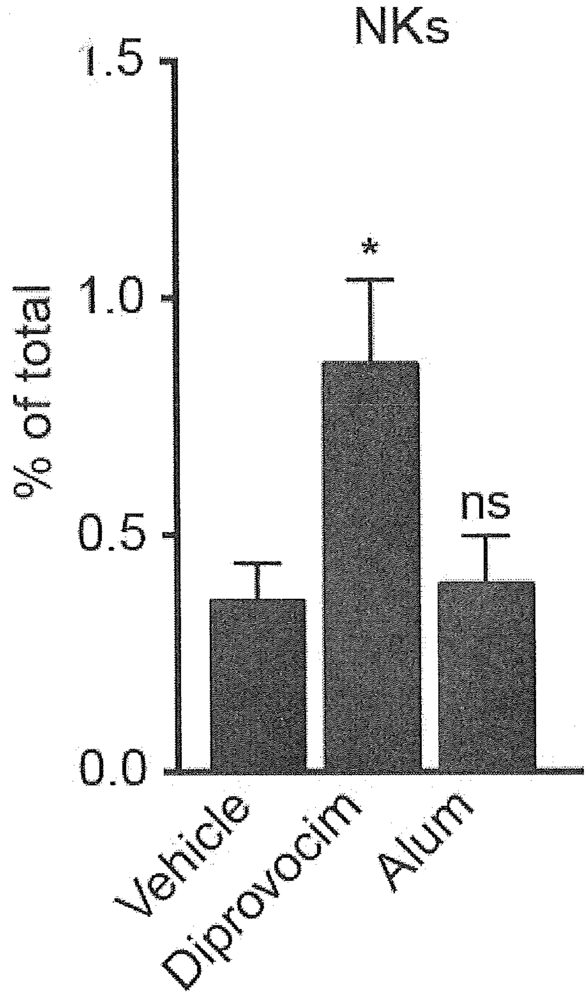

Fig. 5I

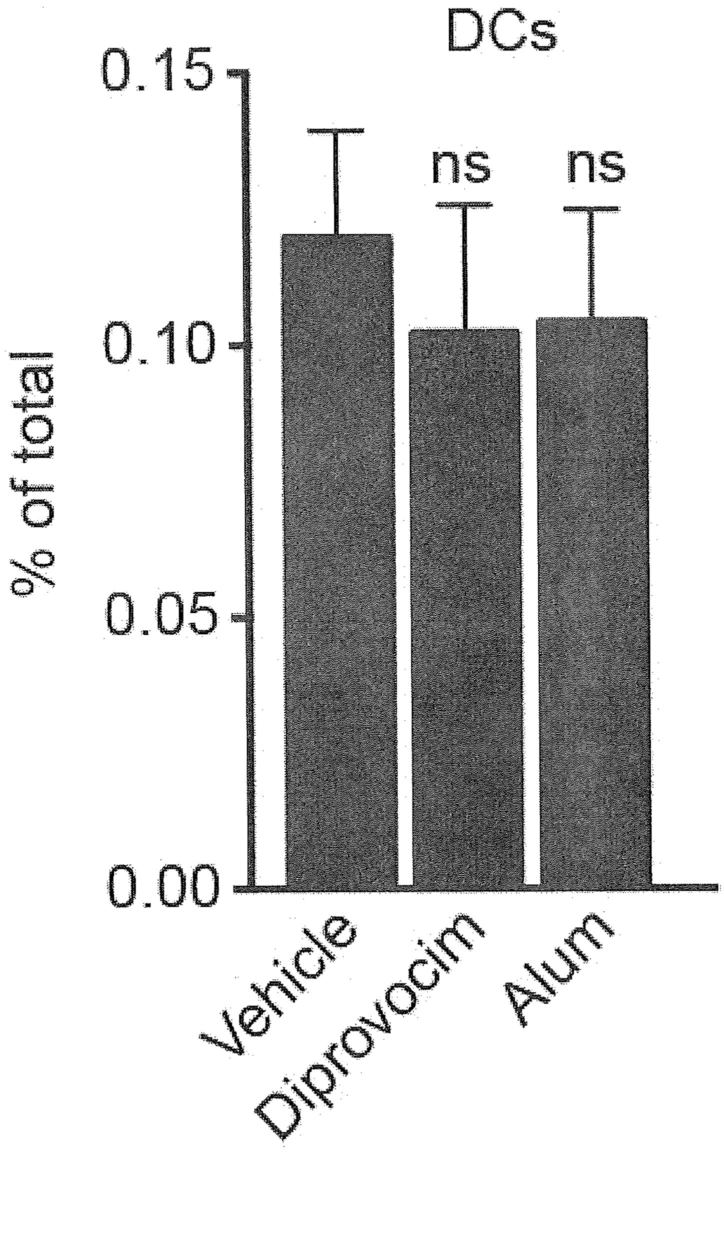

Fig. 5J

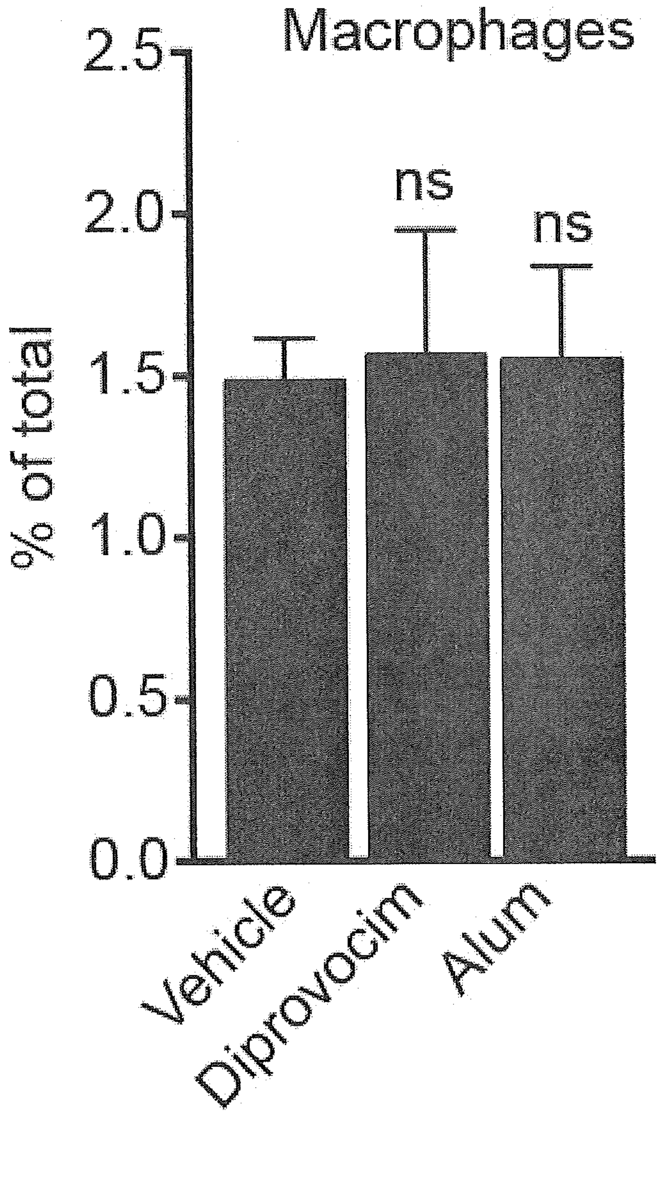
Macrophages
2.5
2.0
1.5
1.0
0.5
0.0
% of total
ns
ns
Vehicle
Diprovocim
Alum

Fig. 5K

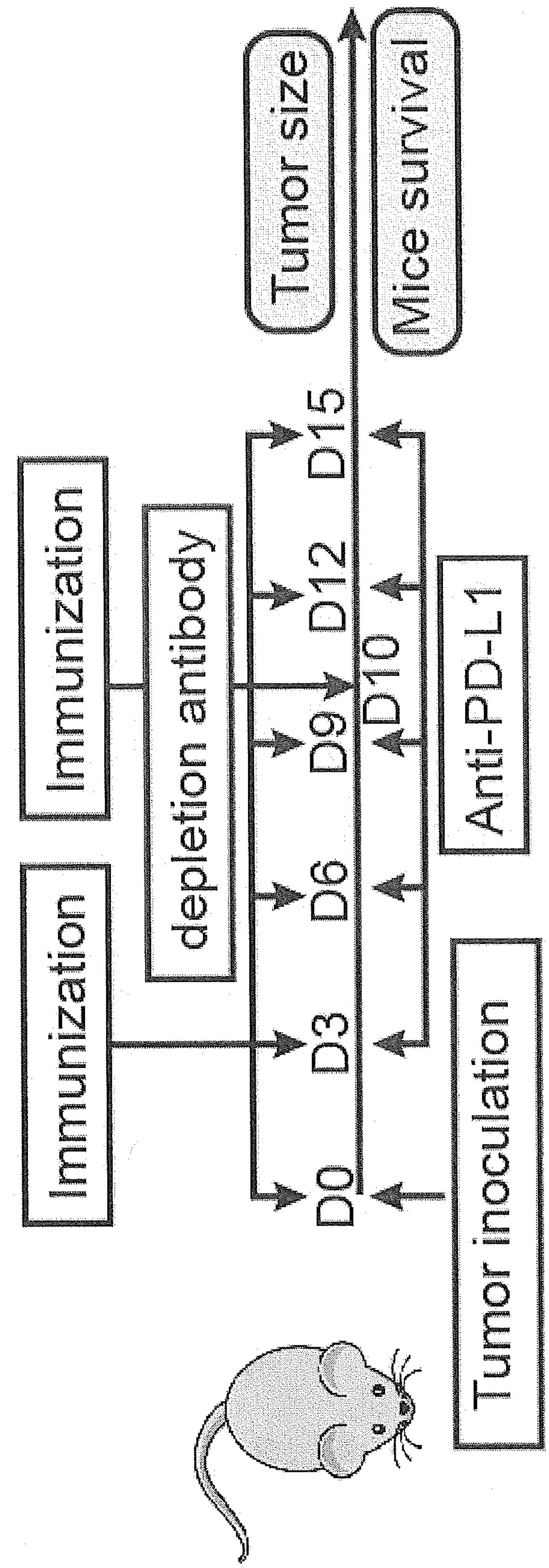
Immunization
Immunization
depletion antibody
D0
D3
D6
D9
D10
D12
D15
Anti-PD-L1
Tumor inoculation
Tumor size
Mice survival

Fig. 5L

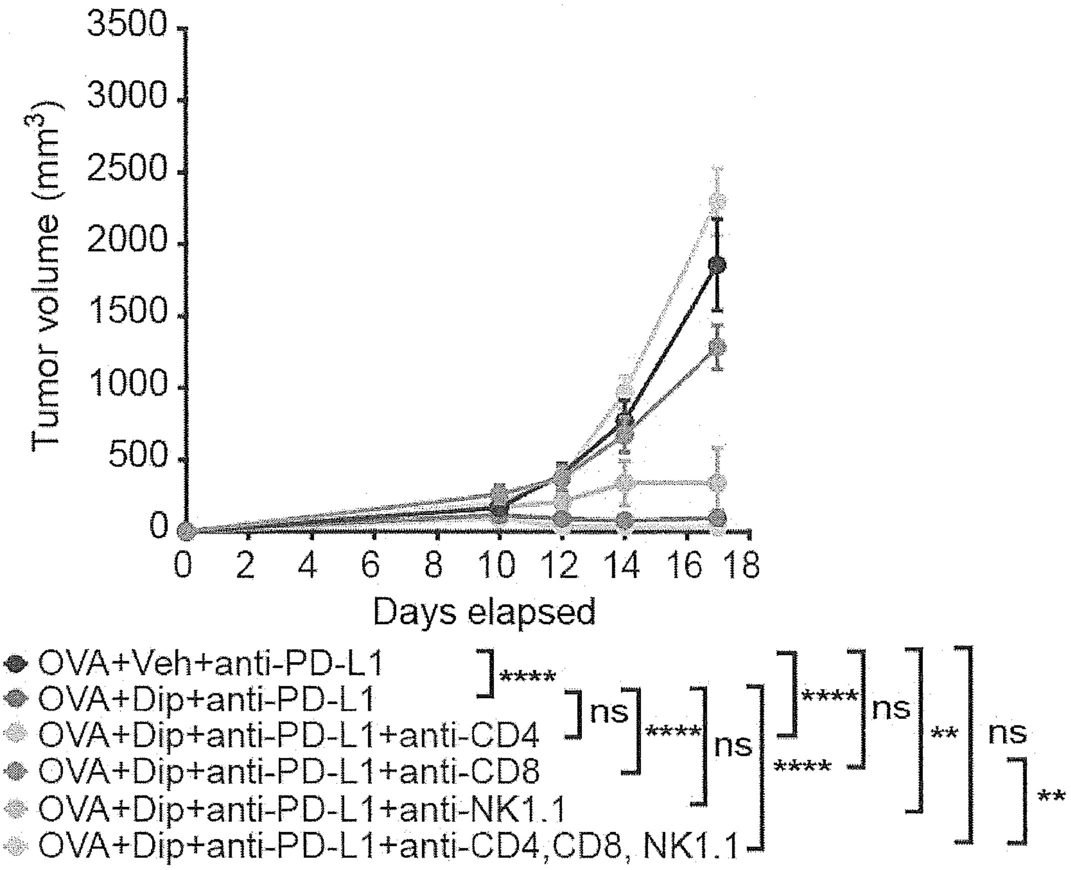
3500
3000
2500
2000
1500
1000
500
0
Tumor volume (mm3)
0 2 4 6 8 10 12 14 16 18
Days elapsed
OVA+Veh+anti-PD-L1
OVA+Dip+anti-PD-L1
OVA+Dip+anti-PD-L1+anti-CD4
OVA+Dip+anti-PD-L1+anti-CD8
OVA+Dip+anti-PD-L1+anti-NK1.1
OVA+Dip+anti-PD-L1+anti-CD4,CD8, NK1.1
****
ns
****
ns
****
****
ns
**
ns
**

# Fig. 5M

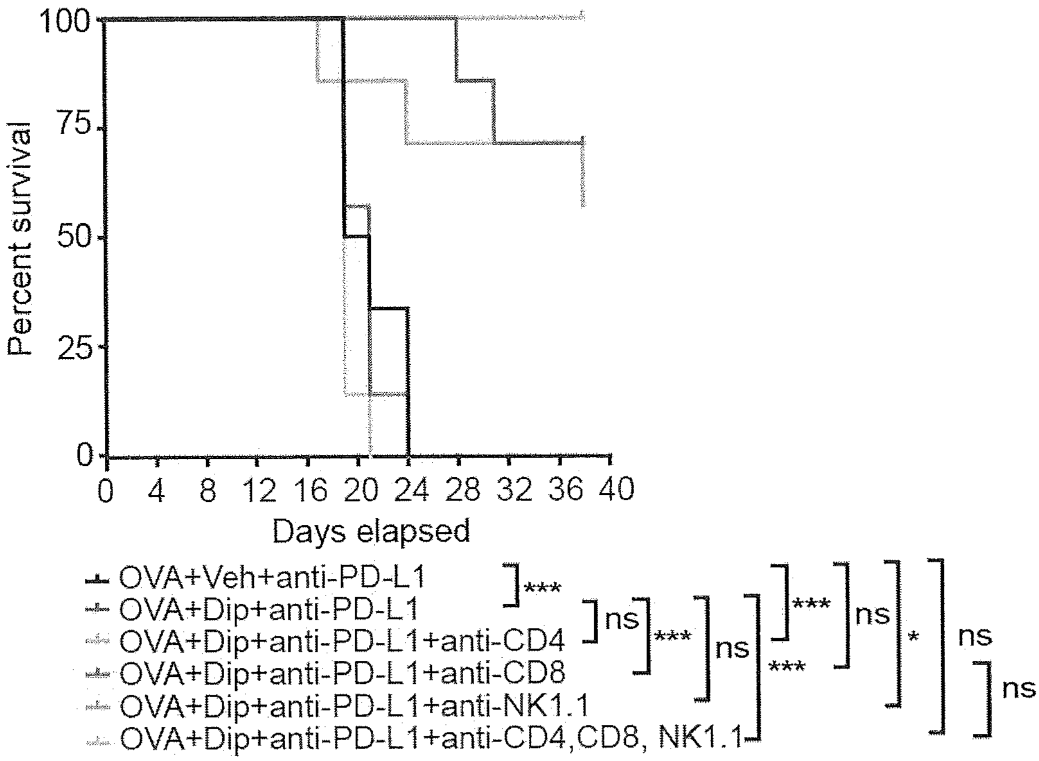
100
75
50
25
0
Percent survival
0 4 8 12 16 20 24 28 32 36 40
Days elapsed
OVA+Veh+anti-PD-L1
OVA+Dip+anti-PD-L1
OVA+Dip+anti-PD-L1+anti-CD4
OVA+Dip+anti-PD-L1+anti-CD8
OVA+Dip+anti-PD-L1+anti-NK1.1
OVA+Dip+anti-PD-L1+anti-CD4,CD8, NK1.1
***
ns
***
ns
***
***
ns
*
ns
ns

Fig. 6

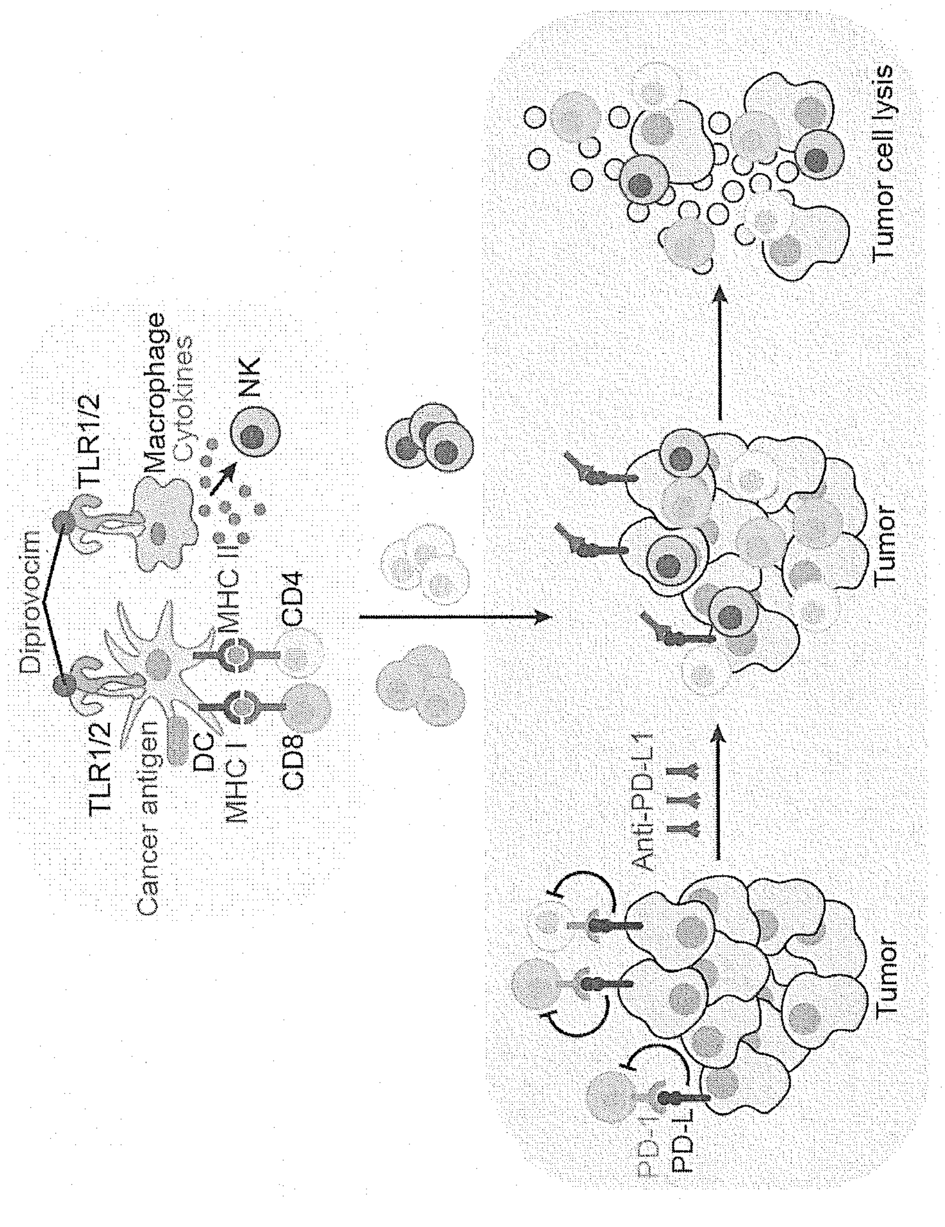
Diprovocim
TLR1/2
TLR1/2
Macrophage
Cytokines
NK
Cancer antigen
DC
MHC I
MHC II
CD8
CD4
PD-1
PD-L
Anti-PD-L1
Tumor
Tumor
Tumor cell lysis

Fig. 7

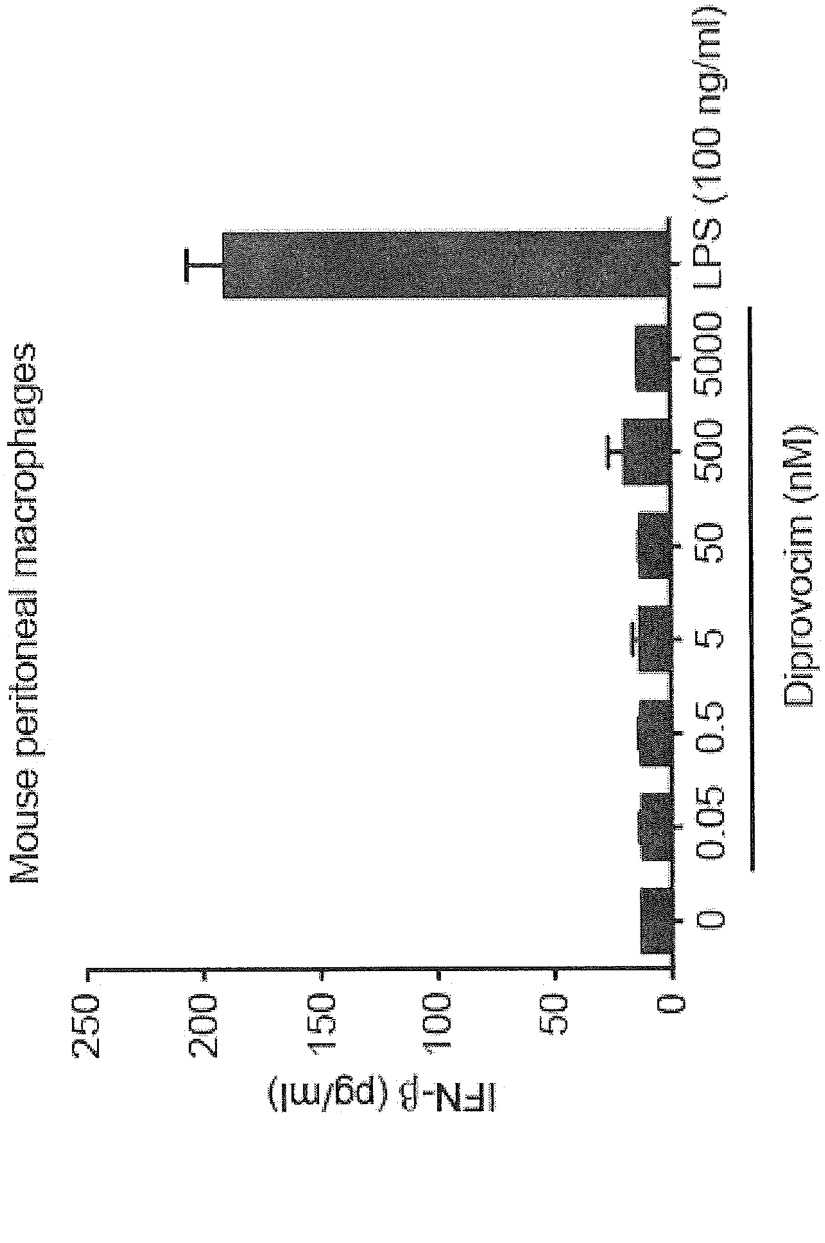
Mouse peritoneal macrophages
IFN-β (pg/ml)
250
200
150
100
50
0
0
0.05
0.5
5
50
500
5000
LPS (100 ng/ml)
Diprovocim (nM)

Fig. 8A

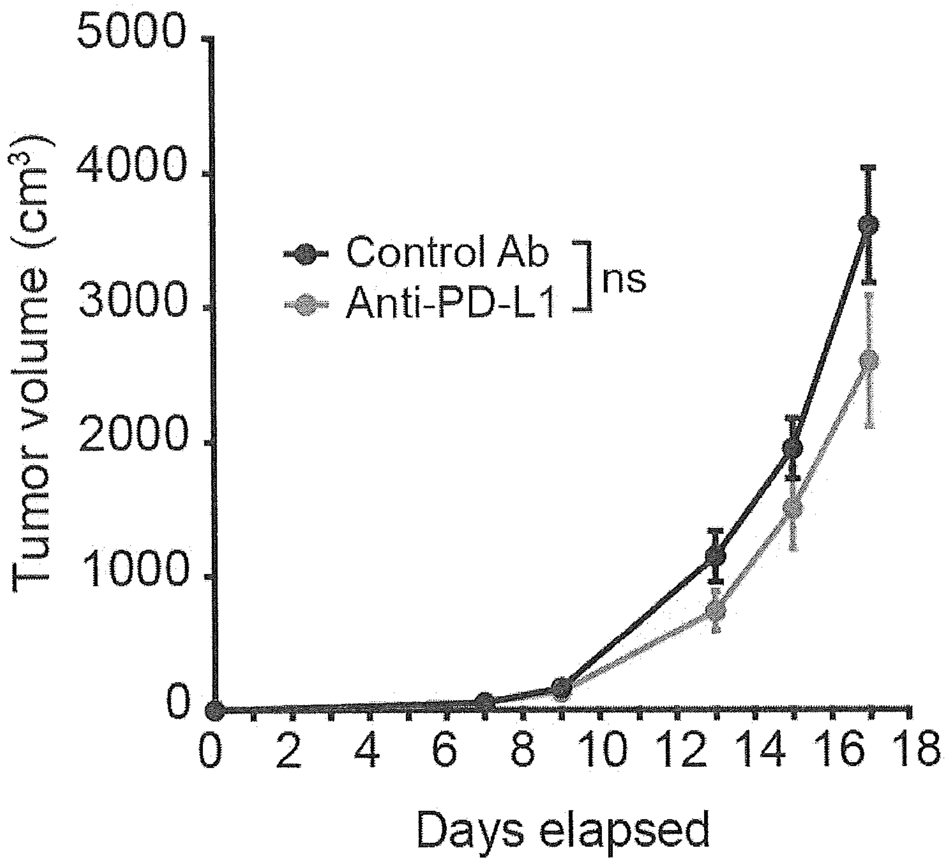
5000
4000
3000
2000
1000
0
Tumor volume (cm$^3$)
Control Ab
Anti-PD-L1
ns
0 2 4 6 8 10 12 14 16 18
Days elapsed

Fig. 8B

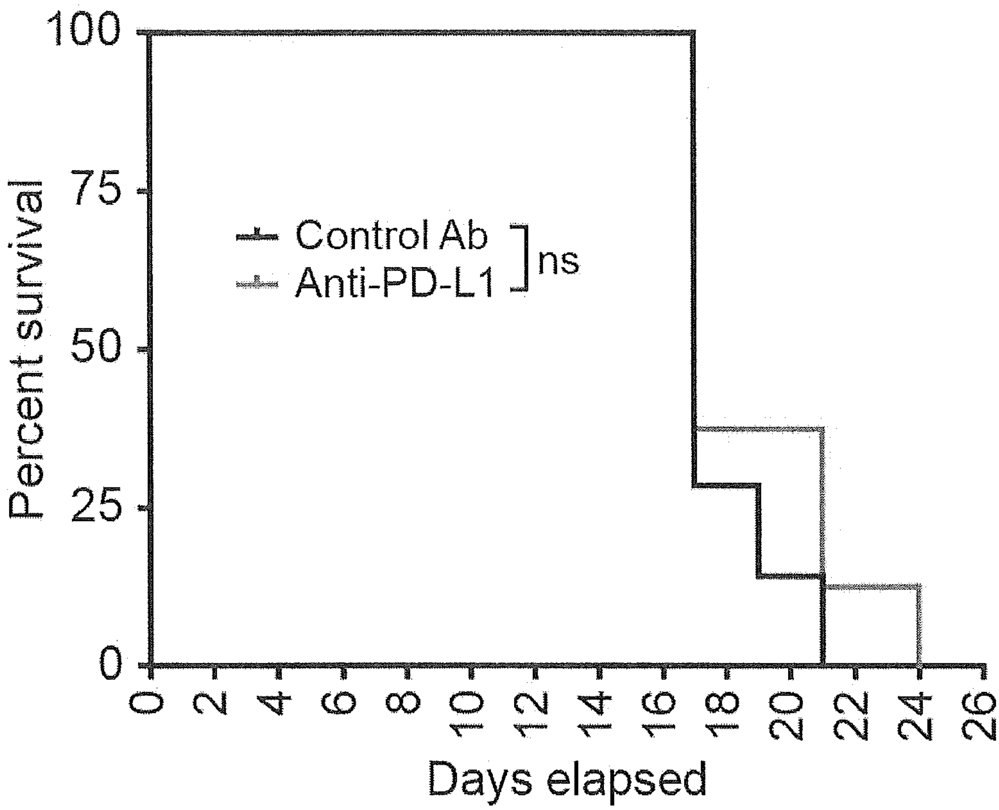
100
75
50
25
0
Percent survival
Control Ab
Anti-PD-L1
ns
0
2
4
6
8
10
12
14
16
18
20
22
24
26
Days elapsed

Fig. 9A

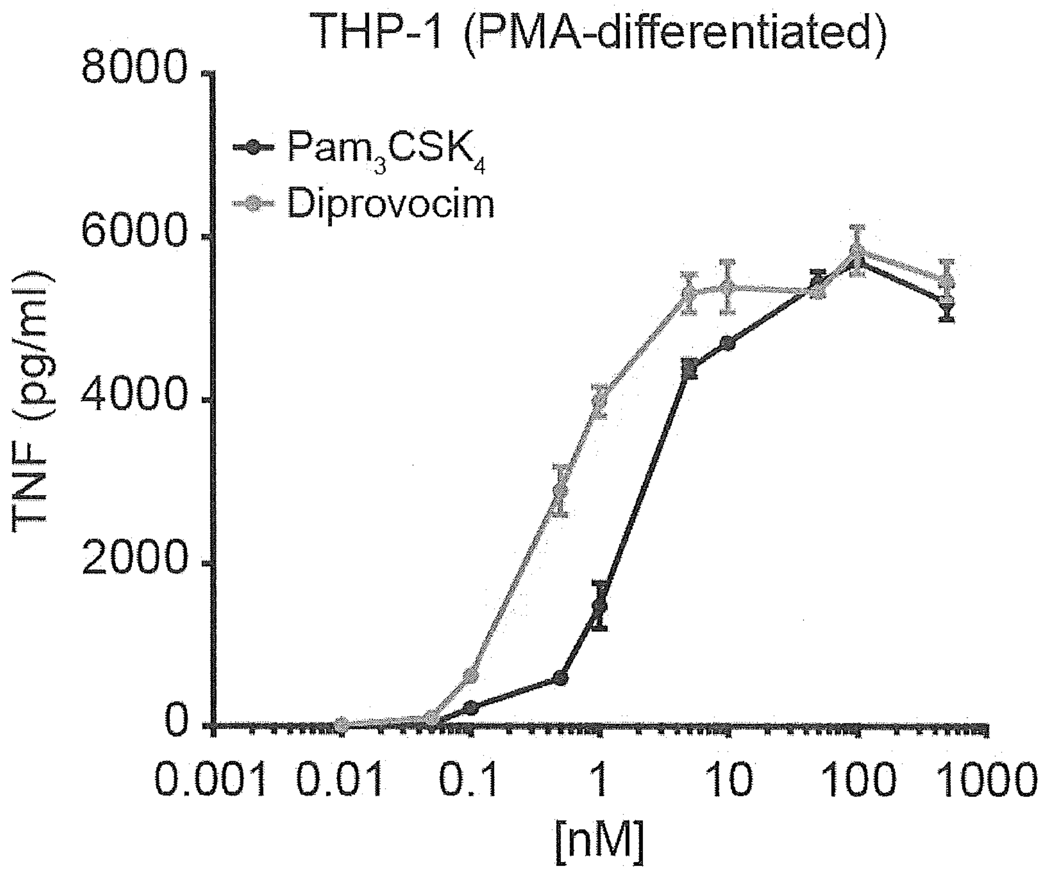
THP-1 (PMA-differentiated)
Pam3CSK4
Diprovocim
TNF (pg/ml)
8000
6000
4000
2000
0
0.001 0.01 0.1 1 10 100 1000
[nM]

Fig. 9B

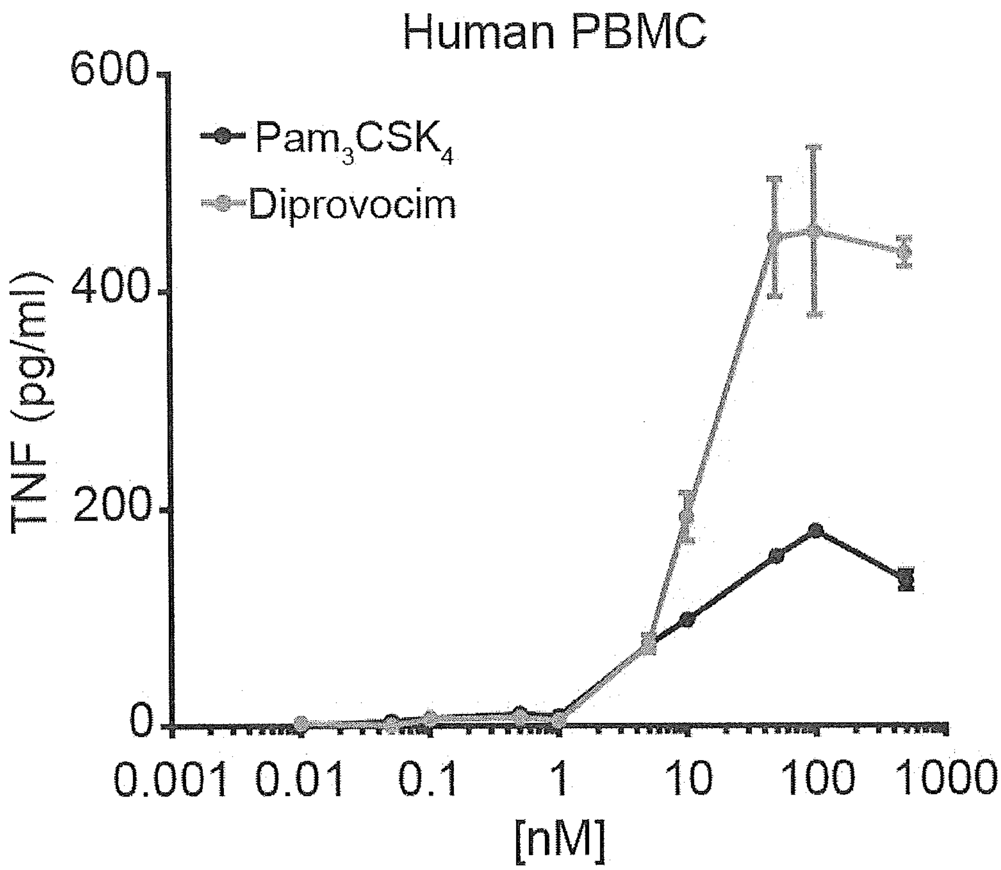
Human PBMC
600
400
200
0
TNF (pg/ml)
Pam3CSK4
Diprovocim
0.001 0.01 0.1 1 10 100 1000
[nM]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2018169224 A
- WO 2018005812 A
- US 6942866 B
- US 8017127 B
- US 4599231 A
- US 5180806 A
- WO 20180058121 A
- US 6489354 B1, Bao


### Non-patent literature cited in the description


- **MORIN et al.** J Am Chem Soc. In Press, 2018
- International Nonproprietary Names (INN) for biological and biotechnological substances (a review). *World Health Organization*, 2016
- *J. Biol. Chem.*, 1969, vol. 243, 3557-59
- **KIM et al.** *BMB Rep*, 2017, vol. 50 (6), 285-298
- **BERGE**. *J. Pharm. Sci.*, 1977, vol. 68 (1), 1-19
- **MA et al.** *Tetrahedron Asymm.*, 1997, vol. 8, 883-887
- **HOU B** ; **REIZIS B** ; **DEFRANCO AL**. Toll-like receptors activate innate and adaptive immunity by using dendritic cell-intrinsic and - extrinsic mechanisms.. *Immunity*, 2008, vol. 29 (2), 272-282
- **MACLEOD H** ; **WETZLER LM**. T cell activation by TLRs: A role for TLRs in the adaptive immune response.. *Sci STKE*, 2007, vol. 2007 (402), pe48
- **COFFMAN RL** ; **SHER A** ; **SEDER RA**. Vaccine adjuvants: Putting innate immunity to work.. *Immunity*, 2010, vol. 33 (4), 492-503
- **HAANEN JBAG**. Converting cold into hot tumors by combining immunotherapies. *Cell*, 2017, vol. 170 (6), 1055-1056
- **SHARMA P** ; **HU-LIESKOVAN S** ; **WARGO JA** ; **RIBAS A**. Primary, adaptive, and acquired resistance to cancer immunotherapy. *Cell*, 2017, vol. 168 (4), 707-723
- **WANG H et al.** cGAS is essential for the antitumor effect of immune checkpoint blockade. *Proc Natl Acad Sci U S A*, 2017, vol. 114 (7), 1637-1642
- **ZOU W** ; **WOLCHOK JD** ; **CHEN L**. PD-L1 (B7-H1) and PD-1 pathway blockade for cancer therapy: Mechanisms, response biomarkers, and combinations. *Sci Transl Med*, 2016, vol. 8 (328), 328rv4
- **TOPALIAN SL** ; **DRAKE CG** ; **PARDOLL DM**. Immune checkpoint blockade: A common denominator approach to cancer therapy.. *Cancer Cell*, 2015, vol. 27 (4), 450-461
- **TAKEDA Y et al.** A TLR3-specific adjuvant relieves innate resistance to PD-L1 blockade without cytokine toxicity in tumor vaccine immunotherapy.. *Cell Rep*, 2017, vol. 19 (9), 1874-1887
- **SHAH RR** ; **HASSETT KJ** ; **BRITO LA**. Overview of vaccine adjuvants: Introduction, history, and current status.. *Methods Mol Biol*, 2017, vol. 1494, 1-13
- **REED SG** ; **ORR MT** ; **FOX CB**. Key roles of adjuvants in modern vaccines. *Nat Med*, 2013, vol. 19 (12), 1597-1608
- **KAWAI T** ; **AKIRA S**. The role of pattern-recognition receptors in innate immunity: Update on toll-like receptors. *Nat Immunol*, 2010, vol. 11 (5), 373-384
- **KAWASAKI T** ; **KAWAI T**. Toll-like receptor signaling pathways.. *Front Immunol*, 2014, vol. 5, 461
- **BEUTLER BA**. TLRs and innate immunity.. *Blood*, 2009, vol. 113 (7), 1399-1407
- **LIM KH** ; **STAUDT LM**. Toll-like receptor signaling.. *Cold Spring Harb Perspect Biol*, 2013, vol. 5 (1), a011247
- **WANG S et al.** Intratumoral injection of a CpG oligonucleotide reverts resistance to PD-1 blockade by expanding multifunctional CD8+ T cells. *Proc Natl Acad Sci U S A*, 2016, vol. 113 (46), E7240-E7249
- **DOWLING JK** ; **MANSELL A**. Toll-like receptors: The swiss army knife of immunity and vaccine development.. *Clin Transl Immunology*, 2016, vol. 5 (5), e85
- **GOLDBERG J et al.** Erythropoietin mimetics derived from solution phase combinatorial libraries.. *J Am Chem Soc*, 2002, vol. 124 (4), 544-555
- **UEHA S et al.** Robust antitumor effects of combined anti-CD4-depleting antibody and anti-PD-1/PD-L1 immune checkpoint antibody treatment in mice.. *Cancer Immunol Res*, 2015, vol. 3 (6), 631-640

- **LECHNER MG et al.** Immunogenicity of murine solid tumor models as a defining feature of in vivo behavior and response to immunotherapy.. *J Immunother*, 2013, vol. 36 (9), 477-489
- **CHEN L et al.** Tumor immunogenicity determines the effect of B7 costimulation on T cell-mediated tumor immunity.. *J Exp Med*, 1994, vol. 179 (2), 523-532
- **MURGUEITIO MS et al.** Enhanced immunostimulatory activity of in silico discovered agonists of toll-like receptor 2 (TLR2).. *Biochim Biophys Acta*, 2017, vol. 1861 (11), 2680-2689
- **GUO X et al.** The novel toll-like receptor 2 agonist SUP3 enhances antigen presentation and T cell activation by dendritic cells.. *Front Immunol*, 2017, vol. 8, 158
- **GUAN Y** ; **OMUETI-AYOADE K** ; **MUTHA SK** ; **HERGENROTHER PJ** ; **TAPPING RI**. Identification of novel synthetic toll-like receptor 2 agonists by high throughput screening.. *J Biol Chem*, 2010, vol. 285 (31), 23755-23762
- **CHENG K et al.** Specific activation of the TLR1-TLR2 heterodimer by small-molecule agonists.. *Sci Adv*, 2015, vol. 1 (3)
- **JIN MS et al.** Crystal structure of the TLR1-TLR2 heterodimer induced by binding of a tri-acylated lipopeptide.. *Cell*, 2007, vol. 130 (6), 1071-1082
- **FREEMAN GJ et al.** Engagement of the PD-1 immunoinhibitory receptor by a novel B7 family member leads to negative regulation of lymphocyte activation.. *J Exp Med*, 2000, vol. 192 (7), 1027-1034
- **NISHIMURA H** ; **NOSE M** ; **HIAI H** ; **MINATO N** ; **HONJO T**. Development of lupus-like autoimmune diseases by disruption of the PD-1 gene encoding an ITIM motif-carrying immunoreceptor.. *Immunity*, 1999, vol. 11 (2), 141-151
- **DONG H** ; **ZHU G** ; **TAMADA K** ; **CHEN L**. B7-H1, a third member of the B7 family, co-stimulates T-cell proliferation and interleukin-10 secretion. *Nat Med*, 1999, vol. 5 (12), 1365-1369
- **TANG M et al.** Toll-like receptor 2 activation promotes tumor dendritic cell dysfunction by regulating IL-6 and IL-10 receptor signaling.. *Cell Rep*, 2015, vol. 13 (12), 2851-2864
- **YAMAZAKI S et al.** TLR2-dependent induction of IL-10 and Foxp3+ CD25+ CD4+ regulatory T cells prevents effective anti-tumor immunity induced by Pam2 lipopeptides in vivo.. *PLoS One*, 2011, vol. 6 (4), e18833
- **SHIME H et al.** Toll-like receptor 2 ligand and interferon-gamma suppress anti-tumor T cell responses by enhancing the immunosuppressive activity of monocytic myeloid-derived suppressor cells.. *Oncoimmunology*, 2017, vol. 7 (1), e1373231
- **SHEN KY et al.** Molecular mechanisms of TLR2-mediated antigen cross-presentation in dendritic cells.. *J Immunol*, 2014, vol. 192 (9), 4233-4241
- **NYIRENDA MH et al.** TLR2 stimulation drives human naive and effector regulatory T cells into a Th17-like phenotype with reduced suppressive function.. *J Immunol*, 2011, vol. 187 (5), 2278-2290
- **ZHANG Y et al.** TLR1/TLR2 agonist induces tumor regression by reciprocal modulation of effector and regulatory T cells.. *J Immunol*, 2011, vol. 186 (4), 1963-1969
- **AMISET L et al.** TLR2 ligation protects effector T cells from regulatory T-cell mediated suppression and repolarizes T helper responses following MVA-based cancer immunotherapy. *Oncoimmunology*, 2012, vol. 1 (8), 1271-1280